# EUROPEAN PATENT APPLICATION

(11) **EP 2 524 918 A1**
(43) Date of publication of application: **21.11.2012**
(21) Application number: 11382159.9
(22) Date of filing: 19.05.2011
(51) Int. Cl.: C07D 487/04, A61K 31/4985, A61P 35/00, A61P 37/00

(54) **Imidazopyrazines derivates as kinase inhibitors**

(71) Applicant: Centro Nacional de Investigaciones Oncológicas (CNIO), 28029 Madrid (ES)
(72) Inventor: PASTOR FERNANDEZ, Joaquin, E- 28029 Madrid (ES); MARTINEZ DONZALEZ, Sonia, E-28029 Madrid (ES); LORENZO GARCIA, Milagros, E-28029 Madrid (ES); BLANCO-APARICIO, Carmen, E-28029 Madrid (ES); PALACIOS DOIZTUA, Irene, E-28029 Madrid (ES); CEBRIA GOMEZ, Antonio, E-28029 Madrid (ES); QUINONES SANCHEZ, Helena, E- 28029 Madrid (ES); FOMINAYA GUTIERREZ, Jesus, E-28029 Madrid (ES)
(74) Representative: Alonso Langle, Emilio

(57) **Abstract**

There is provided compounds of formula I, 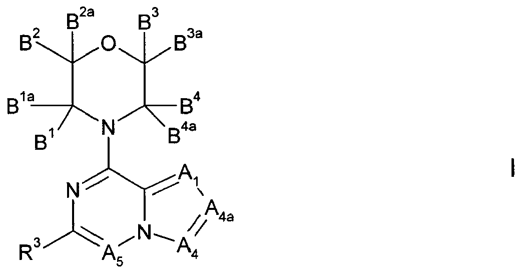 wherein A₁, A₄, A₄ₐ, A₅, B¹, B^{1a}, B², B^{2a}, B³, B^{3a} , B⁴, B^{4a} and R³ have meanings given in the description, and pharmaceutically-acceptable esters, amides, solvates or salts thereof, which compounds are useful in the treatment of diseases in which inhibition of a protein or lipid kinase (e.g. a PI3-K and/or mTOR) and, additionally, HDAC, is desired and/or required, and particularly in the treatment of cancer or a proliferative disease.

## Description

### Field of the Invention

This invention relates to novel pharmaceutically-useful compounds, which compounds are useful as inhibitors of protein or lipid kinases (such as inhibitors of the phosphoinositide 3'OH kinase (PI3 kinase) family, particularly the PI3K class I sub-type, and as inhibitors of histone deacetylase (HDAC). The compounds may also be useful as inhibitors of the mammalian target of rapamycin (mTOR)). The compounds are of potential utility in the treatment of diseases such as cancer. The invention also relates to the use of such compounds as medicaments, to the use of such compounds for *in vitro*, *in situ* and *in vivo* diagnosis or treatment of mammalian cells (or associated pathological conditions), to pharmaceutical compositions containing them, and to synthetic routes for their production.

### Background of the Invention

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis. PKs are also implicated in inflammatory conditions and in the multiplication of viruses and parasites. PKs may also play a major role in the pathogenesis and development of neurodegenerative disorders.

For a general reference to PKs malfunctioning or disregulation see, for instance, Current Opinion in Chemical Biology 1999, 3, 459 - 465.

Phosphatidylinositol 3-kinases (PI3Ks) are a family of lipid and serine/threonine kinases that catalyze the phosphorylation of the membrane lipid phosphatidylinositol (PI) on the 3'-OH of the inositol ring to produce phosphoinositol-3-phosphate (PIP), phosphoinositol-3,4-diphosphate (PIP₂) and phosphoinositol-3,4,5-triphosphate (PIP₃), which act as recruitment sites for various intracellular signalling proteins, which in turn form signalling complexes to relay extracellular signals to the cytoplasmic face of the plasma membrane. These 3'-phosphoinositide subtypes function as second messengers in intracellular signal transduction pathways (see e.g. Trends Biochem. Sci 22 87,267-72 (1997) by Vanhaesebroeck *et al*.; Chem. Rev. 101 (8), 2365-80 (2001) by Leslie et al (2001); Annu. Rev. Cell. Dev. Boil. 17, 615-75 (2001) by Katso *et al*; and Cell. Mol. Life Sci. 59 (5), 761-79 (2002) by Toker et al).

Multiple PI3K isoforms categorized by their catalytic subunits, their regulation by corresponding regulatory subunits, expression patterns and signalling specific funtions (p110α, β, δ, γ) perform this enzymatic reaction (Exp. Cell. Res. 25 (1),. 239-54 (1999) by Vanhaesebroeck and Katso et al., 2001, above).

The closely related isoforms p110α and β are ubiquitously expressed, white δ and γ are more specifically expressed in the haematopoietic cell system, smooth muscle cells, myocytes and endothelial cells (see e.g. Trends Biochem. Sci. 22 (7),. 267-72 (1997) by Vanhaesebroeck et al). Their expression might also be regulated in an inducible manner depending on the cellular, tissue type and stimuli as well as disease context. Inductibility of protein expression includes synthesis of protein as well as protein stabilization that is in part regulated by association with regulatory subunits.

Eight mammalian PI3Ks have been identified so far, including four class I PI3Ks. Class la includes PI3Kα, PI3Kβ and PI3Kδ. All of the class la enzymes are heterodimeric complexes comprising a catalytic subunit (p110α, p110β or p110δ) associated with an SH2 domain containing p85 adapter subunit. Class la PI3Ks are activated through tyrosine kinase signalling and are involved in cell proliferation and survival. PI3Kα and PI3Kβ have also been implicated in tumorigenesis in a variety of human cancers. Thus, pharmacological inhibitors of PI3Kα and PI3Kβ are useful for treating various types of cancer.

PI3Kγ, the only member of the Class Ib PI3Ks, consists of a catalytic subunit p110γ, which is associated with a p110 regulatory subunit. PI3Kγ is regulated by G protein coupled receptors (GPCRs) via association with βγ subunits of heterotrimeric G proteins. PI3Kγ is expressed primarily in hematopoietic cells and cardiomyocytes and is involved in inflammation and mast cell function. Thus, pharmacological inhibitors of PI3Kγ are useful for treating a variety of inflammatory diseases, allergies and cardiovascular diseases.

These observations show that deregulation of phosphoinositol-3-kinase and the upstream and downstream components of this signalling pathway is one of the most common deregulations associated with human cancers and proliferative diseases (see e.g. Parsons et al., Nature 436:792 (2005); Hennessey et al., Nature Rev. Drug Discovery 4: 988-1004 (2005).

The mammalian target of rapamycin (mTOR) also known as FK506 binding protein 12-rapamycin associated protein 1 (FRAP1) is a protein which in humans is encoded by the *FRAP1* gene. mTOR is a serine/threonine protein kinase that regulates cell growth, cell proliferation, cell motility, cell survival, protein synthesis, and transcription. The inhibition of mTORs are believed to be useful for treating various diseases/conditions, such as cancer (for example, as described in Easton et al. (2006). "mTOR and cancer therapy". Oncogene 25 (48): 6436-46).

Histone acetylation is a reversible modification, with deacetylation being catalysed by a family of enzymes termed histone deacetylases (HDACs). HDACs are represented by 18 genes in humans and are divided into four distinct classes (J Mol Biol, 2004, 338:1, 17-31). In mammalians, class I HDACs (HDAC1-3, HDAC8) are related to yeast RPD3 HDAC, class 2 (HDAC4-7, HDAC9 and HDAC10) related to HDAC1, class 4 (HDAC11) and class 3 (a distinct class encompassing the sirtuins which are related to yeast Sir2).

Csordas, Biochem. J., 1990, 286:23-38 teaches that histones are subject to post-translational acetylation of the ε-amino groups of *N*-terminal lysine residues, a reaction that is catalysed by histone acetyl transferase (HAT1). Acetylation neutralises the positive charge of the lysine side chain, and is thought to impact chromatin structure. Indeed, access of transcription factors to chromatin templates is enhanced by histone hyperacetylation, and enrichment in underacetylated histone H4 has been found in transcriptionally silent regions of the genome (Taunton et al., Science, 1996, 272:408-411). In the case of tumour suppressor genes, transcriptional silencing due to histone modification can lead to oncogenic transformation and cancer.

Several classes of HDAC inhibitors are currently being evaluated clinically, including hydroxamic acid derivatives.

Certain cancers may be treated with a combination of drugs, for instance when monotherapy is inadequate. However, such treatment regimes are often limited by dose limiting toxicities and drug-drug interactions.

For the treatment of cancer, targeted therapies are becoming more important. That is, therapy that has the effect of interfering with specific target molecules that are linked to tumor growth and/or carcinogenesis. Such therapy may be more effective than current treatments (e.g. chemotherapy) and less harmful to normal cells (e.g. because chemotherapy has the potential to kill normal cells as well as cancerous cells). This, and also the fact that targeted therapies may be selective (i.e. it may inhibit a certain targeted molecule more selectively as compared to other molecular targets, e.g. as described hereinafter), may have the benefit of reducing side effects and may also have the benefit that certain specific cancers can be treated (also selectively). The latter may in turn also reduce side effects.

Hence, it is a clear goal of current oncologists to develop targeted therapies (e.g. ones that are selective). In this respect, it should be pointed out that several different molecular targets may exist that are linked to certain diseases (e.g. cancer). However, one simply cannot predict if a therapy (e.g. a small molecule as a therapeutic) that interferes with or inhibits one target molecule could inhibit a different molecular target (be it one that will ultimately have the effect of treating the same disease or a different one).

Another goal for oncologists is the development of compounds that may inhibit multiple targets (or even multiple pathways). Such an approach may be considered to be advantageous as it may allow for more efficacious effects using a single compound, whilst avoiding the use of combinations of different compounds and the associated drawbacks. Such an approach may therefore provide associated benefits such as reducing the risk of drug-drug interactions, allowing more suitable doses, etc. The safety of a single compound as a drug may also be more easily demonstrated.

The listing or discussion of an apparently prior-published document in this specification should not necessarily be taken as an acknowledgement that the document is part of the state of the art or is common general knowledge.

International patent application WO 2010/119264 discloses various imidazopyrazines for use as certain kinase inhibitors. Unpublished international patent application PCT/GB2011/000086 and European patent application no. 10380070.2 both disclose various bicyclic compounds for use as certain kinase inhibitors. None of these documents disclose hydroxamic acid derivatives of specific compounds, and nor do these documents mention that those compounds may be useful as HDAC inhibitors (or matrix metalloproteinase (MMP) inhibitors).

Several documents disclose certain polycyclic kinase inhibitors that may be useful as inhibitors of PI3K and/or mTOR, including international patent applications WO 2008/073785, WO 2008/070740 and WO 2007/127183 and US patent application 2008/0242665. An example of a PI3K inhibitor that is currently under clinical investigation is the morpholine-substituted thienopyrimidine GDC-0941.

International patent application WO 2010/080996 discloses various bicyclic compounds that potentially have multiple targeting properties (e.g. inhibiting PI3K and/or mTOR and, additionally HDAC or MMP. However, this document mainly relates to deazapurines, thienopyrimidines and furopyrimidines.

### Disclosure of the Invention

According to the invention, there is now provided a compound of formula I, 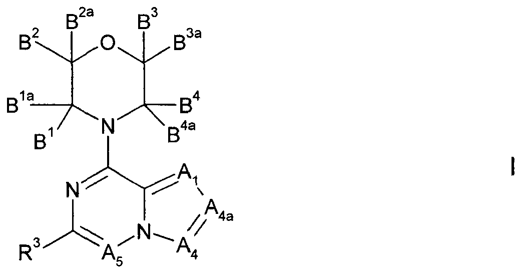 wherein:
A₁ represents N or C(H);
A₄ represents N or C(R^{1a});
A₄ₐ represents N or C(R^{1b});
wherein at least one of A₄ and A₄ₐ does not represent N;
A₅ represents N or C(R²);
each B¹, B^{1a}, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B^{1a}, B², B^{2a}, B³, B^{3a} B⁴ and B^{4a} substituents that are attached to the same carbon atom (i.e. B¹ and B^{1a}; B² and B^{2a}; B³ and B^{3a}; and/or B⁴ and B^{4a}) may together form a =O group;
or, any two B¹, B^{1a}, B², B^{2a}, B³, B^{3a}, B⁴ and B^{4a} substituents may be linked together to form a further 3- to 12- membered (e.g. 3- to 6-membered) ring, optionally containing (in addition to the atom(s) of the morpholine ring) one or more (e.g. two or, preferably, one) heteroatom(s) (preferably selected from sulfur, oxygen and nitrogen), which ring optionally contains one or more (e.g. one to three) double bonds, and which ring is itself optionally substituted by one or more substituents selected from halo, =O and C₁₋₃ alkyl optionally substituted by one or more fluoro atoms;
R² (when present) independently represents hydrogen or a substituent selected from halo, -CN, -OR^{10b}, -N(R^{10b})R^{11b} -C(O)N(R^{10b})R11^{b}, C₁₋₁₂ (e.g. C₁₋₆) alkyl and heterocycloalkyl (e.g. a 3- to 7-membered heterocycloalkyl), which latter two groups are optionally substituted by one or more substituents selected from E³ and =O;
characterised in that:
one of R^{1a} and R^{1b} is present and represents -T¹-Z¹ and the other (if present) represents R^{1c};
T¹ represents a linker group selected from -T^{1a}-X^{1a}-X^{1b}-T1^{b}-;
Z¹ represents a group selected from:
   (i) -C(=Y)N(R^{10a})-OR^{11c};
   (ii) 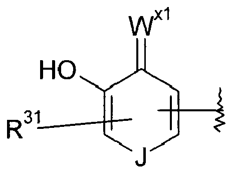 W^{x1} is O or S; J is O, NH or NCH₃; and R³¹ is hydrogen or C₁₋₄ alkyl;
   (iii) 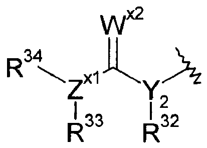 W^{x2} is O or S; Y₂ is absent, N or CH; Z^{x1} is N or CH; R³² and R³⁴ are independently hydrogen, hydroxy, an aliphatic group (i.e. a non-aromatic moiety that may be saturated or contain one or more units of unsaturation), provided that if both R³² and R³⁴ are present, one of R³² and R³⁴ must be hydroxy and if Y₂ is absent, R³⁴ must be hydroxy; and R³³ is hydrogen or aliphatic group;
   (iv) 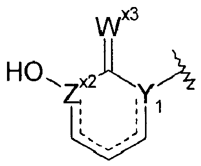 W^{x3} is O or S; Y₁ and Z^{x2} are independently N, C or CH; and
   (v) 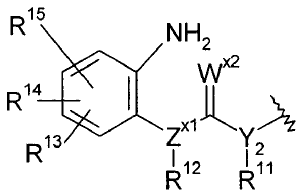 Z^{x1}, Y₂ and W^{x2} are as defined above, R¹¹ and R¹² are independently selected from hydrogen and an aliphatic group; R¹³, R¹⁴ and R¹⁵ are independently selected from hydrogen, hydroxy, amino, halo, alkoxy, alkylamino, dialkylamino, CF₃, CN, NO₂, sulfonyl, acyl, an optionally substituted aliphatic group, optionally substituted aryl, optionally substituted heteroaryl and optionally substituted heterocycloalkyl;
   T^{1a} and T^{1b} independently represent a direct bond or C₁₋₁₂ alkylene optionally substituted by one or more substituents selected from Q^{1a};
   X^{1a} represents a direct bond, -N(R^{t1})-, -C(O)N(R^{t2})-, -N(R^{t3})-C(O)-, -O-, -S-, -S(O)-, -S(O)₂-, arylene (optionally substituted by one or more substituents selected from Q^{1b}), heteroarylene (optionally substituted by one or more substituents selected from Q^{1c}) or cycloalkylene or heterocycloalkylene (which latter two optionally comprise a further ring as defined by Z^{a}, and which ring(s) (i.e. cycloalkyl/heterocycloalkyl and optional further ring) is/are optionally substituted by one or more substituents selected from =O and Q^{1d});
   t1, t2 and t3 independently represent hydrogen or C₁₋₆ alkyl optionally substituted by one or more fluoro atoms;
   when X^{1a} represents -N(R^{t1})-, -C(O)N(R^{t2})-, -N(R^{t3})-C(O)-, -O-, -S-, -S(O)- or -S(O)₂-, then:
   X^{1b} represents a direct bond, arylene (optionally substituted by one or more substituents selected from Q^{1e}), heteroarylene (optionally substituted by one or more substituents selected from Q^{1f}) or cycloalkylene or heterocycloalkylene (which latter two optionally comprise a further ring as defined by Z^{b}, and which ring(s) (i.e. cycloalkyl/heterocycloalkyl and optional further ring) is/are optionally substituted by one or more substituents selected from =O and Q^{1g});
   when X^{1a} represents a direct bond, arylene (optionally substituted by one or more substituents selected from Q^{1b}), heteroarylene (optionally substituted by one or more substituents selected from Q^{1c}) or cycloalkylene or heterocycloalkylene (which latter two optionally comprise a further Z^{a} ring; and which are optionally substituted by one or more substituents selected from =O and Q^{1d}), then:
   X^{1b} may represent a direct bond, -N(R^{t1})-, -C(O)N(R^{t2})-, -N(R^{t3})-C(O)-, -O-, -S-, -S(O)-, -S(O)₂-, arylene (optionally substituted by one or more substituents selected from Q^{1e}), heteroarylene (optionally substituted by one or more substituents selected from Q^{1f}) or cycloalkylene or heterocycloalkylene (which latter two optionally comprise a further ring as defined by Z^{b}, and which ring(s) (i.e. cycloalkyl/heterocycloalkyl and optional further ring) is/are optionally substituted by one or more substituents selected from =O and Q^{1g});
   R^{1c} (when present) represents hydrogen or a substituent selected from halo and C₁₋₃ alkyl (optionally substituted by one or more substituents selected from =O, fluoro and -OCH₃);
   Z^{a} and Z^{b} each independently represent a moiety that results in a further ring sytem (that is present in addition to the "first ring" i.e. in addition to the monocyclic cycloalkyl/heterocycloalkyl groups, to which that Z^{a} to Z^{b} group is attached) that is formed by that Z^{a} or Z^{b} group representing:
   (a) a 3- to 7-membered saturated heterocycloalkyl group containing one to four heteroatoms selected from oxygen, sulfur and nitrogen (preferably oxygen and nitrogen), a 3- to 12-membered saturated carbocyclic ring, or an unsaturated 5- to 12-membered carbocyclic or heterocyclic ring (in which the heteroatoms are preferably selected from sulfur and, especially, nitrogen and oxygen) that is fused to the first ring;
   (b) a linker group -(C(R^{x})₂)ₚ- and/or -(C(R^{x})₂)ᵣ-O-(C(R^{x})₂)ₛ- (wherein p is 1 or 2; r is 0 or 1; s is 0 or 1; and each R^{x} independently represents hydrogen or C₁₋₆ alkyl), linking together any two non-adjacent atoms of the first ring (i.e. forming a bridged structure); or
   (c) a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring *via* a single carbon atom common to both rings (i.e. forming a spiro-cycle);
   R³ represents aryl or heteroaryl (both of which are optionally substituted by one or more substituents selected from E⁴);
   each Q^{1a}, Q^{1b}, Q^{1c}, Q^{1d}, Q^{1e} Q^{1f} and Q^{1g} independently represents, on each occasion when used herein:
   halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -C(=Y)N(R^{10a})-OR^{11c}, -OC(=Y)-R^{10a}, -OC(=Y)-OR^{10a}, -OC(=Y)N(R^{10a})R^{11a}, -OS(O)₂OR^{10a}, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁵), aryl or heteroaryl (which latter two groups are optionally substituted by one or more substituents selected from E⁶);
   each R^{11c} independently represents C₁₋₁₂ alkyl, heterocycloalkyl (which latter two groups are optionally substituted by one or more substituents selected from =O, =S, =N(R²⁰) and E⁷), aryl or heteroaryl (which latter two groups are optionally substituted by one or more substituents selected from E⁸);
   each R^{10a}, R^{11a} R^{10b}, R^{11b} and R^{12a} independently represent, on each occasion when used herein, hydrogen, C₁₋₁₂ alkyl, heterocycloalkyl (which latter two groups are optionally substituted by one or more substituents selected from =O, =S, =N(R²⁰) and E⁷), aryl or heteroaryl (which latter two groups are optionally substituted by one or more substituents selected from E⁸); or
   any relevant pair of R^{10a} and R^{11a} or R^{10b} and R^{11b} (for example, when attached to the same atom, adjacent atom (i.e. 1,2-relationship) or to atoms that are two atoms apart, i.e. in a 1,3-relationship) may be linked together to form (e.g. along with the requisite nitrogen atom to which they may be attached) a 4- to 20- (e.g. 4- to 12-) membered ring, optionally containing one or more heteroatoms (for example, in addition to those that may already be present, e.g. (a) heteroatom(s) selected from oxygen, nitrogen and sulfur), optionally containing one or more unsaturations (preferably, double bonds), and which ring is optionally substituted by one or more substituents selected from =O, =S, =N(R²⁰) and E⁹;
   each E¹, E², E³, E⁴, E⁵, E⁶, E⁷, E⁸ and E⁹ independently represents, on each occasion when used herein:
   (i) Q⁴;
   (ii) C₁₋₁₂ alkyl optionally substituted by one or more substituents selected from =O and Q⁵; or
   any two E¹, E², E³, E⁴, E⁵, E⁶, E⁷, E⁸ or E⁹ groups (for example on C₁₋₁₂ alkyl groups, e.g. when they are attached to the same or adjacent carbon atoms, or, on aromatic groups, when attached to adjacent atoms), may be linked together to form a 3- to 12-membered ring, optionally containing one or more (e.g. one to three) unsaturations (preferably, double bonds), and which ring is optionally substituted by one or more substituents selected from =O and J¹;
   each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²⁰, -OP(=Y)(OR²⁰)(OR²¹), -OP(OR²⁰)(OR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²¹, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³);
   each Y independently represents, on each occasion when used herein, =O, =S, =NR²³ or =N-CN;
   each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵);
   each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
   any relevant pair of R²⁰, R²¹ and R²², may (for example, when attached to the same atom, adjacent atom (i.e. 1,2-relationship) or to atoms that are two atoms apart, i.e. in a 1,3-relationship) be linked together to form (e.g. along with the requisite nitrogen atom to which they may be attached) a 4- to 20- (e.g. 4- to 12-) membered ring, optionally containing one or more heteroatoms (for example, in addition to those that may already be present, e.g. (a) heteroatom(s) selected from oxygen, nitrogen and sulfur), optionally containing one or more unsaturations (preferably, double bonds), and which ring is optionally substituted by one or more substituents selected from J⁶ and =O;
   each J¹, J², J³, J⁴, J⁵ and J⁶ independently represents, on each occasion when used herein:
   (i) Q⁷;
   (ii) C₁₋₆ alkyl or heterocycloalkyl, both of which are optionally substituted by one or more substituents selected from =O and Q⁸;
   each Q⁷ and Q⁸ independently represents, on each occasion when used herein: halo, -CN, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -N(R⁵²)C(=Y^{a})R⁵¹, -NR⁵²S(O)₂R⁵⁰, -S(O)₂N(R⁵⁰)R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocyclalkyl, aryl or heteroaryl (which latter three groups are optionally substituted by one or more substituents selected from halo, -OR⁶⁰ and -N(R⁶¹)R⁶²);
   each Y^{a} independently represents, on each occasion when used herein, =O, =S, =NR⁵³ or =N-CN;
   each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
   any relevant pair of R⁵⁰, R⁵¹ and R⁵² may (for example when attached to the same or adjacent atoms) be linked together to form, a 3- to 8-membered ring, optionally containing one or more heteroatoms (for example, in addition to those that may already be present, heteroatoms selected from oxygen, nitrogen and sulfur), optionally containing one or more unsaturations (preferably, double bonds), and which ring is optionally substituted by one or more substituents selected from =O and C₁₋₃ alkyl;
   R⁶⁰, R⁶¹ and R⁶² independently represent hydrogen or C₁₋₆ alkyl optionally substituted by one or more fluoro atoms;
   or a pharmaceutically acceptable ester, amide, solvate or salt thereof,
   which compounds, esters, amides, solvates and salts are referred to hereinafter as "the compounds of the invention".

Pharmaceutically-acceptable salts include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo*, by freeze-drying or by filtration). Salts may also be prepared by exchanging a counter-ion of a compound of the invention in the form of a salt with another counter-ion, for example using a suitable ion exchange resin.

By "pharmaceutically acceptable ester, amide, solvate or salt thereof", we include salts of pharmaceutically acceptable esters or amides, and solvates of pharmaceutically acceptable esters, amides or salts. For instance, pharmaceutically acceptable esters and amides such as those defined herein may be mentioned, as well as pharmaceutically acceptable solvates or salts.

Pharmaceutically acceptable esters and amides of the compounds of the invention are also included within the scope of the invention. Pharmaceutically acceptable esters and amides of compounds of the invention may be formed from corresponding compounds that have an appropriate group, for example an acid group, converted to the appropriate ester or amide. For example, pharmaceutically acceptable esters (of carboxylic acids of compounds of the invention) that may be mentioned include optionally substituted C₁₋₆ alkyl, C₅₋₁₀ aryl and/or C₅₋₁₀ aryl-C₁-₆ alkyl- esters. Pharmaceutically acceptable amides (of carboxylic acids of compounds of the invention) that may be mentioned include those of the formula -C(O)N(R^{z1})R^{z2}, in which R^{z1} and R^{z2} independently represent optionally substituted C₁₋₆ alkyl, C₅₋₁₀ aryl, or C₅₋₁₀ aryl-C₁₋₆ alkylene-. Preferably, C₁₋₆ alkyl groups that may be mentioned in the context of such pharmaceutically acceptable esters and amides are not cyclic, e.g. linear and/or branched.

Further compounds of the invention that may be mentioned include carbamate, carboxamido or ureido derivatives, e.g. such derivatives of existing amino functional groups.

For the purposes of this invention, therefore, prodrugs of compounds of the invention are also included within the scope of the invention.

The term "prodrug" of a relevant compound of the invention includes any compound that, following oral or parenteral administration, is metabolised *in vivo* to form that compound in an experimentally-detectable amount, and within a predetermined time (e.g. within a dosing interval of between 6 and 24 hours (i.e. once to four times daily)). For the avoidance of doubt, the term "parenteral" administration includes all forms of administration other than oral administration.

Prodrugs of compounds of the invention may be prepared by modifying functional groups present on the compound in such a way that the modifications are cleaved, *in vivo* when such prodrug is administered to a mammalian subject. The modifications typically are achieved by synthesising the parent compound with a prodrug substituent. Prodrugs include compounds of the invention wherein a hydroxyl, amino, sulfhydryl, carboxy or carbonyl group in a compound of the invention is bonded to any group that may be cleaved *in vivo* to regenerate the free hydroxyl, amino, sulfhydryl, carboxy or carbonyl group, respectively.

Examples of prodrugs include, but are not limited to, esters and carbamates of hydroxy functional groups, esters groups of carboxyl functional groups, N-acyl derivatives and N-Mannich bases. General information on prodrugs may be found e.g. in Bundegaard, H. "Design of Prodrugs" p. I-92, Elesevier, New York-Oxford (1985).

Compounds of the invention may contain double bonds and may thus exist as E (*entgegen*) and Z (*zusammen*) geometric isomers about each individual double bond. Positional isomers may also be embraced by the compounds of the invention. All such isomers (e.g. if a compound of the invention incorporates a double bond or a fused ring, the cis- and trans- forms, are embraced) and mixtures thereof are included within the scope of the invention (e.g. single positional isomers and mixtures of positional isomers may be included within the scope of the invention).

Compounds of the invention may also exhibit tautomerism. All tautomeric forms (or tautomers) and mixtures thereof are included within the scope of the invention. The term "tautomer" or "tautomeric form" refers to structural isomers of different energies which are interconvertible *via* a low energy barrier. For example, proton tautomers (also known as prototropic tautomers) include interconversions *via* migration of a proton, such as keto-enol and imine-enamine isomerisations. Valence tautomers include interconversions by reorganisation of some of the bonding electrons.

Compounds of the invention may also contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person.

All stereoisomers (including but not limited to diastereoisomers, enantiomers and atropisomers) and mixtures thereof (e.g. racemic mixtures) are included within the scope of the invention.

In the structures shown herein, where the stereochemistry of any particular chiral atom is not specified, then all stereoisomers are contemplated and included as the compounds of the invention. Where stereochemistry is specified by a solid wedge or dashed line representing a particular configuration, then that stereoisomer is so specified and defined.

The compounds of the present invention may exist in unsolvated as well as solvated forms with pharmaceutically acceptable solvents such as water, ethanol, and the like, and it is intended that the invention embrace both solvated and unsolvated forms.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature). All isotopes of any particular atom or element as specified herein are contemplated within the scope of the compounds of the invention. Exemplary isotopes that can be incorporated into compounds of the invention include isotopes of hydrogen, carbon, nitrogen, oxygen, phosphorus, sulfur, fluorine, chlorine and iodine, such as ²H, ³H, ¹¹C, ¹³C, ¹⁴C, ¹³N, ¹⁵O, ¹⁷O, ¹⁸O, ³²P, ³³P, ³⁵S, ¹⁸F, ³⁶Cl, ¹²³I, and ¹²⁵I. Certain isotopically-labeled compounds of the present invention (e.g., those labeled with ³H and ¹⁴C) are useful in compound and for substrate tissue distribution assays. Tritiated (³H) and carbon-I4 (¹⁴C) isotopes are useful for their ease of preparation and detectability. Further, substitution with heavier isotopes such as deuterium (i.e., ²H may afford certain therapeutic advantages resulting from greater metabolic stability (e.g., increased *in vivo* half-life or reduced dosage requirements) and hence may be preferred in some circumstances. Positron emitting isotopes such as ¹⁵Q, ¹³N, ¹¹C and ¹⁸F are useful for positron emission tomography (PET) studies to examine substrate receptor occupancy. Isotopically labeled compounds of the present invention can generally be prepared by following procedures analogous to those disclosed in the Scheme 1 and/or in the Examples herein below, by substituting an isotopically labeled reagent for a non-isotopically labeled reagent.

Unless otherwise specified, C_{1-q} alkyl groups (where q is the upper limit of the range) defined herein may be straight-chain or, when there is a sufficient number (i.e. a minimum of two or three, as appropriate) of carbon atoms, be branched-chain, and/or cyclic (so forming a C_{3-q}-cycloalkyl group). Such cycloalkyl groups may be monocyclic or bicyclic and may further be bridged. Further, when there is a sufficient number (i.e. a minimum of four) of carbon atoms, such groups may also be part cyclic. Such alkyl groups may also be saturated or, when there is a sufficient number (i.e. a minimum of two) of carbon atoms, be unsaturated (forming, for example, a C_{2-q} alkenyl or a C_{2-q} alkynyl group).

Unless otherwise stated, the term C_{1-q} alkylene (where q is the upper limit of the range) defined herein may be straight-chain or, when there is a sufficient number of carbon atoms, be saturated or unsaturated (so forming, for example, an alkenylene or alkynylene linker group). However, such C_{1-q} alkylene groups may not be branched.

C_{3-q} cycloalkyl groups (where q is the upper limit of the range) that may be specifically mentioned may be monocyclic or bicyclic alkyl groups, which cycloalkyl groups may further be bridged (so forming, for example, fused ring systems such as three fused cycloalkyl groups). Such cycloalkyl groups may be saturated or unsaturated containing one or more double bonds (forming for example a cycloalkenyl group). Substituents may be attached at any point on the cycloalkyl group. Further, where there is a sufficient number (i.e. a minimum of four) such cycloalkyl groups may also be part cyclic. For the avoidance of doubt, cycloalkylene refers specifically to cyclic alkylene groups, i.e. those cycloalkylene groups are linked to the the remainder of the compound of formula I.

Hence "alkylene" and "cycloalkylene" groups may be appropriate linker groups that are linked at an appropriate point to the compound of formula I. For the avoidance of doubt, any optional substituents on the alkylene/cycloalkylene groups are not an integral part of the linking moiety, i.e. when "X^{1a}" represents substituted cycloalkylene, then the substituent(s) are not linked to "T^{1a}" or "X^{1b}", but are located on the cycloalkylene moiety.

The term "halo", when used herein, preferably includes fluoro, chloro, bromo and iodo.

Heterocycloalkyl groups that may be mentioned include non-aromatic monocyclic and bicyclic heterocycloalkyl groups in which at least one (e.g. one to four) of the atoms in the ring system is other than carbon (i.e. a heteroatom), and in which the total number of atoms in the ring system is between 3 and 20 (e.g. between three and ten, e.g between 3 and 8, such as 5- to 8-). Such heterocycloalkyl groups may also be bridged. Further, such heterocycloalkyl groups may be saturated or unsaturated containing one or more double and/or triple bonds, forming for example a C_{2-q} heterocycloalkenyl (where q is the upper limit of the range) group. C_{2-q} heterocycloalkyl groups that may be mentioned include 7-azabicyclo[2.2.1]heptanyl, 6-azabicyclo[3.1.1 ]heptanyl, 6-azabicyclo[3.2.1]-octanyl, 8-azabicyclo-[3.2.1]octanyl, aziridinyl, azetidinyl, dihydropyranyl, dihydropyridyl, dihydropyrrolyl (including 2,5-dihydropyrrolyl), dioxolanyl (including 1,3-dioxolanyl), dioxanyl (including 1,3-dioxanyl and 1,4-dioxanyl), dithianyl (including 1,4-dithianyl), dithiolanyl (including 1,3-dithiolanyl), imidazolidinyl, imidazolinyl, morpholinyl, 7-oxabicyclo[2.2.1]heptanyl, 6-oxabicyclo-[3.2.1]octanyl, oxetanyl, oxiranyl, piperazinyl, piperidinyl, pyranyl, pyrazolidinyl, pyrrolidinonyl, pyrrolidinyl, pyrrolinyl, quinuclidinyl, sulfolanyl, 3-sulfolenyl, tetrahydropyranyl, tetrahydrofuranyl, tetrahydropyridyl (such as 1,2,3,4-tetrahydropyridyl and 1,2,3,6-tetrahydropyridyl), thietanyl, thiiranyl, thiolanyl, thiomorpholinyl, trithianyl (including 1,3,5-trithianyl), tropanyl and the like. Substituents on heterocycloalkyl groups may, where appropriate, be located on any atom in the ring system including a heteroatom. The point of attachment of heterocycloalkyl groups may be *via* any atom in the ring system including (where appropriate) a heteroatom (such as a nitrogen atom), or an atom on any fused carbocyclic ring that may be present as part of the ring system. Heterocycloalkyl groups may also be in the *N*- or S- oxidised form. Heterocycloalkyl mentioned herein may be stated to be specifically monocyclic or bicyclic.

Heterocycloalkylene refers to a heterocycloalkyl group that is a part of a linker group, i.e. -heterocycloalkyl-, where the hyphens represent an appropriate point of attachment. The point of attachment may be *via* any appropriate atom (e.g. a nitrogen or carbon atom of that heterocycloalkyl moiety). Where it is indicated that such a moiety may be substituted, the point of attachment (to the remainder of the compound of formula I) may not be *via* the optional substituents, e.g. when X^{1a} represents substituted -heterocycloalkylene-, then those substituents are not directly linked to "T^{1a}" or "X^{1b}".

For the avoidance of doubt, the term "bicyclic" (e.g. when employed in the context of heterocycloalkyl groups) refers to groups in which the second ring of a two-ring system is formed between two adjacent atoms of the first ring. The term "bridged" (e.g. when employed in the context of cycloalkyl or heterocycloalkyl groups) refers to monocyclic or bicyclic groups in which two non-adjacent atoms are linked by either an alkylene or heteroalkylene chain (as appropriate).

Aryl groups that may be mentioned include C₆₋₂₀, such as C₆₋₁₂ (e.g. C₆₋₁₀) aryl groups. Such groups may be monocyclic, bicyclic or tricyclic and have between 6 and 12 (e.g. 6 and 10) ring carbon atoms, in which at least one ring is aromatic. C₆₋₁₀ aryl groups include phenyl, naphthyl and the like, such as 1,2,3,4-tetrahydronaphthyl. The point of attachment of aryl groups may be *via* any atom of the ring system. For example, when the aryl group is polycyclic the point of attachment may be *via* atom including an atom of a non-aromatic ring. However, when aryl groups are polycyclic (e.g. bicyclic or tricyclic), they are preferably linked to the rest of the molecule *via* an aromatic ring.

Arylene refers to an aryl group that is a part of a linker group, i.e. -aryl-, where the hyphens represent an appropriate point of attachment (see also definition of "heterocycloalkylene").

Unless otherwise specified, the term "heteroaryl" when used herein refers to an aromatic group containing one or more heteroatom(s) (e.g. one to four heteroatoms) preferably selected from N, O and S. Heteroaryl groups include those which have between 5 and 20 members (e.g. between 5 and 10) and may be monocyclic, bicyclic or tricyclic, provided that at least one of the rings is aromatic (so forming, for example, a mono-, bi-, or tricyclic heteroaromatic group). When the heteroaryl group is polycyclic the point of attachment may be *via* atom including an atom of a non-aromatic ring. However, when heteroaryl groups are polycyclic (e.g. bicyclic or tricyclic), they are preferably linked to the rest of the molecule *via* an aromatic ring. Heteroaryl groups that may be mentioned include 3,4-dihydro-1*H*-isoquinolinyl, 1,3-dihydroisoindolyl, 1,3-dihydroisoindolyl (e.g. 3,4-dihydro-1*H*-isoquinolin-2-yl, 1,3-dihydroisoindol-2-yl, 1,3-dihydroisoindol-2-yl; i.e. heteroaryl groups that are linked *via* a non-aromatic ring), or, preferably, acridinyl, benzimidazolyl, benzodioxanyl, benzodioxepinyl, benzodioxolyl (including 1,3-benzodioxolyl), benzofuranyl, benzofurazanyl, benzothiadiazolyl (including 2,1,3-benzothiadiazolyl), benzothiazolyl, benzoxadiazolyl (including 2,1,3-benzoxadiazolyl), benzoxazinyl (including 3,4-dihydro-2*H*-1,4-benzoxazinyl), benzoxazolyl, benzomorpholinyl, benzoselenadiazolyl (including 2,1,3-benzoselenadiazolyl), benzothienyl, carbazolyl, chromanyl, cinnolinyl, furanyl, imidazolyl, imidazo[1,2-a]pyridyl, indazolyl, indolinyl, indolyl, isobenzofuranyl, isochromanyl, isoindolinyl, isoindolyl, isoquinolinyl, isothiaziolyl, isothiochromanyl, isoxazolyl, naphthyridinyl (including 1,6-naphthyridinyl or, preferably, 1,5-naphthyridinyl and 1,8-naphthyridinyl), oxadiazolyl (including 1,2,3-oxadiazolyl, 1,2,4-oxadiazolyl and 1,3,4-oxadiazolyl), oxazolyl, phenazinyl, phenothiazinyl, phthalazinyl, pteridinyl, purinyl, pyranyl, pyrazinyl, pyrazolyl, pyridazinyl, pyridyl, pyrimidinyl, pyrrolyl, quinazolinyl, quinolinyl, quinolizinyl, quinoxalinyl, tetrahydroisoquinolinyl (including 1,2,3,4-tetrahydroisoquinolinyl and 5,6,7,8-tetrahydroisoquinolinyl), tetrahydroquinolinyl (including 1,2,3,4-tetrahydroquinolinyl and 5,6,7,8-tetrahydroquinolinyl), tetrazolyl, thiadiazolyl (including 1,2,3-thiadiazolyl, 1,2,4-thiadiazolyl and 1,3,4-thiadiazolyl), thiazolyl, thiochromanyl, thiophenetyl, thienyl, triazolyl (including 1,2,3-triazolyl, 1,2,4-triazolyl and 1,3,4-triazolyl) and the like. Substituents on heteroaryl groups may, where appropriate, be located on any atom in the ring system including a heteroatom. The point of attachment of heteroaryl groups may be *via* any atom in the ring system including (where appropriate) a heteroatom (such as a nitrogen atom), or an atom on any fused carbocyclic ring that may be present as part of the ring system. Heteroaryl groups may also be in the N- or S- oxidised form. Heteroaryl groups mentioned herein may be stated to be specifically monocyclic or bicyclic. When heteroaryl groups are polycyclic in which there is a non-aromatic ring present, then that non-aromatic ring may be substituted by one or more =O group.

Heteroarylene refers to an aryl group that is a part of a linker group, i.e. -heteroaryl-, where the hyphens represent an appropriate point of attachment (see also definition of "heterocycloalkylene").

It may be specifically stated that the heteroaryl group is monocyclic or bicyclic. In the case where it is specified that the heteroaryl is bicyclic, then it may be consist of a five-, six- or seven-membered monocyclic ring (e.g. a monocyclic heteroaryl ring) fused with another a five-, six- or seven-membered ring (e.g. a monocyclic aryl or heteroaryl ring).

Heteroatoms that may be mentioned include phosphorus, silicon, boron and, preferably, oxygen, nitrogen and sulfur.

For the avoidance of doubt, where it is stated herein that a group (e.g. a C₁₋₁₂ alkyl group) may be substituted by one or more substituents (e.g. selected from E⁶), then those substituents (e.g. defined by E⁶) are independent of one another. That is, such groups may be substituted with the same substituent (e.g. defined by E⁶) or different substituents (defined by E⁶).

For the avoidance of doubt, in cases in which the identity of two or more substituents in a compound of the invention may be the same, the actual identities of the respective substituents are not in any way interdependent. For example, in the situation in which there is more than one e.g. Q^{1a}, Q^{1b} or E¹ to E⁹ (such as E⁶) substituent present, then those Q^{1a}, Q^{1b} or E¹ to E⁹ (e.g. E⁶) substituents may be the same or different. Further, in the case where there are e.g. Q^{1a}, Q^{1b} or E¹ to E⁹ (such as E⁶) substituents present, in which one represents -OR^{10a} (or e.g. -OR²⁰, as appropriate) and the other represents -C(O)₂R^{10a} (or e.g. -C(O)₂R²⁰, as appropriate), then those R^{10a} or R²⁰ groups are not to be regarded as being interdependent. Also, when e.g. there are two -OR^{10a} substituents present, then those -OR^{10a} groups may be the same or different (i.e. each R^{10a} group may be the same or different).

For the avoidance of doubt, when a term such as "*E¹ to* E*⁹*" is employed herein, this will be understood by the skilled person to mean E¹, E², E³, E⁴, E⁵, E⁶, E⁷, E⁸ and E⁹, inclusively. The term "*B¹ to B⁴*" as employed herein will be understood to mean B¹, B^{1a}, B², B^{2a}, B³, B^{3a}, B⁴ and B^{4a}, inclusively.

All individual features (e.g. preferred features) mentioned herein may be taken in isolation or in combination with any other feature (including preferred feature) mentioned herein (hence, preferred features may be taken in conjunction with other preferred features, or independently of them).

The skilled person will appreciate that compounds of the invention that are the subject of this invention include those that are stable. That is, compounds of the invention include those that are sufficiently robust to survive isolation from e.g. a reaction mixture to a useful degree of purity.

The skilled person will appreciate that the bicyclic core of the compounds of the invention (containing A₁, A₄, A₄ₐ and A₅) is/are aromatic. It is further stated herein that at least one of A₄ and A₄ₐ does not represent N, i.e. that at least one of C(R^{1a}) or C(R^{1b}) is present. Both C(R^{1a}) and C(R^{1b}) may also be present, and, at least one of R^{1a} and R^{1b} is present that represents -T¹-Z¹ (this is an essential feature).

In compounds of the invention, when either T^{1a} and T^{1b} represent C₁₋₁₂ alkylene optionally substituted by one or more substituents selected from Q^{1a}, then the Q^{1a} substituent may be cyclic (e.g. C₃₋₁₂ cycloalkyl or heterocycloalkyl) in which the Q^{1a} is attached to a single carbon atom of that C₁₋₁₂ alkylene group.

For the avoidance of doubt, the requisite bicycle of formula I refers to the aromatic bicycle containing the integers A¹, A⁴, A^{4a} and A⁵ (as well as the further two nitrogen atoms and three carbon atoms). The following requisite bicycles of formula I are particularly preferred: 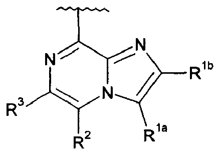 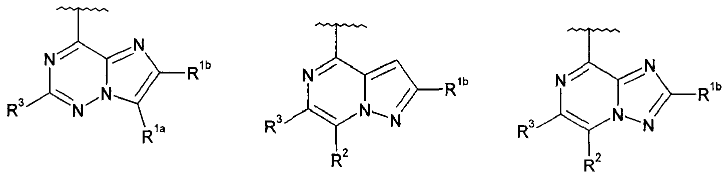 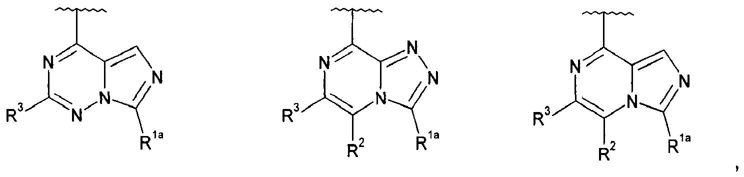 in which R^{1a}, R^{1b}, R² and R³ are as hereinbefore defined, and the squiggly lines represent the point of attachment of the bicyclic heteroaryl group (of formula I) to the requisite (optionally substituted) morpholinyl group of the compound of formula I. The preferred heterobicyclic cores depicted above represent an embodiment of the invention in which A^{4a} represents C(R^{1a}) (which is particularly preferred) and an embodiment of the invention in which A^{4a} represents N. In further, more specific embodiments of the invention, the compounds of the invention may represent any one (or more) of the specific bicyclic heteroaryl groups (in which A^{4a} represents either C(R^{1a}) or N) depicted above. It is preferred that at least one of A₁, A₄ and A₄ₐ represents N and hence, it is also preferred that a total of one or two (e.g. one) of A₁, A₄ and A₄ₐ represents N (and preferably a total of one or two (e.g. one) of A₁, A₄, A₄ₐ and A₅ represents N). Hence, the following bicycles are possible:
(a) A₁ represents N, A₄ represents C(R^{1a}), A₄ₐ represents C(R^{1b}) and A₅ represents C(R²);
(b) A₁ represents N, A₄ represents N, A₄ₐ represents C(R^{1b}) and A₅ represents C(R²);
(c) A₁ represents N, A₄ represents C(R^{1a}), A₄ₐ represents C(R^{1b}) and A₅ represents N;
(d) A₁ represents C(H), A₄ represents N, A₄ₐ represents C(R^{1b}) and A₅ represents C(_{R}²);
(e) A₁ represents N, A₄ represents C(R^{1a}), A₄ₐ represents N and A₅ represents C(R²);
(f) A₁ represents C(H), A₄ represents C(R^{1a}), A₄ₐ represents N and A₅ represents N;
(g) A₁ represents C(H), A₄ represents C(R^{1a}), A₄ₐ represents N and A₅ represents C(R²).

The most preferred of the above bicycles are (a), (b), (c), (d), (f) and (g).

The most preferred requisite bicycles of formula I are: 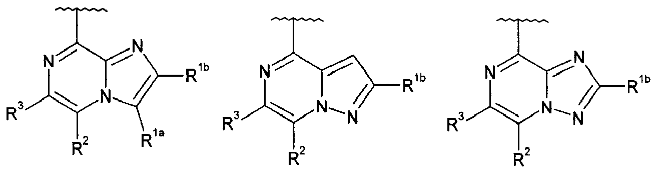 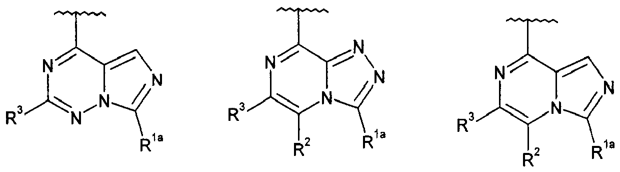 wherein the integers are as defined herein.

The preferred bicycles depicted above may exist in different forms, for example as the following alternative aromatic bicycle: 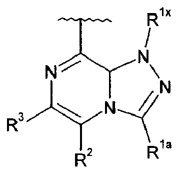 wherein R^{1a}, R² and R³ are as hereinbefore defined, and R^{1x} may represent hydrogen.

The most preferred compounds of the invention are those represented by the following formula:

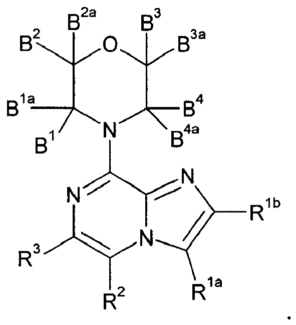

Compounds of the invention that may be mentioned include those in which, for example when A₁ and A₄ₐ both represent N, and A₄ and A₅ respectively represent C(R^{1a}) and C(R²) (and hence, the requisite bicyclic core of the compound of formula I is a [1,2,4]-triazolo[4,3-a]pyrazine), preferably, R³ does not represent phenyl (e.g. optionally substituted, preferably unsubstituted, phenyl).

It is stated hereinbefore that compounds of the invention may represent certain Z¹ groups, i.e. hydroxamic acids (e.g. certain -C(=Y)N(R^{10a})-OR^{11c} groups) or isosteres thereof. The skilled person will appreciate which isosteres may achieve the desired result and certain isosteres are specifically mentioned hereinbefore.
In the Z¹ groups of compounds of the invention, it is preferred that:
R^{10a} represents H;
R^{11c} represents C₁₋₃ alkyl or preferably, H;
W^{x1}, W^{x2} and W^{x3} independently represent O;
R³¹ represents H;
R³³ represents H;
R³² and R³⁴ are independently hydrogen, hydroxyl or an unsubstituted C₁₋₄ alkyl group, provided that if both R³² and R³⁴ are present, one of R³² and R³⁴ represents hydroxy and the other represents hydrogen;
if Y₂ is absent, R³⁴ represents hydroxy;
R¹¹ and R¹² are independently selected from hydrogen and an unsubstituted C₁₋₄ alkyl group (more preferably, these groups represent hydrogen);
R¹³, R¹⁴ and R¹⁵ are independently selected from hydrogen, hydroxy, -NH₂, halo, -OC₁₋₄alkyl, -N(H)(C₁₋₄alkyl), -N(C₁₋₄alkyl)₂, -CF₃, -CN, -NO₂, -S(O)₂-C₁₋₄alkyl, -C(O)C₁₋₄alkyl, C₁₋₄ alkyl (optionally substituted by one or more fluoro atoms), heterocycloalkyl (optionally substituted by one or more substituents selected from halo and C₁₋₃ alkyl), aryl or heteroaryl (which latter two groups are optionally substituted by one or more substituents selected from halo and d₁₋₃ alkyl);
more preferably, R¹³, R¹⁴ and R¹⁵ are independently selected from hydrogen.

The most preferred Z¹ group (of the compounds of the invention) is -C(=Y)N(R^{10a})-OR^{11c}.

Preferred compounds of the invention that may be mentioned include those in which:
R^{1b} represents -T¹-Z¹;
if present, R^{1a} represents R^{1c};
R^{1c} represents hydrogen or a substituent selected from halo and optionally substituted C₁-₃ alkyl;
R^{1c} is preferably not optionally substituted C₁₋₃ alkyl;
preferably, R^{1c} represents halo (e.g. fluoro) when it is a substituent;
most preferably, R^{1c} represents hydrogen.

More preferred compounds of the invention that may be mentioned include those in which:
T^{1a} represents a direct bond or, preferably, C₁₋₈ (e.g. C₁₋₆) alkylene (preferably unsubstituted);
T^{1b} represents C₁₋₈ (e.g. C₁₋₆) alkylene (preferably unsubstituted) or, preferably, a direct bond;
X^{1a} represents a direct bond, -N(R^{t1})-, -C(O)N(R^{t2})-, -N(R^{t3})C(O)-, -O-, -S-, heterocycloalkylene (which group preferably does not comprise a further ring, and which group is preferably unsubstituted) or heteroarylene (which group is preferably unsubstituted);
X^{1b} represents a direct bond, -N(R^{t1}) or heteroalkylene (which group is preferably unsubstituted);
Z¹ represents -C(O)N(R^{10a})-OR^{11c};
R^{10a} represents hydrogen;
R^{11c} represents hydrogen.

Preferred linker groups defined by "*-T^{1a}-X^{1a}-X^{1b}-T^{1b}-*" include: 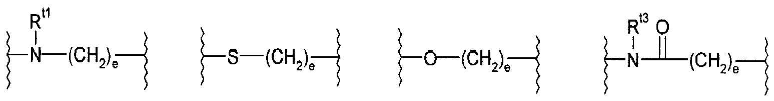 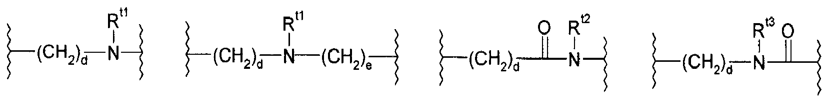 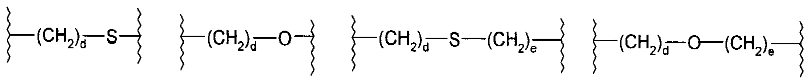 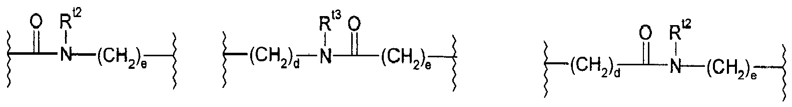 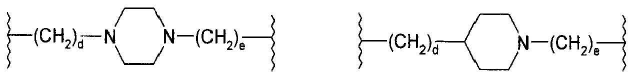 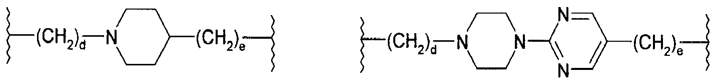 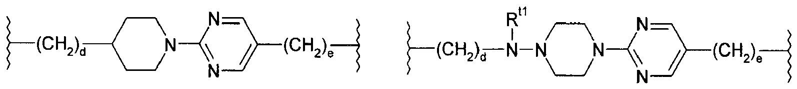 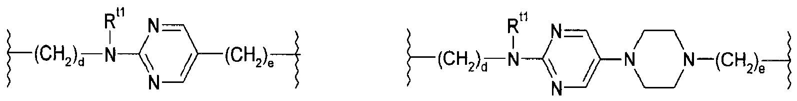 in which d and e are each independently 0, 1, 2, 3, 4, 5, 6, 7 or 8 (e.g. 0, 1, 2, 3 or 4); each R^{t1}, R^{t2} and R^{t3} independently represent hydrogen or C₁₋₈ (e.g. C₁₋₄) alkyl (and, preferably such groups independently represent hydrogen or methyl) (and wherein the first squiggly line represents the point of attachment to the requisite bicycle of formula I and the second squiggly line represents the point of attachment to the Z¹ group).

Preferred linker groups ("*-T^{1a}-X^{1a}-X^{1b}-T^{1b}-*") of compounds of the invention include those in which:
T^{1a} represents a direct bond or, preferably C₁₋₈ alkylene (e.g. C₁₋₂ alkylene, such as -CH₂-);
X^{1a} represents heteroarylene or, preferably, -N(R^{t1})- or heterocycloalkylene; when X^{1a} represents heterocycloalkylene, it is preferably a 5- or 6-membered ring (more preferably a 6-membered ring) preferably containing one or two heteroatoms (e.g. selected from nitrogen), e.g. piperidinyl or piperazinyl, which groups may be linked to T^{1a} and X^{1b} *via* a nitrogen or carbon atom (so forming, e.g. a 1-piperidinyl, 4-piperidinyl or 1-piperazinyl linking moiety), such as: 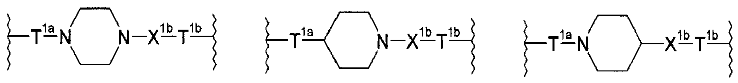 when X^{1a} represents heterocycloalkylene, then that group is preferably unsubstituted (and preferably does not comprise a further ring, as defined by Z^{a});
X^{1b} represents a direct bond or heteroarylene;
when X^{1a} or X^{1b} represents heteroarylene, then that group is preferably a 5- or 6-membered heteroaryl moiety (more preferably a 6-membered ring) preferably containing one or, especially, two heteroatoms (e.g. nitrogen heteroatoms) so forming e.g. a pyrimidinyl moiety, preferably linked to X^{1a} and T^{1b} *via* carbon atoms, so forming e.g. 2-pyrimidinyl linking moiety, such as: 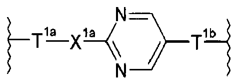
when X^{1a} or X^{1b} represent heteroarylene, then that group is preferably unsubstituted.

Particularly preferred linker groups ("*-T^{1a}-X^{1a}-X^{1b}-X^{1b}-*") of compounds of the invention include those in which: 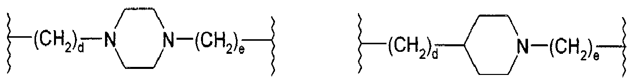 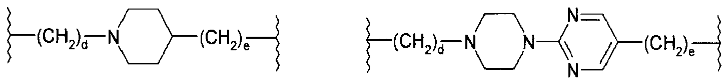 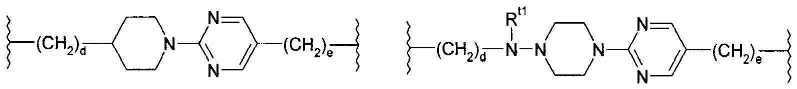 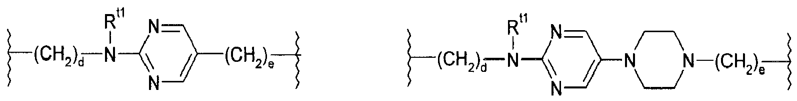 in which preferably:
d represents 1;
e represents 0;
R^{t1} represents hydrogen or C₁₋₂ alkyl (e.g. methyl).

Especially preferred linker groups ("*-T^{1a}-X^{1a}-X^{1b}-T^{1b}-*") of compounds of the invention include those in which: 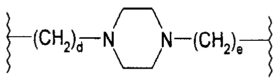 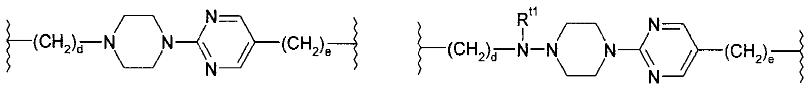 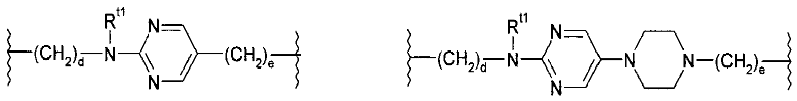 and more preferably: 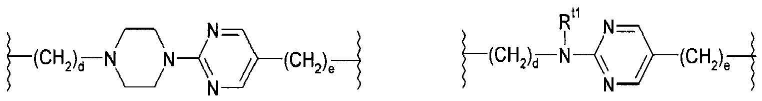 in which preferably:
d represents 1;
e represents 0;
R^{t1} represents hydrogen or C₁₋₂ alkyl (e.g. methyl).

Compounds of the invention that may be mentioned include those in which, for example when A₁ and A₄ₐ represent N, A₅ represents C(R²) and A₄ represents C(R^{1a}) then, R^{1a} preferably does not represent -OR^{10a} in which R^{10a} represents H.

Further compounds of the invention that may be mentioned include those in which, for example when A₁ represents N, A₄ represents C(R^{1a}), A₄ₐ represents N and A₅ represents C(R²), then R³ does not represent aryl (e.g. phenyl), especially unsubstituted aryl/phenyl.

When X^{1a} or X^{1b} represent a cycloalkylene/heterocycloalkylene group comprising a further ring (defined by Z^{a} or Z^{b}), then the following group may be formed: 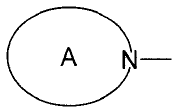 in which:
(a) ring A is a first 3- to 7-membered saturated N-containing heterocyclic ring which is fused to a second ring as hereinbefore defined to form a heteropolycyclic ring system in which the first ring is selected from, but not limited to, azetidine, pyrrolidine, piperidine, piperazine, morpholine, thiomorpholine and homopiperazine, said group being fused to a second ring as hereinbefore defined. The second ring is typically a 3- to 7-membered saturated N-containing heterocyclic ring as defined above in respect of the first ring, or the second ring is a 5- to 12-membered unsaturated heterocyclic group. More typically the second ring is a 5-, 6-or 7- membered saturated N-containing heterocyclic ring or a 5- to 7-membered unsaturated heterocyclic ring. Typical examples of the second ring include azetidine, pyrrolidine, piperidine, piperazine, morpholine, thiomorpholine, homopiperazine, pyrrole, imidazole, pyridine, pyridazine, pyrimidine, pyrazine, tetrahydrofuran and tetrahydropyran. Examples of the resulting heteropolycyclic system include octahydropyrrolo[1,2-a]pyrazine and octahydro-pyrrolo[3,4-c]pyrrole. Specific examples of the heteropolycyclic system include the following structures: 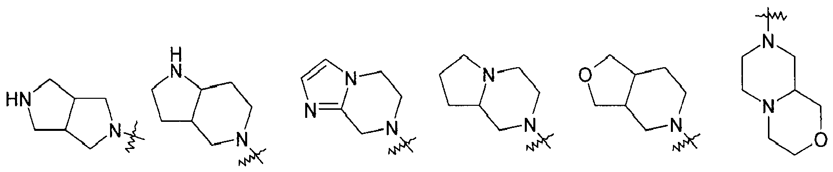
(b) ring A is a first 3- to 7-membered saturated containing heterocyclic group as hereinbefore defined, which includes, but is not limited to, a bridgehead group (i.e. a linker group linking any two non-adjacent atoms of the first ring), thereby forming, for example 3,8-diaza-bicyclo[3.2.1]octane, 2,5-diaza-bicyclo [2.2.1]heptane, 8-aza-bicyclo[3.2.1]octane, 2-azabicyclo[2.2.1 ]heptane, 3,6-diaza-bicyclo[3.1.1]heptane, 6-azabicyclo[3.1.1]heptane, 3,9-diaza-bicyclo[4.2.1]nonane and/or 2-oxa-7,9-diazabicyclo[3.3.1]nonane. Specific examples of this group include the following structures: 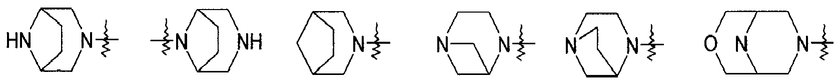
(c) ring A is a first 3- to 7-membered saturated N-containing heterocyclic group as hereinbefore defined, which is spiro-fused at any available ring carbon atom to a second 3- to 12- membered saturated carbocyclic ring, typically to a 3- to 6- membered saturated carbocyclic ring, or to a 4- to 7-membered saturated N-containing heterocyclic group. Examples include a group in which the first ring is selected from azetidine, pyrrolidine, piperidine and piperazine which is spiro-fused at a ring carbon atom to a second ring selected from cyclopropane, cyclobutane, cyclopentane, cyclohexane, azetidine, pyrrolidine, piperidine, piperazine and tetrahydropyran. The ring so formed may, for instance, be a group derived from 3,9-diazaspiro[5.5]undecane, 2,7-diazaspiro[3.5]nonane, 2,8-diazaspiro[4.5]decane or 2,7-diazaspiro[4.4]nonane. Specific examples of such groups include the following structures:

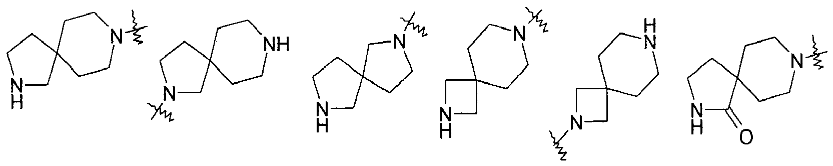

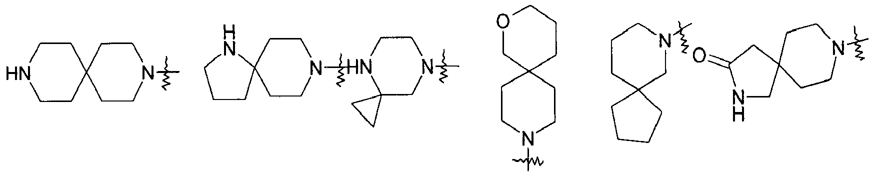

Exemplary embodiments of R³ include, but are not limited to: pyrrole, pyrazole, triazole, tetrazole, thiazole, isothiazole, oxazole, isoxazole, isoindole, 1,3-dihydro-indol-2-one, pyridine-2-one, pyridine, pyridine-3-ol, imidazole,1H-indazole, 1 H-indole, indolin-2-one, 1-(indolin-1-yl)ethanone, pyrimidine, pyridazine, pyrazine and isatin groups. 1H-benzo[d][1,2,3]triazole, 1H-pyrazolo [3,4-b]pyridine, 1 H-pyrazolo[3,4-d]pyrimidine, 1 H-benzo[d]imidazole, 1 H-benzo[d]imidazol-2(3H)-one, 1H-pyrazolo[3,4-c]pyridine, 1H-pyrazolo[4,3-d]pyrimidine, 5H-pyrrolo[3,2-d]pyrimidine, 2-amino-1H-purin-6(9H)-one, quinoline, quinazoline, quinoxaline, isoquinoline, isoquinolin-1 (2H)-one, 3,4-dihydroisoquinolin-1 (2H)-one, 3,4-dihydroquinolin-2(1H)-one, quinazolin-2(1H)-one, quinoxalin-2(1H)-one, 1,8-napthyridine, pyrido[3,4-d]pyrimidine, and pyrido[3,2-b]pyrazine, 1,3-dihydro benzimidazolone, benzimidazole, benzothiazole and benzothiadiazole, groups. These groups may be unsubstituted or substituted.

The attachment site of the R³ group to the relevant carbon atom of the requisite bicyclic A₁, A₄, A₄ₐ and A₅-containing ring of formula I may preferably be *via* any carbon of the R³ group (carbon-linked).

More exemplary embodiments of R³ include, but are not limited to, the following groups, where the wavy line indicates the site of attachment to the requisite bicyclic core of formula I:

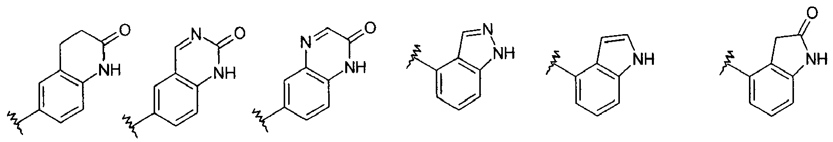

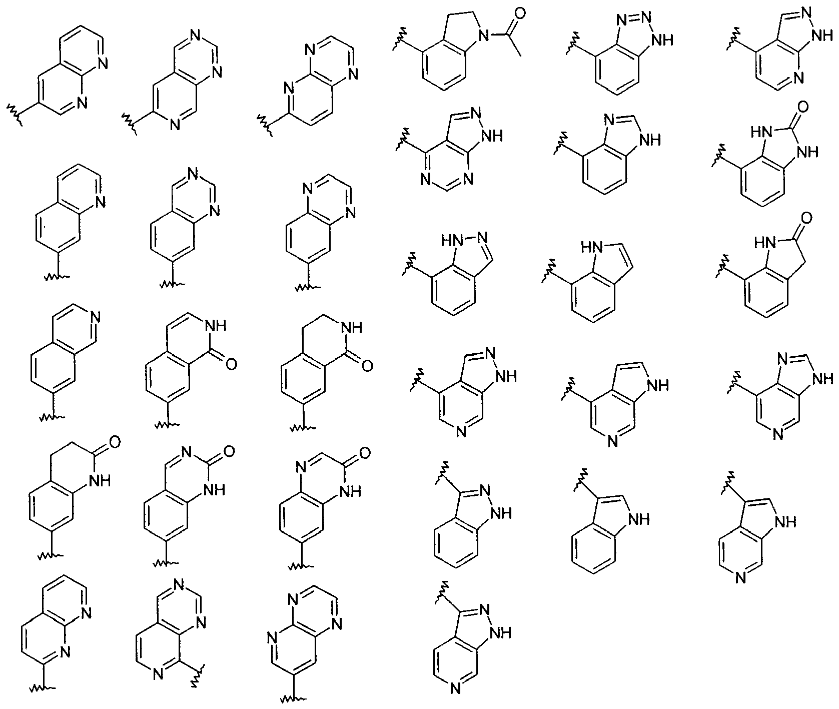

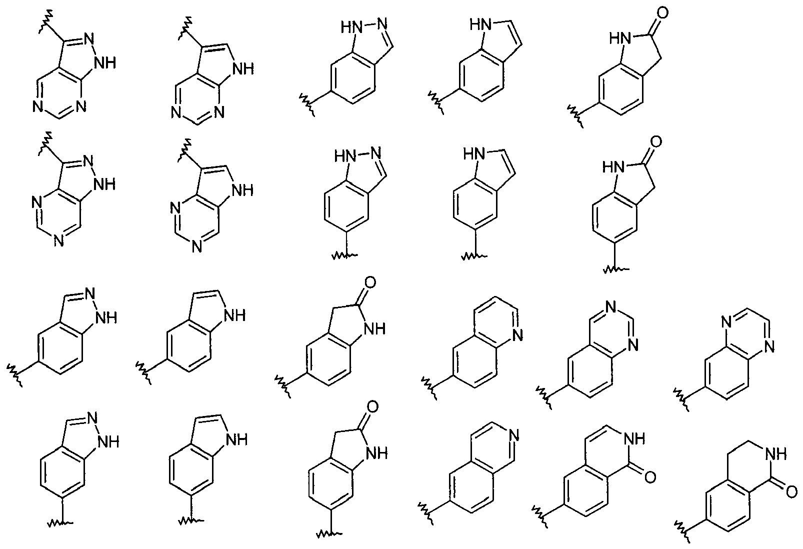

Preferred compounds of the invention include those in which:
when R³ represents aryl (e.g. phenyl), then that group may be unsubstituted but is preferably substituted by at least one (e.g. two or, preferably, one) substituent(s) selected from E⁴;
when R³ represents monocyclic heteroaryl (e.g. a 5- or 6-membered heteroaryl group), then that group preferably contains 1, 2, 3 or 4 nitrogen atoms and, optionally 1 or 2 additional heteroatoms selected from oxygen and sulfur, and
which heteroaryl group is optionally substituted by one or more substituents selected from E⁴ (preferably, such monocyclic heteroaryl groups preferably contain a maximum of four heteroatoms);
when R³ represents bicyclic heteroaryl (e.g. a 8-, 9- or 10-membered heteroaryl group), then that group preferably consists of a 5- or 6-membered ring fused to another 5- or 6-membered ring (in which either one of those rings may contain one or more (e.g. four, or, preferably one to three) heteroatoms), in which the total number of heteroatoms is preferably one to four, and which ring is optionally substituted by one or more (e.g. two or, preferably, one) substituent(s) selected from E⁴ (and, if there is a non-aromatic ring present in the bicyclic heteroaryl group, then such a group may also be substituted by one or more (e.g. one) =O groups);
optional substituents (e.g. the first optional substituent) on the R³ group (e.g. when it represents aryl, such as phenyl) are preferably selected from -OR, -SR, -CH₂OR, CO₂R CF₂OH, CH(CF₃)OH, C(CF₃)₂OH, -(CH₂)_{w}OR, -(CH₂)_{w}NR₂, -C(O)N(R)₂, -NR₂, -NRC(O)R, -NRC(O)NHR, -NRC(O)N(R)₂, -S(O)_{y}N(R)₂, -OC(O)R, OC(O)N(R)₂, -NRS(O)_{y}R, -NRC(O)N(R)₂, CN, halogen and -NO₂ (in which each R is independently selected from H, C₁-C₆ alkyl, C₃-C₁₀ cycloalkyl and a 5- to 12-membered aryl or heteroaryl group, the groups being unsubstituted or substituted (for example by one or more substituents as defined herein, e.g. substituents on E⁴ moieties, e.g. =O, J², J³, J⁴ and/or J⁵), w is 0, 1 or 2 and y is 1 or 2);
when R³ represents aryl (e.g. phenyl), then that group is substituted by one or two substituents (e.g. by a first substituent as defined above, and, optionally a further substituent (or a further two substituents) preferably selected from halo, C₁₋₁₂ alkyl, CN, NO₂, OR^{d}, SR^{d}, NR^{d}₂, C(O)R^{d}, SOR^{d}, SO₂R^{d}, SO₂N(R)^{d}₂, NC(O)R^{d} and CO₂R^{d} (wherein each R^{d} is independently H or C₁-C₆ alkyl);
when R³ represents substituted aryl (e.g. phenyl), then the substituent may be situated at the 2-, 3-, 4-, 5- or 6- position of the phenyl ring (typically it is situated at position 3 or 4; particularly preferred are phenyl groups substituted by -OR^{d} (in which R^{d} is independently H or C₁-C₆ alkyl, e.g. methyl), e.g. -OH; in this embodiment the -OR^{d} group, or -OH group, is typically situated at the 3- or 4-position of the phenyl ring, so forming a 3-hydroxyphenyl or 4-hydroxyphenyl group or an isostere thereof, which is unsubstituted or substituted; an isostere as used herein is a functional group which possesses binding properties which are the same as, or similar to, the 3-hydroxyphenyl or 4- hydroxyphenyl group in the context of the compounds of the invention; isosteres of 3-hydroxyphenyl and 4-hydroxyphenyl groups are encompassed within definitions (b) above for R⁵);
when R³ represents heteroaryl, it is unsubstituted or substituted (when substituted, it may be substituted by one or more substitutents selected from those listed in respect of substituents on R³, when R³ is a phenyl group; typically, the substituents are selected from -OC₁₋₆ alkyl and, preferably, OH and NH₂).

Preferred compounds of the invention include those in which:
B¹, B^{1a}, B², B^{2a}, B³, B^{3a}, B⁴ and B^{4a} independently represent hydrogen, C₁₋₆ (e.g. C₁₋₃) alkyl optionally substituted by one or more substituents selected from =O and E¹, or any two of these together form a =O substituent on the morpholinyl ring, or, any two B¹, B^{1a}, B², B^{2a}, B³, B^{3a}, B⁴ and B^{4a} substituents when linked together, may form a linkage, for example between a B² or B^{2a} substituent and a B³ or B^{3a} substituent for a further ring, e.g. a five membered ring such as the one depicted below: 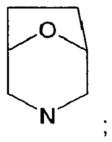
for instance, the B¹ to B⁴ substituted morpholinyl group may represent N-morpholinyl which is unsubstituted or substituted, for instance by one or more B¹ to B⁴ and/or =O substituents;
when it represents substituted morpholinyl, it is preferably selected from the following structures:

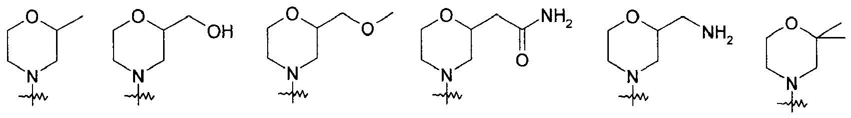

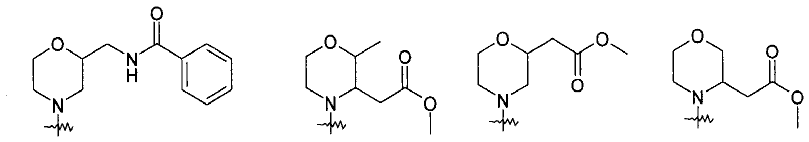

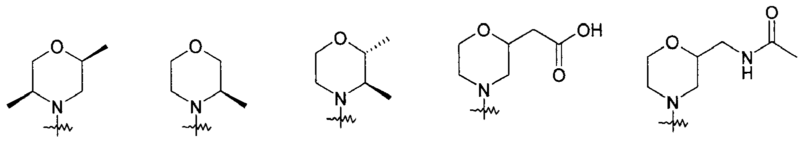

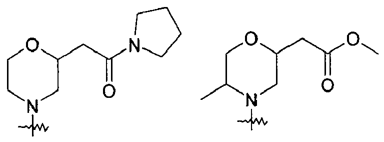

Further preferred compounds of the invention include those in which:
each R^{10a}, R^{11a}, R^{10b}, R^{11b} and R^{12a} independently represent, on each occasion when used herein, hydrogen or C₁₋₁₂ (e.g. C₁₋₆) alkyl (which latter group is optionally substituted by one or more substituents selected from =O and E⁷); or
any relevant pair of R^{10a} and R^{11a} and/or R^{10b} and R^{11b} may, when attached to the same nitrogen atom, be linked together to form (along with the requisite nitrogen atom to which they are attached) a 3- to 12- (e.g. 4- to 12-) membered ring, optionally containing one or more (e.g. one to three) double bonds, and which ring is optionally substituted by one or more substituents selected from E⁹ and =O;
each R^{11c} independently represents C₁₋₁₂ (e.g. C₁₋₆) alkyl (which latter group is optionally substituted by one or more substituents selected from =O and E⁷);
each of E¹, E², E³, E⁴, E⁵, E⁶, E⁷, E⁸ and E⁹ independently represents, on each occasion when used herein, Q⁴ or C₁₋₆ alkyl (e.g. C₁₋₃) alkyl optionally substituted by one or more substituents selected from =O and Q⁵;
each Q⁴ and Q⁵ independently represent halo, -CN, -NO₂, -N(R²⁰)R²¹, -OR²⁰, -C(=Y)-R²⁰, -C(=Y)-OR²⁰, -C(=Y)N(R²⁰)R²¹, -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -S(O)₂R²⁰, -SR²⁰, -S(O)R²⁰ or C₁₋₆ alkyl optionally substituted by one or more fluoro atoms (and each Q⁵ more preferably represents halo, such as fluoro);
any two E¹, E², E³, E⁴, E⁵, E⁶, E⁷, E⁸ or E⁹ groups may be linked together, but are preferably not linked together;
each R²⁰, R²¹, R²² and R²³ independently represent, on each occasion when used herein, aryl (e.g. phenyl; preferably unsubstituted, but which may be substituted by one to three J⁵ groups) or, more preferably, hydrogen or C₁₋₆ (e.g. C₁₋₃) alkyl optionally substituted by one or more substituents selected from =O and J⁴; or
any pair of R²⁰ and R²¹, may, when attached to the same nitrogen atom, be linked together to form a 4- to 8-membered (e.g. 5- or 6-membered) ring, optionally containing one further heteroatom selected from nitrogen and oxygen, optionally containing one double bond, and which ring is optionally substituted by one or more substituents selected from j⁶ and =O;
each R^{21a} independently represents C₁₋₆ (e.g. C₁₋₃) alkyl optionally substituted by one or more substituents selected from =O and J⁴;
each J¹, J², J³, J⁴, J⁵ and J⁶ independently represent C₁₋₆ alkyl (e.g. acyclic C₁₋₃ alkyl or, e.g. in the case of J⁴, C₃₋₅ cycloalkyl) optionally substituted by one or more substituents selected from =O and Q⁸, or, more preferably, such groups independently represent a substituent selected from Q⁷;
each Q⁷ and Q⁸ independently represents a substituent selected from fluoro, -N(R⁵⁰)R⁵¹, -OR⁵⁰, -C(=Y^{a})-R⁵⁰, -C(=Y^{a})-OR⁵⁰, -C(=Y^{a})N(R⁵⁰)R⁵¹, -NR⁵²S(O)₂R⁵⁰, -S(O)₂R⁵⁰ or C₁₋₆ alkyl optionally substituted by one or more fluoro atoms;
each R⁵⁰, R⁵¹, R⁵² and R⁵³ substituent independently represents, on each occasion when used herein, hydrogen or C₁₋₆ (e.g. C₁₋₃) alkyl optionally substituted by one or more substituents selected from fluoro;
when any relevant pair of R⁵⁰, R⁵¹ and R⁵² are linked together, then those pairs that are attached to the same nitrogen atom may be linked together (i.e. any pair of R⁵⁰ and R⁵¹), and the ring so formed is preferably a 5- or 6-membered ring, optionally containing one further nitrogen or oxygen heteroatom, and which ring is optionally substituted by one or more substituents selected from =O and C₁₋₃ alkyl (e.g. methyl);
R⁶⁰, R⁶¹ and R⁶² independently represent hydrogen or C₁₋₃ (e.g. C₁₋₂) alkyl optionally substituted by one or more fluoro atoms.

Preferred optional substituents on R³ (and, possibly when they represent a substituent other than hydrogen on R¹, R^{1a}, R^{1b} and R² groups) include:
=O (e.g. in the case of alkyl, cycloalkyl or heterocycloalkyl groups);
-CN;
halo (e.g. fluoro, chloro or bromo);
C₁₋₆ alkyl, such as C₃₋₆ cycloalkyl or acyclic C₁₋₄ alkyl, which alkyl group may be cyclic, part-cyclic, unsaturated or, preferably, linear or branched (e.g. C₁₋₄ alkyl (such as ethyl, *n*-propyl, isopropyl, *t*-butyl or, preferably, *n*-butyl or methyl), all of which are optionally substituted with one or more halo (e.g. fluoro) groups (so forming, for example, fluoromethyl, difluoromethyl or, preferably, trifluoromethyl) or substituted with an aryl, heteroaryl or heterocycloalkyl group (which themselves may be substituted with one or more -OR^{z1}, -C(O)R^{z2}, -C(O)OR^{z3}, -N(R^{z4})R^{z5}, -S(O)₂R^{z6}, -S(O)₂N(R^{z7})R^{z8}; -N(R^{z9})-C(O)-R^{z10}, -C(O)-N(R^{z11})R^{z12} and/or -N(R^{z9})-C(O)-N(R^{z10}) substituents);
a 5- or 6-membered heterocycloalkyl group (optionally substituted with one or more -OR^{z1}, -C(O)R^{z2}, -C(O)OR^{z3}, -N(R^{z4})R^{z5}, -S(O)₂R^{z6}, -S(O)₂N(R^{z7})R^{z8}; -N(R^{z9})-C(O)-R^{z10}, -C(O)-N(R^{z11})R^{z12} and/or -N(R^{z9})-C(O)-N(R^{z10}) substituents) (such heterocycloalkyl groups are preferably present on alkyl groups);
aryl (e.g. phenyl), if appropriate (e.g. when the substitutent is on an alkyl group, thereby forming e.g. a benzyl group);
-OR^{z1};
-C(O)R^{z2};
-C(O)OR^{z3};
-N(R^{z4})R^{z5};
-S(O)₂R^{z6};
-S(O)₂N(R^{z7})R^{z8};
-N(R^{z9})-C(O)-R^{z10};
-C(O)-N(R^{z11})R^{z12};
-N(R^{z9})-C(O)-N(R^{z10});
-N(R^{z9})-S(O)₂-R^{z10};
-N(R^{z9})-S(O)₂-N(R^{z10});
wherein each R^{z1} to R^{z12} independently represents, on each occasion when used herein, H or C₁₋₄ alkyl (e.g. ethyl, n-propyl, t-butyl or, preferably, n-butyl, methyl, isopropyl or cyclopropylmethyl (i.e. a part cyclic alkyl group)) optionally substituted by one or more halo (e.g. fluoro) groups (so forming e.g. a trifluoromethyl group) or, may also be substituted by one aryl (e.g. phenyl) group (so forming e.g. a benzyl group). Further, any two R^{z} groups (e.g. R^{z4} and R^{z5}), when attached to the same nitrogen heteroatom may also be linked together to form a ring such as one hereinbefore defined in respect of corresponding linkage of R¹⁰ and R¹¹ or R^{10a} and R^{11a} groups.

Preferred compounds of the invention include those in which:
R² represents hydrogen or a substituent selected from -N(R^{10b})R^{11b} and, preferably, halo (e.g. chloro, bromo or iodo) and -CN; Q^{1a}, Q^{1b}, Q^{1c}, Q^{1d}, Q^{1e}, Q^{1f} and Q^{1g} independently represent halo, -CN, -NO₂, -N(R^{10a})R^{11a}, -OR^{10a}, -C(=Y)N(R^{10a})R^{10b}, -C(=Y)-R^{10a}, -C(=Y)-OR^{10a}, -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a} or heterocycloalkyl (optionally substituted by one or more (e.g. one) substituent selected from E⁵);
R^{10a}, R^{11a} and R^{12a} independently represent hydrogen or C₁₋₆ (e.g. C₁₋₃) alkyl optionally substituted by one or more fluoro atoms;
R^{11c} represents C₁₋₆ (e.g. C₁₋₃) alkyl optionally substituted by one or more fluoro atoms;
each E¹ E², E³, E⁴, E⁵, E⁶, E⁷, E⁸ and E⁹ independently represents C₁₋₁₂ alkyl optionally substituted by one or more substituents selected from =O and Q⁵, or, preferably (each E¹ to E⁹ independently represent) Q⁴;
each R²⁰, R²¹, R²² and R²³ (e.g. each R²⁰ and R²¹) independently represents heteroaryl, preferably, aryl (e.g. phenyl) (which latter two groups are optionally substituted by one or more substituents selected from J⁵), or, more preferably, hydrogen or C₁₋₆ (e.g. C₁₋₄) alkyl optionally substituted by one or more substituents selected from =O and J⁴; or
any relevant pair of R²⁰, R²¹ and R²² (e.g. R²⁰ and R²¹) may (e.g. when both are attached to the same nitrogen atom) may be linked together to form a 3- to 8-(e.g. 4- to 8-) membered ring, optionally containing a further heteroatom, and optionally substituted by one or more substituents selected from =O and J⁶;
R^{21a} represents C₁₋₆ (e.g. C₁₋₄) alkyl optionally substituted by one or more substituents selected from =O and J⁴;
each J¹, J², J³, J⁴, J⁵ and J⁶ independently represent C₁₋₆ alkyl (e.g. C₁₋₃ acyclic alkyl or C₃₋₅ cycloalkyl) optionally substituted by one or more substituents selected from Q⁸, or, J¹ to J⁶ more preferably represent a substituent selected from Q⁷;
each Q⁷ and Q⁸ independently represent halo, -N(R⁵⁰)R⁵¹, -OR⁵⁰, -C(=Y^{a})-OR⁵⁰, -C(=Y^{a})-R⁵⁰, -S(O)₂R⁵⁰ or C₁₋₃ alkyl optionally substituted by one or more fluoro atoms;
each R⁵⁰, R⁵¹, R⁵² and R⁵³ independently represents hydrogen or C₁₋₆ (e.g. C₁₋₄) alkyl optionally substituted by one or more fluoro atoms;
each R⁶⁰, R⁶¹ and R⁶² independently represents hydrogen or C₁₋₂ alkyl (e.g. methyl).

More preferred compounds of the invention include those in which:
Q^{1a}, Q^{1b} Q^{1c}, Q^{1d}, Q^{1e}, Q^{1f} and Q^{1g} independently represent halo, -CN, -NO₂, -N(R^{10a})R^{11a}, -OR^{10a}, -C(=Y)N(R^{10a})R^{10b}, -C(=Y)-R^{10a}, -C(=Y)-OR^{10a}, -N(R^{12a})C(=Y)R^{11a} or heterocycloalkyl (optionally substituted by one or more (e.g. one) substituent selected from E⁵);
R² represents hydrogen, chloro, bromo, iodo or -CN;
each R^{10a}, R^{11a} R^{10b}, R^{11b} and R^{12a} independently represents hydrogen or C₁₋₄ (e.g. C₁₋₃) alkyl, which alkyl group may by substituted by one or more substituents selected from =O and E⁷ (but which alkyl group is more preferably unsubstituted); or
any relevant pair of R^{10a} and R^{11a} and/or R^{10b} and R^{11b}, may be linked together to form a 5- or, preferably, a 6-membered ring, optionally containing a further heteroatom (preferably selected from nitrogen and oxygen), which ring is preferably saturated (so forming, for example, a piperazinyl or morpholinyl group), and optionally substituted by one or more substituents selected from =O and E⁹ (which E⁹ substituent may be situated on a nitrogen heteroatom; and/or E⁹ is preferably halo (e.g. fluoro) or C₁₋₃ alkyl optionally substituted by one or more fluoro atoms);
each E¹, E², E³, E⁴, E⁵, E⁶, E⁷, E⁸ and E⁹ independently represent C₁₋₄ alkyl optionally substituted by one or more Q⁵ substituents, or, each of these preferably represent a substituent selected from Q⁴;
Q⁴ and Q⁵ independently represent halo (e.g. fluoro), -OR²⁰, -N(R²⁰)R²¹, -C(=Y)OR²⁰, -C(=Y)N(R²⁰)R²¹, -NR²²S(O)₂R²⁰, heterocycloalkyl, aryl, heteroaryl (which latter three groups are optionally substituted with one or more substitutents selected from J² or J³, as appropriate) and/or C₁₋₆ alkyl (e.g. C₁₋₃ alkyl) optionally substituted by one or more fluoro atoms;
each Y represents, on each occasion when used herein, =S, or preferably =O;
each R²⁰, R²¹, R²² and R²³ (e.g. each R²⁰ and R²¹) independently represents hydrogen or C₁₋₄ (e.g. C₁₋₃) alkyl (e.g. tert-butyl, ethyl, methyl or a part cyclic group such as cyclopropylmethyl) optionally substituted (but preferably unsubstituted) by one or more (e.g. one) J⁴ substituent(s); or
any relevant pair of R²⁰, R²¹ and R²² (e.g. R²⁰ and R²¹) may (e.g. when both are attached to the same nitrogen atom) may be linked together to form a 5- or, preferably, a 6-membered ring, optionally containing a further heteroatom (preferably selected from nitrogen and oxygen), which ring is preferably saturated (so forming, for example, a piperazinyl or morpholinyl group), and optionally substituted by one or more substituents selected from =O and J⁶ (which J⁶ substituent may be situated on a nitrogen heteroatom);
R²² represents C₁₋₃ alkyl or, preferably, hydrogen;
each J¹ J², J³, J⁴, J⁵ and J⁶ independently represent a substituent selected from Q⁷, or J¹ to J⁶ (e.g. J⁴) represents C₁₋₆ alkyl (e.g. C₃₋₅ cycloalkyl);
each Q⁷ and Q⁸ independently represent -C(=Y^{a})-OR⁵⁰, -C(=Y^{a})-R⁵⁰, -S(O)₂R⁵⁰ or C₁₋₃ alkyl optionally substituted by one or more fluoro atoms;
each Y^{a} independently represents =S or, preferably, =O;
each R⁵⁰ independently represents C₁₋₄ alkyl (e.g. *tert*-butyl or methyl).

Preferred R³ groups of the compounds of the compounds of the invention include optionally substituted phenyl, indazolyl (e.g. 4-indazolyl), pyrimidinyl (e.g. 5-pyrimidinyl), azaindolyl (e.g. azaindol-5-yl), indolyl (e.g. 5-indolyl or 4-indolyl) and pyridyl (e.g. 3-pyridyl). Particularly preferred R³ groups of the compounds of the compounds of the invention include optionally substituted pyridyl (e.g. 3-pyridyl) and, preferably, phenyl, indazolyl (e.g. 4-indazolyl) and pyrimidinyl (e.g. 5-pyrimidinyl).

Preferred compounds of the invention include those in which:
R² represents hydrogen or halo (e.g. chloro);
R³ represents aryl (e.g. phenyl) or heteroaryl (e.g. a 5- or 6-membered monocyclic heteroaryl group or a 9- or 10-membered bicyclic heteroaryl group; which groups may contain one to four, e.g. 3 or, preferably, 1 or 2, heteroatoms preferably selected from nitrogen, oxygen and sulfur) both of which are optionally substituted by one or more (e.g. two, or, preferably, one) substituent(s) selected from E⁴ (e.g. -CF₃, preferably, -OH, -OC₁₋₆ alkyl (e.g. -OCH₃) and/or -N(R²⁰)R²¹ (e.g. -NH₂));
E¹ to E⁹ independently represent C₁₋₆ (e.g. C₁₋₃, such as methyl) alkyl optionally substituted by one or more Q⁵ substituents, or, preferably, Q⁴;
Q⁴ represents C₁₋₃ alkyl or, preferably, Q⁴ represents -OR²⁰, -N(R²⁰)R²¹, -S(O)₂R²⁰, heterocycloalkyl (e.g. a 4- to 6-membered ring, containing preferably one or two heteroatoms selected from nitrogen and oxygen), aryl (e.g. phenyl;
optionally substituted with two or, preferably, one substituent selected from J³) or heteroaryl (e.g. a 5- or 6-membered monocyclic heteroaryl group preferably containing one or two heteroatoms preferably selected from nitrogen, oxygen and sulfur; which group may be substituted, but is preferably unsubstituted);
when E⁴ represents Q⁴, then Q⁴ preferably represents -OR²⁰ or -N(R²⁰)R²¹;
when E⁵ represents Q⁴, then Q⁴ preferably represents C₁₋₃ alkyl or, more preferably, -S(O)₂R²⁰;
when E⁷ represents Q⁴, then Q⁴ preferably represents -OR²⁰, -S(O)₂R²⁰ or aryl (e.g. phenyl; which is preferably unsubstituted);
Q⁵ represents halo (e.g. fluoro);
Y represents =O;
R²⁰ and R²¹ independently represent hydrogen, C₁₋₃ alkyl (e.g. isopropyl or, preferably, methyl or ethyl), which latter group is optionally substituted by one or more (e.g. one) substituent(s) selected from J⁴;
when there is a -N(R²⁰)R²¹ moiety present, then one of R²⁰ and R²¹ represents hydrogen, and the other represents hydrogen or C₁₋₃ alkyl (e.g. methyl or ethyl), which latter group is optionally substituted by one or more (e.g. one) substituent(s) selected from J⁴;
J³ represents Q⁷;
J⁴ represents Q⁷ or C₁₋₆ alkyl (such as C₃₋₆ alkyl, e.g. C₃₋₆ cycloalkyl);
J⁴ more preferably represents C₁₋₆ alkyl, such as C₃₋₆ alkyl (especially C₃₋₆ cycloalkyl, such as cyclopropyl);
Q⁷ represents -S(O)₂R⁵⁰ or aryl (e.g. phenyl) optionally substituted by one or more (e.g. one) substitutents (e.g. at the 4-position) by -OR⁶⁰;
Q⁷ more preferably represents -S(O)₂R⁵⁰;
when J³ represents Q⁷, then Q⁷ preferably represents -S(O)₂R⁵⁰;
when J⁴ represents Q⁷, then Q⁷ preferably represents aryl (e.g. phenyl) optionally substituted by one or more (e.g. one) substitutents (e.g. at the 4-position) by -OR⁶⁰;
R⁵⁰ represents C₁₋₃ alkyl (e.g. methyl);
R⁶⁰ represents C₁₋₃ alkyl (e.g. methyl);
B¹, B^{1a}, B², B^{2a}, B³, B^{3a}, B⁴ and B^{4a} independently represent C₁₋₆ (e.g. C₁₋₃) alkyl optionally substituted by halo, or, these groups preferably represent hydrogen.

Particularly preferred compounds of the invention include those in which:
R^{1b} represents -T¹-Z¹ and R^{1a} (if present) represents R^{1c};
R^{1a} and R^{1b} are preferably both present (i.e. A₄ preferably represents C(R^{1a}) and A₄ₐ preferably represents C(R^{1b}));
R^{1c} represents C₁₋₃ (e.g. C₁₋₂) alkyl or, more preferably, hydrogen or halo such as fluoro (especially, R^{1c} represents hydrogen);
T¹ represents a linker/spacer group as defined herein, preferably, -CH₂-N(R^{t1})-[2-pyrimidin-5-yl]- or -CH₂-[1-piperazin-4-yl]-[2-pyrimidin-5-yl]-;
Z¹ represents -C(O)N(H)OH (i.e. a hydroxamic acid or derivative thereof);
R² represents hydrogen or halo (e.g. chloro);
R³ represents hydroxyphenyl (e.g. 3-hydroxyphenyl), methoxyphenyl (e.g. 3-methoxyphenyl), indazolyl (e.g. 4-indazolyl), pyrimidinyl (e.g. 5-pyrimidinyl, such as 2-amino-5-pyrimidinyl (i.e. 2-[-N(R²⁰)(R²¹)]-pyrimidin-5-yl such as 2-NH₂-pyrimidin-5-yl or 2-[N(H)(CH₂-cyclopropyl)-pyrimidin-5-yl] or 2-methoxy-5-pyrimidinyl), azaindolyl (e.g. 7-azaindol-5-yl), indolyl (e.g. 5-indolyl or 4-indolyl, such as 5-fluoro-4-indolyl), pyridyl (e.g. 3-pyridyl, such as 6-NH₂-pyrid-3-yl, 5-OCH₃-pyrid-3-yl or 5-CF₃,6-NH₂-pyrid-3-yl) (other R³ groups that may be mentioned include 2-[-N(H)-CH₂-(4-methoxyphenyl)]-pyrimidin-5-yl and 6-[-N(H)-CH₂-(4-methoxyphenyl)]-pyridin-3-yl);
Y represents =O;
Y^{a} represents =O;
B¹, B^{1a}, B² B^{2a}, B³, B^{3a}, B⁴ and B^{4a} independently represent hydrogen.

More preferred compounds of the invention:
R² represents H or halo (e.g. chloro);
R³ represents aryl (e.g. phenyl) optionally substituted by one or more (e.g. one) substituents selected from E⁴, or, R³ may represent heteroaryl (e.g. a monocyclic 5- or preferably 6-membered heteroaryl group or a bicyclic 10- or, preferably, 9-membered heteroaryl group, in which there is one or two (e.g. two) heteroatoms present selected from sulfur, oxygen or, preferably, nitrogen, so forming for example a pyridyl, pyrimidinyl or indazolyl group), which is optionally substituted by one or more (e.g. one) substituent selected from E⁴;
R³ more preferably represents an optionally substituted monocyclic 6-membered heteroaryl group, especially pyrimidinyl optionally substituted by one or more (e.g. one) substituent selected from E⁴;
R^{10a} represents H, C₁₋₃ alkyl or heterocycloalkyl (e.g. a 7- or, preferably, 5- or more preferably 6-membered heterocycloalkyl group, e.g. containing one or two (e.g. one) heteroatoms in which one is preferably nitrogen, e.g. a diazepanyl group (e.g. [1,4]diazepany-1-yl), morpholinyl (e.g. 4-morpholinyl), piperazinyl (e.g. 1-piperazinyl), or, preferably, a piperidinyl group, such as 4-piperidinyl), which latter two groups are optionally substituted with one or more (e.g. one) substituent(s) selected from E⁷ (which may be located on a nitrogen heteroatom of a heterocycloalkyl group);
more preferably, R^{10a} represents H or C₁₋₃ alkyl (e.g. methyl) optionally substituted by one or more (e.g. one) E⁷ group;
R^{10b} represents H or C₁₋₃ alkyl (e.g. methyl);
R^{11a} represents H or, preferably, C₁₋₃ alkyl (e.g. ethyl) optionally substituted by one or more (e.g. one) E⁷ group (e.g. -OR²⁰);
R^{10a} and R^{11a} may be linked together to form a 5- or, preferably 6-membered ring (optionally containing one further heteroatom; in addition to the nitrogen heteroatom that is necessarily present), but preferably, R^{10a} and R^{11a} are not linked together;
E⁴ represents Q⁴;
E⁵ represents C₁₋₃ alkyl or, preferably, Q⁴;
E⁷ represents Q⁴;
Y represents =O;
Q⁴ represents C₁₋₃ alkyl or, preferably, -N(R²⁰)R²¹ (e.g. -NH₂), -OR²⁰, -S(O)₂R²⁰, or aryl (e.g. phenyl, which is preferably unsubstituted);
when E⁴ represents Q⁴, Q⁴ is preferably -N(R²⁰) R²¹;
when E⁵ represents Q⁴, Q⁴ is C₁₋₃ alkyl or, preferably, -S(O)₂R²⁰;
when E⁷ represents Q⁴, Q⁴ is preferably -OR²⁰ or -S(O)₂R²⁰;
R²⁰ represents H or C₁₋₃ alkyl (e.g. isopropyl or, preferably, methyl) optionally substituted by one or more (e.g. one) substituents selected from J⁴ (for instance, when Q⁴ represents -N(R²⁰)R²¹, then R²⁰ may represent substituted alkyl);
R²⁰ more preferably represents H or C₁₋₃ alkyl (e.g. isopropyl or, preferably, methyl);
R²¹ represents H;
J⁴ represents Q⁷;
Q⁷ represents aryl (e.g. phenyl) optionally substituted by one or more (e.g. one) substitutents (e.g. at the 4-position) by -OR⁶⁰;
R⁶⁰ represents C₁₋₃ alkyl (e.g. methyl).

Particularly preferred compounds of the invention include those in which:
the requisite bicycle of formula I represents: 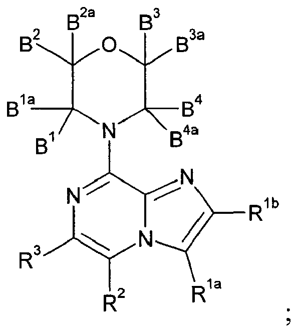
R^{1b} represents -T¹-Z¹;
T¹ represents: 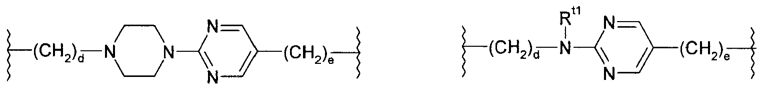
d represents 1; e represents 0; R^{t1} represents hydrogen or C₁₋₂ alkyl (e.g. methyl);
Z¹ represents -C(O)N(H)OH;
R^{1a} represents R^{1c};
R^{1c} represents hydrogen;
R² represents hydrogen;
R³ represents 2-amino-pyrimidin-5-yl.

Particularly preferred compounds of the invention include those of the examples described hereinafter.

Compounds of the invention may be made in accordance with techniques that are well known to those skilled in the art, for example as described hereinafter.

According to a further aspect of the invention there is provided a process for the preparation of a compound of formula I which process comprises:
(i) reaction of a compound of formula II, 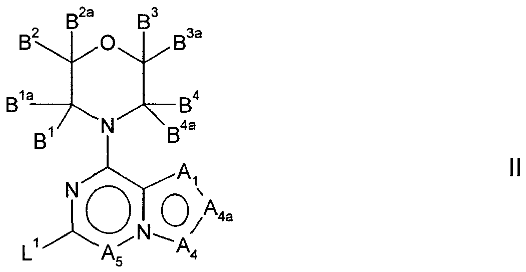 wherein L¹ represents a suitable leaving group, such as iodo, bromo, chloro, a sulfonate group (e.g. -OS(O)₂CF₃, -OS(O)₂CH₃ or -OS(O)₂PhMe), or a sulfide group (e.g. -S-C₁₋₆ alkyl, such as -SCH₃) and A₁, A₄, A₄ₐ, A₅, B¹, B^{1a}, B², B^{2a}, B³, B^{3a}, B^{a} and B^{4a} are as hereinbefore defined, with a compound of formula III,

   R³-L² III

   wherein L² represents a suitable group such as -B(OH)₂, -B(OR^{wx})₂ or -Sn(R^{wx})₃, in which each R^{wx} independently represents a C₁₋₆ alkyl group, or, in the case of -B(OR^{wx})₂, the respective R^{wx} groups may be linked together to form a 4- to 6-membered cyclic group (such as a 4,4,5,5-tetramethyl-1,3,2-dioxaborolan-2-yl group), thereby forming e.g. a pinacolato boronate ester group, (or L² may represent iodo, bromo or chloro, provided that L¹ and L² are mutually compatible) and R³ is as hereinbefore defined. The reaction may be performed, for example in the presence of a suitable catalyst system, e.g. a metal (or a salt or complex thereof) such as Pd, Cul, Pd/C, PdCl₂, Pd(OAc)₂, Pd(Ph₃P)₂Cl₂, Pd(Ph₃P)₄ (i.e. palladium tetrakistriphenylphosphine), Pd₂(dba)₃ and/or NiCl₂ (preferred catalysts include palladium) and a ligand such as PdCl₂(dppf).DCM, *t*-Bu₃P, (C₆H₁₁)₃P, Ph₃P, AsPh₃, P(*o*-Tol)₃, 1,2-bis(diphenylphosphino)ethane, 2,2'-bis(di-*tert*-butyl-phosphino)-1,1'-biphenyl, 2,2'-bis(diphenylphosphino)-1,1'-bi-naphthyl, 1,1'-bis(diphenyl-phosphino-ferrocene), 1,3-bis(diphenylphosphino)propane, xantphos, or a mixture thereof (preferred ligands include PdCl₂(dppf).DCM), together with a suitable base such as, Na₂CO₃, K₃PO₄, Cs₂CO₃, NaOH, KOH, K₂CO₃, CsF, Et₃N, (*i*-Pr)₂NEt, *t*-BuONa or *t*-BuOK (or mixtures thereof; preferred bases include Na₂CO₃ and K₂CO₃) in a suitable solvent such as dioxane, toluene, ethanol, dimethylformamide, dimethoxyethane, ethylene glycol dimethyl ether, water, dimethylsulfoxide, acetonitrile, dimethylacetamide, *N*-methylpyrrolidinone, tetrahydrofuran or mixtures thereof (preferred solvents include dimethylformamide and dimethoxyethane). When L¹ represents a sulfide (e.g. -SCH₃), then an additive such as CuMeSal (copper(I) 3-methylsalicylate) or CuTC (copper(I)thiophene-2-carboxylate) may also be employed. The reaction may be carried out for example at room temperature or above (e.g. at a high temperature such as at about the reflux temperature of the solvent system). Alternative reaction conditions include microwave irradiation conditions, for example at elevated temperature, e.g. of about 130°C;
(ii) reaction of a compound of formula IV, 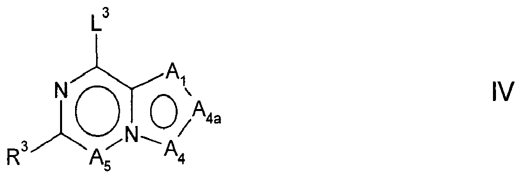 wherein L³ represents a suitable leaving group, such as one hereinbefore defined in respect of L¹ or a sulfone (e.g. -S(O)₂C₁₋₆ alkyl moiety, such as -S(O)₂CH₃) or sulfide (e.g. -S-C₁₋₆ alkyl moiety, such as -SCH₃) and A₁, A₄, A₄ₐ, A₅ and R³ as hereinbefore defined, with a compound of formula V, 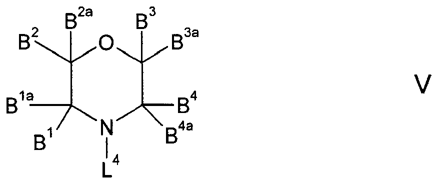 wherein L⁴ may represent hydrogen (so forming an amine group), and B¹, B^{1a}, B², B^{2a} B³, B^{3a}, B⁴ and B^{4a} are as hereinbefore defined, and the reaction may optionally be performed in the presence of an appropriate metal catalyst (or a salt or complex thereof) such as Cu, Cu(OAc)₂, Cul (or Cul/diamine complex), copper tris(triphenylphosphine)bromide, Pd(OAc)₂, tris(dibenzylideneacetone)-dipalladium(0) (Pd₂(dba)₃) or NiCl₂ and an optional additive such as Ph₃P, 2,2'-bis(diphenylphosphino)-1,1'-binaphthyl, xantphos, Nal or an appropriate crown ether such as 18-crown-6-benzene, in the presence of an appropriate base such as NaH, Et₃N, pyridine, *N*,*N*'-dimethylethylenediamine, Na₂CO₃, K₂CO₃, K₃PO₄, Cs₂CO₃, *t*-BuONa or *t*-BuOK (or a mixture thereof, optionally in the presence of 4A molecular sieves), in a suitable solvent (e.g. dichloromethane, dioxane, toluene, ethanol, isopropanol, dimethylformamide, ethylene glycol, ethylene glycol dimethyl ether, water, dimethylsulfoxide, acetonitrile, dimethylacetamide, *N*-methylpyrrolidinone, tetrahydrofuran or a mixture thereof). This reaction may be performed at elevated temperature or under microwave irradiation reaction conditions, for example as described in process step (i). The compound of formula IV (e.g. in which L³ is chloro) may be prepared *in situ*, for example from a compound corresponding to a compound of formula IV, but in which L³ represents -OC₁₋₃ alkyl (e.g. methoxy) by reaction in the presence of e.g. a chlorinating agent (such as POCl₃);
(iii) for compounds of formula I in which (A⁵ represents C(R²) and) R² represents halo (e.g. bromo, iodo or chloro), reaction of a corresponding compound of formula I, in which R² represents hydrogen, with a reagent that is a source of halide ions (a halogenating reagent). For instance, an electrophile that provides a source of iodide ions includes iodine, diiodoethane, diiodotetrachloroethane or, preferably, *N*-iodosuccinimide, a source of bromide ions includes *N-*bromosuccinimide and bromine, and a source of chloride ions includes *N-*chlorosuccinimide, chlorine and iodine monochloride, for instance in the presence of a suitable solvent, such as CHCl₃ or an alcohol (e.g. methanol), optionally in the presence of a suitable base, such as a weak inorganic base, e.g. sodium bicarbonate. Typically, the reaction maybe performed by heating at a convenient temperature, either by conventional heating under reflux or under microwave irradiation;
(iv) for compounds of formula I in which R² (if present; i.e. if A⁵ represents C(R²)) represents a substituent other that hydrogen, or halo (e.g. bromo, iodo or chloro), reaction of a corresponding compound of formula I, in which R² represents halo (e.g. bromo, chloro or iodo), with a compound of formula VI,
   R^{2a}-L⁷ VI
   wherein R^{2a} represents R² as hereinbefore described provided that it does not represent hydrogen or halo, and L⁷ represents a suitable leaving group such as one hereinbefore described in respect of L¹ or L² (see e.g. process step (i); reaction conditions such as those mentioned above may also be employed). Alternatively, the skilled person will appreciate that different reagents and reaction steps may be employed, depending on the particular R^{2a} substituent required;
(v) compounds of formula I in which A^{4a} represents C(R^{1b}) and R^{1b} represents C₁₋₁₂ alkyl or heterocycloalkyl linked to the carbon atom of the requisite bicycle (and forms a part of the "-T¹-Z¹" linker group) or R^{1a} is present, which represents -C(O)OR^{10a}, halo, C₁₋₁₂ alkyl or heterocycloalkyl (which latter two groups are optionally substituted as hereinbefore defined; and hence a -C(O)H group is possible) may be prepared from corresponding compounds of formula I in which R^{1a} or R^{1b} (as appropriate) represents hydrogen, which may be reacted in the presence of a suitable base, such as an organometallic base (e.g. an organolithium base, such as *t*-, *s*- or *n*-butyllithium or, preferably a lithium amide base such as diisopropylamide; which deprotonates and/or lithiates at the relevant position), followed by reaction in the presence of an electrophile that is a source of halide ions (e.g. as described in respect of process step (iii)), or a compound of formula VII,

   L⁸-R^{1b1} VII

   wherein L⁸ represents a suitable leaving group, such as one hereinbefore defined in respect of L¹ (or another suitable leaving group, such as -N(CH₃)₂), and R^{1b1} represents -C(O)OR^{10a} (and R^{10a} is preferably not hydrogen), C₁₋₁₂ alkyl or heterocycloalkyl (which latter two groups are optionally substituted by one or more substituents as hereinbefore defined in respect of substituents on such alkyl or heterocycloalkyl groups (which may be a part of the relevant linker/spacer group); i.e. R^{1b1} may represent -C(O)H, and hence the compound of formula VII may be dimethylformamide, which is employed to introduce the -C(O)H group), under standard reaction conditions, for example the deprotonation/lithiation may be performed in an inert atmosphere (e.g. under N₂) in the presence of an anhydrous polar aprotic solvent (such as THF, dimethoxyethane, ethyl ether and the like), which may be performed at below room temperature (e.g. at below 0°C, at temperatures down to -78°C, depending on the strength of the base to be employed), and the subsequent 'quench', i.e. reaction with the electrophile (e.g. halide source or compound of formula VII) may also be performed at low temperatures (e.g. at the temperature of the deprotonation/lithiation), which temperature may be raised up to 0°C (or to rt) to ensure the complete reaction, before the mixture is worked up;
(vi) for compounds of formula I which contain a -C(OH)(H)-C₁₋₁₁ alkyl group (which alkyl group may be substituted by one or more substituents selected from e.g. E³ and =O; Q^{1a}; or Q², =O, =S and =N(R^{10a}) as appropriate, but is preferably unsubstituted), for example when there is a R¹, R^{1a}, R^{1b} and/or R² group present which represent such a -C(OH)(H)-C₁₋₁₁ alkyl group, reaction of a corresponding compound of formula I in which there is a -C(O)H group present (i.e. R¹, R^{1a}, R^{1b} and/or R² represents -C(O)H), with a compound of formula VIII,

   R^{xx}MgX¹ VIII

   wherein R^{xx} represents C₁₋₁₁ alkyl optionally substituted by one or more substituents selected from E³ and =O (but is preferably unsubstituted) and X¹ represents halo (e.g. iodo, bromo or, preferably, chloro), under standard Grignard reaction conditions, e.g. in the presence of an inert atmosphere and, optionally, an anhydrous solvent;
(vii) compounds of formula I in which A₁ and A₄ both represent N, A₅ represents C(R²) and A₄ₐ represents C(R^{1b}) may be prepared by reaction of a compound of formula IX, 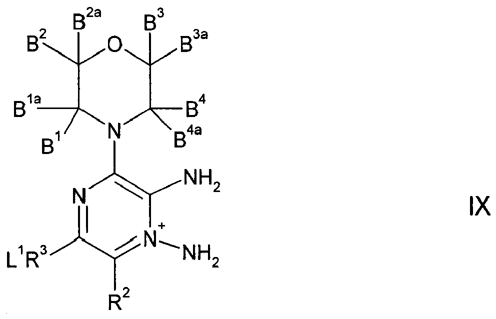 wherein L¹R³ represents either L¹ as hereinbefore defined or R³ as hereinbefore defined, and R², B¹, B^{1a}, B², B^{2a}, B³, B^{3a}, B⁴ and B^{4a} are as hereinbefore defined, with a compound of formula X,

   H-C(O)-R^{1b} X

   wherein R^{1b} is as hereinbefore defined (and preferably represents hydrogen or C₁₋₁₂ alkyl optionally substituted as hereinbefore defined; hence the compound of formula X may be paraformaldehyde or another aldehyde), optionally in the presence of a suitable base (for instance a sterically hindered base, such as an amidine base, e.g. DBU) and a suitable solvent (e.g. dichloromethane) at an appropriate temperature (e.g. room temperature) for an appropriate period of time. When L¹R³ in the compound of formula IX represents L¹, then this process step may be proceeded by process step (i) as defined above. Corresponding reactions may also take place in which A₅ represents N (instead of C(R²));
(viii) compounds of formula I in which A₁ represents N, A₄ represents C(R^{1a}), A₄ₐ represents N and A₅ represents C(R²) may be prepared by reaction of a compound of formula XI, 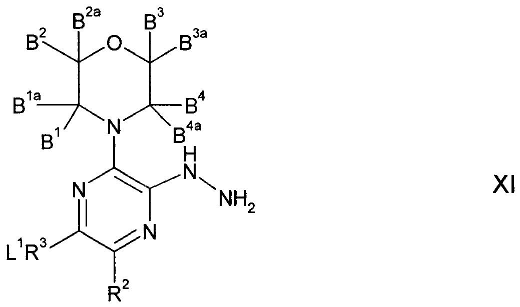 wherein L¹R³, R², B¹, B^{1a}, B², B^{2a}, B³, B^{3a}, B⁴ and B^{4a} are as hereinbefore defined, with a compound of formula XII,

   R^{1a}-C(OC₁₋₆ alkyl)₃ XII

   or, a compound of formula XIII,

   R^{1a}-C(O)OH XIII

   or, derivatives of either, wherein R^{1a} is as hereinbefore defined (and is preferably hydrogen or optionally substituted C₁₋₁₂ alkyl; so forming e.g. triethyl orthoformate, triethyl orthoacetate, formic acid, and the like), under standard reaction conditions. When L¹R³ in the compound of formula XI represents L¹, then this process step may be proceeded by process step (i) as defined above. Corresponding reactions may also take place in which A₅ represents N (instead of C(R²));
(ix) compounds of formula I in which Z¹ represents -C(=Y)N(R^{10a})-OH (and Y is preferably =O) may be prepared by reaction of a corresponding compound of formula I in which there is a -C(=Y)OR^{xx} (in which R^{xx} represents H or C₁₋₆ alkyl such as ethyl) with a compound of formula XIIIA,

   HN(R^{10a})-OH XIIIA

   wherein R^{10a} is as hereinbefore defined, which compound generally exists as a solution and reaction with such a solution may take place at room temperature or elevated temperature (e.g. at reflux). The solution of compound of formula XIIIA may be freshly prepared from the corresponding acid (e.g. HCl salt) in a solvent (e.g. a alcoholic solvent, such as methanol) to which a base (e.g. hydroxide, such as KOH) optionally in solvent (e.g. a alcoholic solvent, such as methanol) may be added (to introduce an R^{11c} that is not hydrogen, an appropriate substitution reaction may be performed, e.g. in the presence of a base such as NaH and e.g. an alkylating reagent);
(x) for compounds of formula I in which T^{1a} represents -CH₂- and X^{1a} represents -N(R^{t1})- or heterocycloalkylene (linked to T^{1a} *via* a nitrogen atom of that cyclic group), reaction of a compound corresponding to a compound of formula I but in which -T¹-Z¹ represents -CH₂-L^{xx} (in which L^{xx} represents a suitable leaving group, such as chloro) with a compound containing the X^{1a} moiety (i.e. HN(R^{t1})-X^{1b}-T^{1b}-Z¹ or heterocycloalkyl-X^{1b}-T^{1b}-Z¹) under appropriate couple reaction conditions such as those hereinbefore described in respect of process step (ii) above;
(xi) for compounds of formula I in which T^{1a} represents -CH₂- and X^{1a} represents -N(R^{t1})-, reaction of a compound corresponding to a compound of formula I but in which -T¹-Z¹ represents -CH₂-N(R^{t1})H with a compound L^{xx}-X^{1b}-T^{1b}-Z¹ (where the integers are hereinbefore defined, so forming e.g. heteroaryl substituted by chloro) under appropriate couple reaction conditions such as those hereinbefore described in respect of process step (ii) above.

Compounds of formula II may be prepared by reaction of a compound of formula XIV, 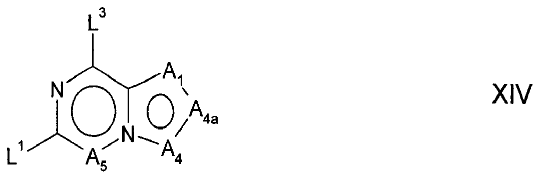 wherein L¹, L³, A₁, A₄, A₄ₐ, A₅ and R³ are as hereinbefore defined, with a compound of formula V, as hereinbefore defined, for example under reaction conditions such as those hereinbefore described in respect of preparation of compounds of formula I (process step (ii) above).

Compounds of formula IV (for example, in which A₁ represents C(R¹), A₄ represents C(R^{1a}), A₄ₐ represents C(R^{1b}) and A₅ represents C(R²)) in which L³ represents e.g. chloro, bromo or iodo, may be prepared by reaction of a compound of formula XV, 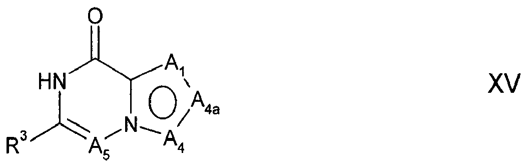 wherein A₁, A₄, A₄ₐ, A₅ and R³ as hereinbefore defined (or its tautomer), in the presence of a suitable reagent that provides the source of the chloro, bromo or iodo (e.g. POCl₃ may be employed, or, a reagent such as p-toluenesulfonyl chloride or the like) under reaction conditions known to the skilled person, for example at reflux (e.g. in the case of reaction with POCl₃) or, in the case of reaction with *p*-toluenesulfonyl chloride, in the presence of a base, such as an organic amine e.g. triethylamine, *N,N*-dimethylaniline (or the like), and optionally a catalyst such as DMAP (and optionally in the presence of a suitable solvent, such as acetonitrile). In the case of the latter, the compound of formula I may be prepared directly form the intermediate compound IV that may be formed by reaction in the presence of a compound of formula V (which latter reaction need not follow the reaction conditions set out above in respect of process step (ii); for instance, the reaction mixture may simply be heated in the same pot, e.g. at elevated temperature such as at about 65°C.

Compounds of formula IV in which L³ represents a sulfonate, such as -S(O)₂C₁₋₆ alkyl (e.g. -S(O)₂CH₃) may be prepared by oxidation of a compound of formula XVI, 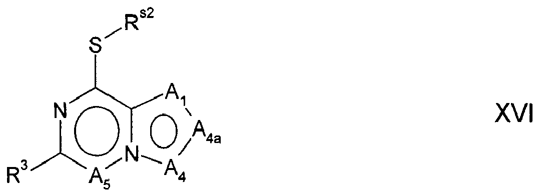 wherein R^{s2} represents C₁₋₆ alkyl (e.g. methyl), and A₁, A₄, A₄ₐ, A₅ and R³ are as hereinbefore defined, in the presence of an oxidising agent such as *m-*chloroperbenzoic acid and, if necessary, a suitable solvent (e.g. dichloromethane).

Compounds of formula IX (e.g. in which L¹R³ represents L¹) may be prepared by reaction of a compound of formula XVII, 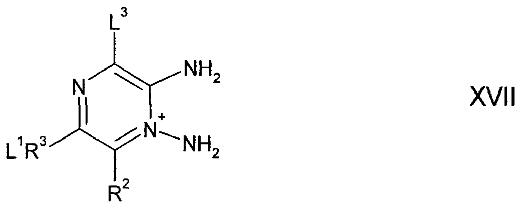 wherein L¹R³, R² and L³ are as hereinbefore defined, with a compound of formula V as hereinbefore defined, for example under reaction conditions such as those hereinbefore defined in respect of preparation of compounds of formula I (process step (ii) above).

Compounds of formula IX and XVII may be prepared by reaction of a compound of formula XVIII, 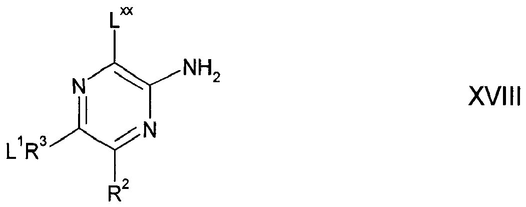 wherein L^{xx} represents L³ (in the case of preparation of compounds of formula XVII) or represents the following moiety: 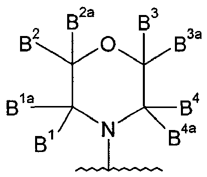 (in the case of preparation of compounds of formula IX), and R², L¹R³, B¹, B^{1a}, B², B^{2a}, B³, B^{3a}, B⁴ and B^{4a} are as hereinbefore defined, with *o-*(mesitylsulfonyl)hydroxylamine (or the like; i.e. another suitable source of -NH₂), under standard reaction conditions known to those skilled in the art, e.g. in the presence of a suitable solvent (e.g. dichloromethane).

Compounds of formula XI may be prepared by reaction of a compound of formula XIX, 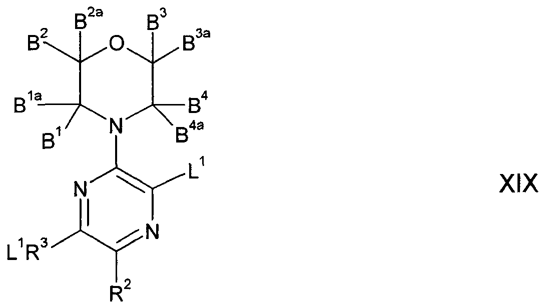 wherein L¹, R², L¹R³, B¹, B^{1a}, B², B^{2a}, B³, B^{3a}, B⁴ and B^{4a} are as hereinbefore defined, with hydrazine (or a derivative thereof, e.g. hydrazine hydrate), under standard conditions.

Compounds of formula XIV in which L¹ represents a sulfide such as -SCH₃, L³ represents a sulfide such as -SCH₃, and A₁ and A₅ both represent N, A₄ represents C(R^{1a}) and A₄ₐ represents C(R^{1b}) may be prepared by reaction of a compound of formula XX, 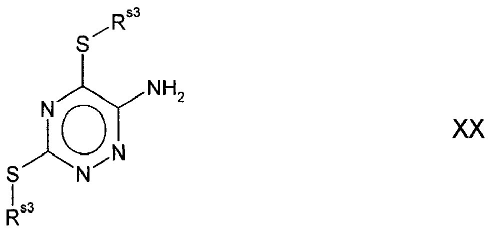 wherein R^{s3} represents C₁₋₆ alkyl (preferably methyl), with a compound of formula XXI,

L¹⁵-C(H)(R^{1a})-C(O)-R^{1b} XXI

wherein L¹⁵ represents a suitable leaving group, such as one hereinbefore defiend by L¹ (e.g. halo, such as bromo) and R^{1a} and R^{1b} are as hereinbefore defined, and R^{1a} preferably represents hydrogen (or a protected derivative thereof; e.g. the compound of formula XXI may be bromoacetaldehyde diethyl acetal, or, when R^{1b} represents -C(O)Oethyl, the compound of formula XXI may be ethyl bromopyruvate), for example in the presence of an acid catalyst (e.g. p-toluenesulfonic acid or the like), which reaction may be performed at room temperature or preferably at elevated temperature e.g. at about 65°C. Corresponding reactions may also take place in which A₅ represents C(R²).

Compounds of formula XIV in which L³ represents halo (e.g. chloro) and L¹ represents a sulfide (e.g. -SCH₃) (and, preferably, A₅ represents N, A₄ represents C(R^{1a}), A₄ₐ represents C(R^{1b}) and A₁ represents N), may also be prepared by reaction of a compound of formula XXII, 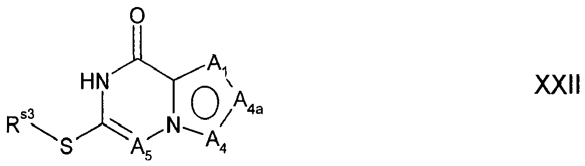 wherein A₁, A₄, A₄ₐ, A₅ and R^{s3} are as hereinbefore defined (and R^{s3} represents a group defined by R^{s2} and is preferably methyl), under halogenation reaction conditions such as those described herein, e.g. in the presence POCl₃.

Compounds of formula XIV in which L³ represents halo (especially chloro) may be prepared by reaction of a compound of formula XXIII, 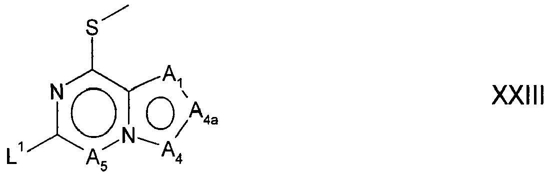 wherein L¹, A₁, A₄, A₄ₐ and A₅ are as hereinbefore defined, for example, in the presence of a base such as a metal hydroxide (e.g. KOH), in the presence of solvent (e.g. an alcohol such as methanol), followed by isolation of any intermediate product and then reaction under conditions such as those hereinbefore described in respect of preparation of compounds of formula IV (e.g. the conditions deployed in the reaction of a compound of formula XV in the presence of POCl₃, which reaction mixture may be heated at reflux for an appropriate period of time).

Compounds of formula XV may be prepared by reaction of a corresponding compound of formula XXIV, 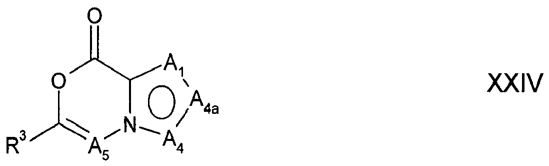 wherein A₁, A₄, A₄ₐ, A₅ and R³ as hereinbefore defined, in the presence of a suitable reagent for the replacement of the -O- moiety with a -N(H)- moiety, for example ammonia or a source thereof (e.g. ammonium acetate), under standard reaction conditions, for instance optionally in the presence of a suitable solvent (e.g. acetic acid), at elevated temperature (e.g. at about 160°C under microwave irradiation reaction conditions).

Compounds of formula XV in which A₅ represents C(R²) (and preferably, A₁ represents C(R¹), A₄ represents N, A₄ₐ represents C(R^{1b}) and A₅ represents C(H); further, R^{1b} may represent -C(O)OR^{10a}) may also be prepared by reaction of a compound of formula XXV, 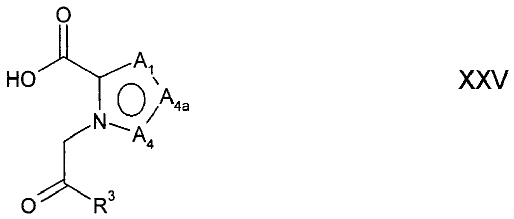 or a derivative thereof (e.g. a carboxylic acid ester such as a -C(O)O-ethyl, for instance A₄ₐ may represent -C(R^{1b}), in which R^{1b} represents -C(O)OR^{10a} and R^{10a} is preferably ethyl), wherein A₁, A₄, A₄ₐ and R³ are as hereinbefore defined (but, preferably, A₁ represents C(R¹), A₄ represents N and A₄ₐ represents C(R^{1b}), in which R^{1b} may represent -C(O)OR^{10a}), with a source of ammonia, such as ammonium acetate, for example under reaction conditions such as those described herein (e.g. above). The skilled person will appreciate that R² may represent hydrogen or an R² substituent may be pending on the -CH₂- moiety bridging the R³-C(O)- moiety and the N of the 5-membered heterocycle.

Compounds of formula XV, or protected derivatives thereof (which includes salts, e.g. a bromide salt), in which A₅ represents C(R²) (and, preferably, A₄ₐ represents N and/or, preferably, A₁ represents C(R¹) and A₄ represents C(R^{1a})) may be prepared by reaction of a compound of formula XXVI, 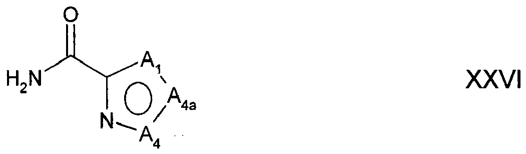 or a protected derivative thereof, e.g. a methyl protected derivative thereof, for instance, when the A₁ to A₄ₐ-containing ring represents an imidazole ring (i.e. A₄ₐ represents N, and the other ring members are C, then the N at A₄ₐ may be protected, e.g. by a methyl group, so forming for example 1-methyl-1H-imidazole-4-carboxamide) and wherein A₁, A₄ and A₄ₐ are as hereinbefore defined, with a compound of formula XXVII,

L¹²-C(H)(R²)-C(O)-R³ XXVII

wherein L¹² represents a suitable leaving group, such as one hereinbefore defined in respect of L¹ (e.g. halo, preferably, bromo), and R² and R³ are as hereinbefore defined (and R² is preferably hydrogen), for example at elevated temperature (e.g at reflux) in the presence of an appropriate solvent (e.g. acetonitrile, dimethylformamide, and the like, or mixtures thereof).

Compounds of formula XV, in which A₅ represents N (e.g. A₁, A₄ and A₄ₐ respectively represent C(R¹), C(R^{1a}) and C(R^{1b}) and A₅ represents N, or, A₁ represents N(R^{1x}), A₄ represents C(R^{1a}) and A₄ₐ and A₅ both represent N) may also be prepared by intramolecular cyclisation of a compound of formula XXVIII, 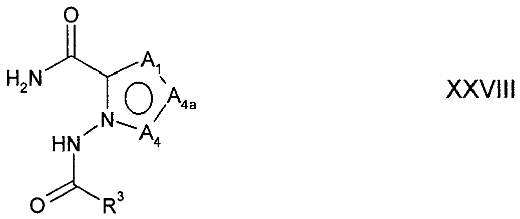 wherein R³, A₁, A₄ and A₄ₐ are as hereinbefore defined, by reaction in the presence of a base, for instance an aqueous basic solution such as ammonium hydroxide, or a metal alkyl-oxide (e.g. potassium tert-butoxide) in an alcoholic solution (e.g. butanol), for instance at elevated temperature e.g. at about 120°C under microwave irradiation reaction conditions.

Compounds of formula XV, in which R³ is replaced with a -OH group and A₅ represents N (e.g. A₁, A₄ and A₄ₐ respectively represent C(R¹), C(R^{1a}) and C(R^{1b}) and A₅ represents N, or, A₁ represents N(R^{1x}), A₄ represents C(R^{1a}) and A₄ₐ and A₅ both represent N) may also be prepared by reaction of a compound of formula XXIX, 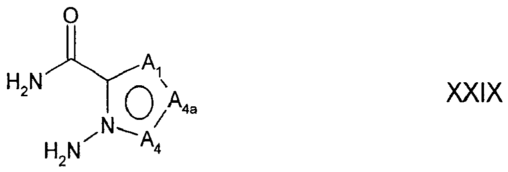 wherein A₁, A₄ and A₄ₐ are as hereinbefore defined, with phosgene, triphosgene, carbonyl diimidazole, or the like, i.e. another suitable reagent that acts as a similar source of a carbonyl group, under reaction conditions such as those described hereinafter. Such amido-compounds may be prepared by coupling of the corresponding carboxylic acid with ammonia (or a suitable source thereof, e.g. NH₄Cl in NH/MeOH).

Compounds of formula XVI may be prepared by reaction of a compound of formula XXX, 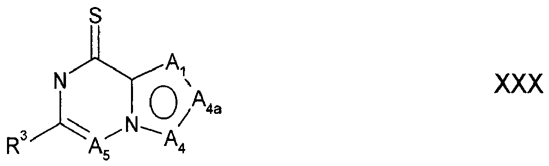 wherein A₁, A₄, A₄ₐ, A₅ and R³ are as hereinbefore defined, with a compound of formula XXXI,

R^{s2}-L¹³ XXXI

wherein L¹³ represents a suitable leaving group (such as halo, e.g. iodo) and R^{s2} is as hereinbefore defined (e.g. methyl iodide), for example in the presence of aqueous NaOH solution and an alocoholic solvent (e.g. methanol).

Compounds of formula XVI may also be prepared by intramolecular reaction of a compound of formula XXXII, 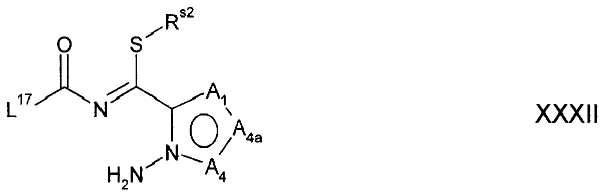 wherein L¹⁷ represents a suitable leaving group (e.g. halo, such as chloro) (or L¹⁷ may represent R³), A₁ is preferably N, A₄ is C(R^{1a}) and A₅ is C(R^{1b}), for example in the presence of base at elevated temperature.

Compounds of formula XVII may be prepared by reaction of a compound of formula XXXIII, 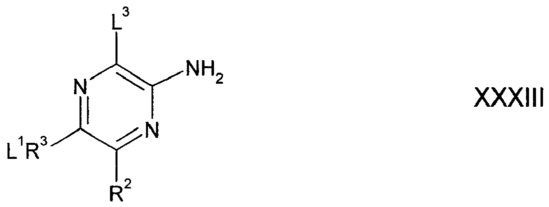 wherein R², L¹R³ and L³ are as hereinbefore defined, with a suitable aminating agent, for instance a hydroxylamine compound (e.g. a sulfonyl-hydroxylamine, such as o-(meistylsulfonyl)hydroxylamine), under standard reaction conditions.

Compounds of formula XIX in which L¹ represents chloro (or halo) may be prepared by reaction of a compound of formula XXXIV, 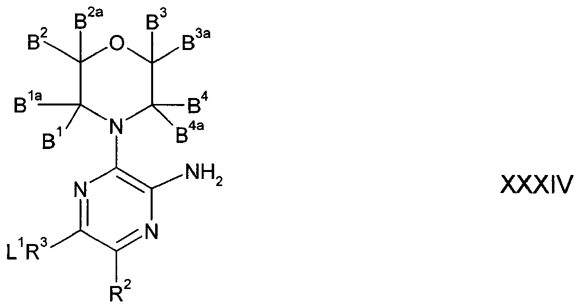 wherein L¹R³, R², B¹, B^{1a}, B², B^{2a}, B³, B^{3a}, B⁴ and B^{4a} are as hereinbefore defined, with a reagent, or mixture of reagents, that are suitable for converting the amino moiety to a chloro (or other halo) moiety, for example, TiCl₄ and tert-butyl nitrite, nuder conditions such as those described hereinafter.

Compounds of formula XXII in which A₅ represents N (and preferably in which A₅ represents N, A₄ represents C(R^{1a}), A₄ₐ represents C(R^{1b}) and A₁ represents N) may be prepared by reaction of a compound of formula XXXV, 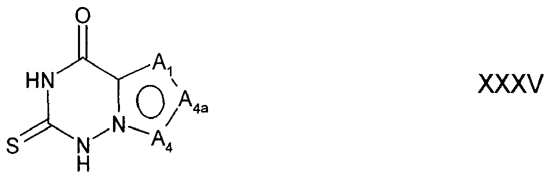 wherein A₁, A₄ and A₄ₐ are as hereinbefore defined (but, preferably, A₄ represents C(R^{1a}), A₄ₐ represents C(R^{1b}) and A₁ represents N), in the presence of a compound of formula XXXI as hereinbefore defined but in which R^{s2} represents R^{s3}.

Compounds of formula XXIV in which in which A₅ represents C(R²) (and, preferably, A₁ represents C(R¹), A₄ represents C(R^{1a}), A₄ₐ represents C(R^{1b}) and A₅ represents C(H)), may be prepared by reaction of a compound of formula XXXVI, 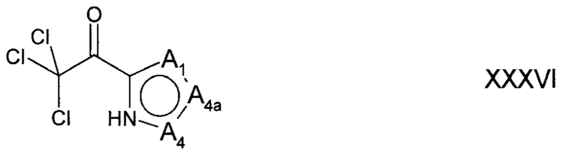 wherein A₁, A₄ and A₄ₐ are as hereinbefore defined, with a compound of formula XXXVII,

R³-C(O)-CH₂-L⁹ XXXVII

wherein L⁹ represents a suitable leaving group, for example one hereinbefore defined in respect of L¹ (e.g. halo, and preferably, bromo), under standard reaction conditions, for example, optionally in the presence of a suitable base (preferably an inorganic base, such as NaH, K₃PO₄, Cs₂CO₃, *t*-BuONa, *t*-BuOK, and, more preferably an inorganic carbonate such as Na₂CO₃ and, preferably, K₂CO₃) and a suitable solvent (e.g. an aprotic solvent such as dichloromethane or, preferably, acetone). The reaction may be performed at elevated temperature, for example, at above 100°C (e.g. at about 120°C) under microwave irradiation conditions.

Compounds of formula XXV may be prepared by reaction of a compound of formula XXXVIII, 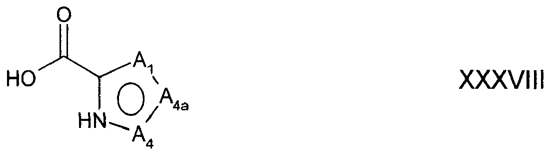 or a derivative thereof (e.g. ester such as ethyl ester), wherein A₁, A₄ and A₄ₐ are as hereinbefore defined, with a compound of formula XXXIX,

L¹⁰-CH₂-C(O)-R³ XXXIX

wherein L¹⁰ represents a suitable leaving group, such as one hereinbefore defined in respect of L¹ (e.g. halo, such as bromo), and R³ is as hereinbefore defined, under standard reaction conditions, for example optionally in the presence of a suitable base and solvent (such as those hereinbefore described in respect of preparation of compounds of formula XXIV (by reaction of a compound of formula XXXVI and XXXVII), e.g. K₂CO₃ in acetone).

Compounds of formula XXVI may be prepared by reaction of a compound of formula XXXVIII as hereinbefore defined, or a derivative thereof (e.g. an ester, such as an ethyl ester), with ammonia or a suitable source thereof (e.g. NH₄Cl in a solution of NH₃ in an alcohol such as methanol).

Compounds of formula XXVIII may be prepared by reaction of a compound of formula XXIX as hereinbefore defined with a compound of formula XL,

R³-C(O)-L¹¹ XL

wherein L¹¹ represents a suitable leaving group such as one hereinbefore defined by L¹ (e.g. halo, such as chloro) or -OH (or an ester, thereof) under standard acylation or amide coupling reaction conditions, e.g. in the case of acylation, the presence of an appropriate base (e.g. an organic amine base such as triethylamine) and an appropriate solvent (e.g. pyridine, dichloromethane, dioxane, etc, or mixtures thereof), or, in the case of amide couplings, under conditions described hereinafter (or e.g. in the presence of polyphosphoric acid, which advantageously may form a compound of formula XXVIII *in situ*, which may undergo subsequent reaction to provide the compound of formula XV isoquinolinone directly).

Compounds of formula XXIX may be prepared by (partial) hydrolysis of a compound of formula XLI, 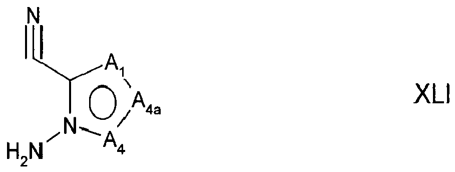 wherein A₁, A₄ and A₄ₐ are as hereinbefore defined, under standard hydrolysis reaction conditions, e.g. in the presence of an aqueous hydroxide base (e.g. potassium hydroxide) in a suitable solvent such as tetrahydrofuran.

Compounds of formula XXIX (or the corresponding carboxylic acid or ester) may also be prepared by amination of a compound of formula XXVI as hereinbefore defined, or compounds of formula XLI may also be prepared by amination of a compound of formula XLII, 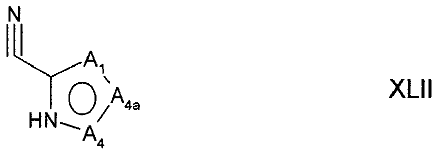 wherein A₁, A₄ and A₄ₐ are as hereinbefore defined, under reaction conditions such as those described hereinafter, e.g. in the presence of sodium hydride, followed by o-(diphenylphosphinyl)hydroxylamine.

Compounds of formula XXXIX in which L¹⁰ represents halo (e.g. chloro or, preferably, bromo) may be prepared by reaction of a compound corresponding to a compound of formula XXXIX but in which L¹⁰ represents hydrogen, with a source of halide ions (e.g. such as one hereinbefore described in respect of preparation of compounds of formula I; process step (iii) above), such as *N-*chlorosuccinimide or *N*-bromosuccinimide, under standard reaction conditions e.g. in the presence of a suitable base (such as an organic base e.g. triethylamine or the like) and trimethylsilylfluoromethanesulfonate, or the like.

Compounds corresponding to compounds of formula XXXIX but in which L¹⁰ represents hydrogen may themselves be prepared from compounds of formula XLIII,

R³-L¹¹ XLIII

in which L¹¹ represents a suitable leaving group, such as one hereinbefore defined in respect of L¹ (e.g. halo, such as chloro or, preferably bromo), with a compound that allows the introduction of the -C(O)CH₃ moiety, such as tributyl(1-ethoxyvinyl)tin in the presence of a precious metal catalyst/ligand (e.g. dichlorobis(triphenyl-phosphine)palladium (II)) and a suitable solvent (e.g. dimethylformamide, or the like).

Compounds of formula XLI may be prepared by reaction of a compound of formula XLII as hereinbefore defined, for example by reaction in the presence of base (e.g. a metal hydride, such as sodium hydride) and an appropriate reagent for the introduction of the amino group, e.g. o-(diphenylphosphinyl)-hydroxylamine, or another reagent suitable for electrophilic aminations, under reaction conditions such as those described hereinafter.

Compounds of formula XLII may be prepared by reaction of a compound of formula XLIV, 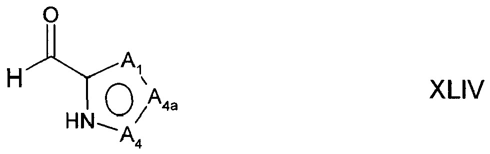 wherein A₁, A₄ and A₄ₐ are as hereinbefore defined, in the presence of hydroxylamine (e.g. the hydrochloride thereof), followed by dehydration (in the presence of a suitable dehydrating agent, such as phthalic anhydride).

Other specific transformation steps (including those that may be employed in order to form compounds of formula I) that may be mentioned include:
(i) reductions, for example of a carboxylic acid (or ester) to either an aldehyde or an alcohol, using appropriate reducing conditions (e.g. -C(O)OH (or an ester thereof), may be converted to a -C(O)H or -CH₂-OH group, using DIBAL and LiAlH₄, respectively (or similar chemoselective reducing agents));
(ii) reductions of an aldehyde (-C(O)H) group to an alcohol group (-CH₂OH), using appropriate reduction conditions such as those mentioned at point (i) above;
(iii) oxidations, for example of a moiety containing an alcohol group (e.g. -CH₂OH) to an aldehyde (e.g. -C(O)H), for example in the presence of a suitable oxidising agent, e.g. MnO₂ or the like;
(iv) reductive amination of an aldehyde and an amine, under appropriate reaction conditions, for example in "one-pot" procedure in the presence of an appropriate reducing agent, such as a chemoselective reducing agent such as sodium cyanoborohydride or, preferably, sodium triacetoxyborohydride, or the like. Alternatively, such reactions may be performed in two steps, for example a condensation step (in the presence of e.g. a dehydrating agent such as trimethyl orthoformate or MgSO₄ or molecular sieves, etc) followed by a reduction step (e.g. by reaction in the presence of a reducing agent such as a chemoselective one mentioned above or NaBH₄, AlH₄, or the like);
(v) amide coupling reactions, i.e. the formation of an amide from a carboxylic acid (or ester thereof), for example when R² represents -C(O)OH (or an ester thereof), it may be converted to a -C(O)N(R^{10b})R^{11b} group (in which R^{10b} and R^{11b} are as hereinbefore defined, and may be linked together, e.g. as defined above), and which reaction may (e.g. when R² represents -C(O)OH) be performed in the presence of a suitable coupling reagent (e.g. 1,1'-carbonyldiimidazole, *N,N'-*dicyclohexylcarbodiimide, or the like) or, in the case when R² represents an ester (e.g. -C(O)OCH₃ or -C(O)OCH₂CH₃), in the presence of e.g. trimethylaluminium, or, alternatively the -C(O)OH group may first be activated to the corresponding acyl halide (e.g -C(O)Cl, by treatment with oxalyl chloride, thionyl chloride, phosphorous pentachloride, phosphorous oxychloride, or the like), and, in all cases, the relevant compound is reacted with a compound of formula HN(R^{10a})R^{11a} (in which R^{10a} and R^{11a} are as hereinbefore defined), under standard conditions known to those skilled in the art (e.g. optionally in the presence of a suitable solvent, suitable base and/or in an inert atmosphere);
(vi) conversion of a primary amide to a nitrile functional group, for example under dehydration reaction conditions, e.g. in the presence of POCl₃, or the like;
(vii) nucleophilic substitution reactions, where any nucleophile replaces a leaving group, e.g. methylsulfonylpiperazine may replace a chloro leaving group, or, aromatic nucleophilic substitution reactions such as the substitution of ammonia (or a protected derivative thereof, e.g. a dibenzyl derivative) onto an aromatic group bearing a leaving group (e.g. onto a 2-chloropyrimidinyl moiety);
(viii) transformation of a methoxy group to a hydroxy group, by reaction in the presence of an appropriate reagent, such as boron fluoride-dimethyl sulfide complex or BBr₃ (e.g. in the presence of a suitable solvent such as dichloromethane);
(ix) specific deprotection steps, for example a hydroxy group protected as a silyl ether (e.g. a *tert*-butyl-dimethylsilyl protecting group) may be deprotected by reaction with a source of fluoride ions, e.g. by employing the reagent tetrabutylammonium fluoride (TBAF).

Intermediate compounds described herein are either commercially available, are known in the literature, or may be obtained either by analogy with the processes described herein, or by conventional synthetic procedures, in accordance with standard techniques, from available starting materials using appropriate reagents and reaction conditions. Further, processes to prepare compounds of formula I may be described in the literature, for example in:
Werber,G. et al.; J. Heterocycl. Chem.; EN; 14; 1977; 823-827;
Andanappa K. Gadad et al. Bioorg. Med. Chem. 2004, 12, 5651-5659;
Paul Heinz et al. Monatshefte für Chemie, 1977, 108, 665-680;
M.A. EI-Sherbeny et al. Boll. Chim. Farm. 1997, 136, 253-256;
Nicolaou, K. C.; Bulger, P. G.; Sarlah, D. Angew. Chem. Int Ed. 2005, 44, 2-49;
Bretonnet et al. J. Med. Chem. 2007, 50, 1872 ;
Asunción Marin et al. Farmaco 1992, 47 (1), 63-75;
Severinsen, R. et al. Tetrahedron 2005, 61, 5565-5575;
Nicolaou, K. C.; Bulger, P. G.; Sarlah, D. Angew. Chem. Int. Ed. 2005, 44, 2-49;
M. Kuwahara et al., Chem. Pharm Bull., 1996, 44, 122;
Wipf, P.; Jung, J.-K. J. Org. Chem. 2000, 65(20), 6319-6337;
Shintani, R.; Okamoto, K. Org. Lett. 2005, 7 (21), 4757-4759;
Nicolaou, K. C.; Bulger, P. G.; Sarlah, D. Angew. Chem. Int. Ed. 2005, 44, 2-49;
J. Kobe et a/., Tetrahedron, 1968, 24, 239 ;
P.F. Fabio, A.F. Lanzilotti and S.A. Lang, Journal of Labelled Compounds and Pharmaceuticals, 1978, 15, 407;
F.D. Bellamy and K. Ou, Tetrahedron Lett., 1985, 25, 839;
M. Kuwahara et al., Chem. Pharm Bull., 1996, 44, 122;
A.F. Abdel-Magid and C.A Maryanoff. Synthesis, 1990, 537;
M. Schlosser et al. Organometallics in Synthesis. A Manual, (M. Schlosser, Ed.), Wiley &Sons Ltd: Chichester, UK, 2002**,** and references cited therein;
L. Wengwei et al., Tetrahedron Lett., 2006, 47, 1941;
M. Plotkin et al. Tetrahedron Lett., 2000, 41, 2269;
Seyden-Penne, J. Reductions by the Alumino and Borohydrides, VCH, NY, 1991**;**
O. C. Dermer, Chem. Rev., 1934, 14, 385;
N. Defacqz, et al., Tetrahedron Lett., 2003, 44, 9111;
S.J. Gregson et al., J. Med. Chem., 2004, 47, 1161;
A. M. Abdel Magib, et al., J. Org. Chem., 1996, 61, 3849;
A.F. Abdel-Magid and C.A Maryanoff. Synthesis, 1990, 537;
T. Ikemoto and M. Wakimasu, Heterocycles, 2001, 55, 99;
E. Abignente et al., II Farmaco, 1990, 45, 1075;
T. Ikemoto et al., Tetrahedron, 2000, 56, 7915;
T. W. Greene and P. G. M. Wuts, Protective Groups in Organic Synthesis, Wiley, NY, 1999**;**
S. Y. Han and Y.-A. Kim. Tetrahedron, 2004, 60, 2447;
J. A. H. Lainton et al., J. Comb. Chem., 2003, 5, 400; or
Wiggins, J. M. Synth. Commun., 1988, 18, 741.

The substituents R³, B¹, B^{1a}, B², B^{2a}, B³, B^{3a}, B⁴, B^{4a}, A₁, A₄, A₄ₐ and A₅ in final compounds of the invention or relevant intermediates may be modified one or more times, after or during the processes described above by way of methods that are well known to those skilled in the art. Examples of such methods include substitutions, reductions, oxidations, alkylations, acylations, hydrolyses, esterifications, etherifications, halogenations or nitrations. Such reactions may result in the formation of a symmetric or asymmetric final compound of the invention or intermediate. The precursor groups can be changed to a different such group, or to the groups defined in formula I, at any time during the reaction sequence.

For example, when substituents in the compounds of the invention (e.g. represented by R³, B¹, B^{1a}, B², B^{2a}, B³, B^{3a}, B⁴, B^{4a}, A₁, A₄, A₄ₐ and A₅) such as CO₂Et, CHO, CN and/or CH₂Cl, are present, these groups can be further derivatized to other fragments described (e.g. by those integers mentioned above) in compounds of the invention, following synthetic protocols very well know to the person skilled in the art and/or according to the experimental part described in the patent. Other specific transformation steps that may be mentioned include: the reduction of a nitro or azido group to an amino group; the hydrolysis of a nitrile group to a carboxylic acid group; and standard nucleophilic aromatic substitution reactions, for example in which an iodo-, preferably, fluoro-or bromo-phenyl group is converted into a cyanophenyl group by employing a source of cyanide ions (e.g. by reaction with a compound which is a source of cyano anions, e.g. sodium, copper (I), zinc or potassium cyanide, optionally in the presence of a palladium catalyst) as a reagent (alternatively, in this case, palladium catalysed cyanation reaction conditions may also be employed).

Other transformations that may be mentioned include: the conversion of a halo group (preferably iodo or bromo) to a 1-alkynyl group (e.g. by reaction with a 1-alkyne), which latter reaction may be performed in the presence of a suitable coupling catalyst (e.g. a palladium and/or a copper based catalyst) and a suitable base (e.g. a tri-(C₁₋₆ alkyl)amine such as triethylamine, tributylamine or ethyldiisopropylamine); the introduction of amino groups and hydroxy groups in accordance with standard conditions using reagents known to those skilled in the art; the conversion of an amino group to a halo, azido or a cyano group, for example *via* diazotisation (e.g. generated *in situ* by reaction with NaNO₂ and a strong acid, such as HCl or H₂SO₄, at low temperature such as at 0°C or below, e.g. at about -5°C) followed by reaction with the appropriate nucleophile e.g. a source of the relevant anions, for example by reaction in the presence of a halogen gas (e.g. bromine, iodine or chlorine), or a reagent that is a source of azido or cyanide anions, such as NaN₃ or NaCN; the conversion of -C(O)OH to a -NH₂ group, under Schmidt reaction conditions, or variants thereof, for example in the presence of HN₃ (which may be formed in by contacting NaN₃ with a strong acid such as H₂SO₄), or, for variants, by reaction with diphenyl phosphoryl azide ((PhO)₂P(O)N₃) in the presence of an alcohol, such as *tert*-butanol, which may result in the formation of a carbamate intermediate; the conversion of -C(O)NH₂ to -NH₂, for example under Hofmann rearrangement reaction conditions, for example in the presence of NaOBr (which may be formed by contacting NaOH and Br₂) which may result in the formation of a carbamate intermediate; the conversion of -C(O)N₃ (which compound itself may be prepared from the corresponding acyl hydrazide under standard diazotisation reaction conditions, e.g. in the presence of NaNO₂ and a strong acid such as H₂SO₄ or HCl) to -NH₂, for example under Curtius rearrangement reaction conditions, which may result in the formation of an intermediate isocyanate (or a carbamate if treated with an alcohol); the conversion of an alkyl carbamate to -NH₂, by hydrolysis, for example in the presence of water and base or under acidic conditions, or, when a benzyl carbamate intermediate is formed, under hydrogenation reaction conditions (e.g. catalytic hydrogenation reaction conditions in the presence of a precious metal catalyst such as Pd); halogenation of an aromatic ring, for example by an electrophilic aromatic substitution reaction in the presence of halogen atoms (e.g. chlorine, bromine, etc, or an equivalent source thereof) and, if necessary an appropriate catalyst/Lewis acid (e.g. AlCl₃ or FeCl₃).

Compounds of the invention bearing a carboxyester functional group may be converted into a variety of derivatives according to methods well known in the art to convert carboxyester groups into carboxamides, N-substituted carboxamides, N,N-disubstituted carboxamides, carboxylic acids, and the like. The operative conditions are those widely known in the art and may comprise, for instance in the conversion of a carboxyester group into a carboxamide group, the reaction with ammonia or ammonium hydroxide in the presence of a suitable solvent such as a lower alcohol, dimethylformamide or a mixture thereof; preferably the reaction is carried out with ammonium hydroxide in a methanol/dimethylformamide mixture, at a temperature ranging from about 50°C to about 100°C. Analogous operative conditions apply in the preparation of N-substituted or N,N-disubstituted carboxamides wherein a suitable primary or secondary amine is used in place of ammonia or ammonium hydroxide. Likewise, carboxyester groups may be converted into carboxylic acid derivatives through basic or acidic hydrolysis conditions, widely known in the art. Further, amino derivatives of compounds of the invention may easily be converted into the corresponding carbamate, carboxamido or ureido derivatives.

Hydroxamic acid isosteres may be prepared in accordance with techniques known to those skilled in the art, e.g. from common general knowledge or from the disclosures of international patent application WO 2010/080996.

Compounds of the invention may be isolated from their reaction mixtures using conventional techniques (e.g. recrystallisations).

It will be appreciated by those skilled in the art that, in the processes described above and hereinafter, the functional groups of intermediate compounds may need to be protected by protecting groups.

The need for such protection will vary depending on the nature of the remote functionality and the conditions of the preparation methods (and the need can be readily determined by one skilled in the art). Suitable amino-protecting groups include acetyl, trifluoroacetyl, t-butoxycarbonyl (BOC), benzyloxycarbonyl (CBz), 9-fluorenylmethyleneoxycarbonyl (Fmoc) and 2,4,4-trimethylpentan-2-yl (which may be deprotected by reaction in the presence of an acid, e.g. HCl in water/alcohol (e.g. MeOH)) or the like. The need for such protection is readily determined by one skilled in the art.

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described herein may be converted chemically to unprotected compounds using standard deprotection techniques.

The type of chemistry involved will dictate the need, and type, of protecting groups as well as the sequence for accomplishing the synthesis.

The use of protecting groups is fully described in "Protective Groups in Organic Synthesis", 3rd edition, T.W. Greene & P.G.M. Wutz, Wiley-Interscience (1999).

### Medical and Pharmaceutical Uses

Compounds of the invention are indicated as pharmaceuticals. According to a further aspect of the invention there is provided a compound of the invention, as hereinbefore defined, for use as a pharmaceutical.

Compounds of the invention may inhibit protein or lipid kinases, such as a PI3 kinase (especially a class I P13K), for example as may be shown in the tests described below (for example, the test for PI3Kα inhibition described below) and/or in tests known to the skilled person. The compounds of the invention may also inhibit mTOR. Additionally, the compounds of the invention may inhibit HDAC. Thus, the compounds of the invention may be useful in the treatment of those disorders in an individual in which the inhibition of such protein or lipid kinases (e.g. PI3K, particularly class I PI3K, and/or mTOR) and, additionally, HDAC, is desired and/or required (for instance compounds of the invention may inhibit HDAC and PI3K, particularly class I PI3K and, optionally, may also inhibit mTOR).

The term "inhibit" may refer to any measurable reduction and/or prevention of enzyme activity (e.g. HDAC) or catalytic kinase (e.g. PI3K, particularly class I PI3K, and/or mTOR) activity. The reduction and/or prevention of enzyme/kinase activity may be measured by comparing the kinase activity in a sample containing a compound of the invention and an equivalent sample of enzyme/kinase (e.g. HDAC and PI3K, particularly class I PI3K, and/or mTOR) in the absence of a compound of the invention, as would be apparent to those skilled in the art. The measurable change may be objective (e.g. measurable by some test or marker, for example in an *in vitro* or *in vivo* assay or test, such as one described hereinafter, or otherwise another suitable assay or test known to those skilled in the art) or subjective (e.g. the subject gives an indication of or feels an effect).

Compounds of the invention may be found to exhibit 50% inhibition of a protein or lipid kinase (e.g. PI3K, such as class I PI3K, and/or mTOR) at a concentration of 100 µM or below (for example at a concentration of below 50 µM, or even below 10 µM, such as below 1 µM), when tested in an assay (or other test), for example as described hereinafter, or otherwise another suitable assay or test known to the skilled person. Additionally compounds of the invention may be found to exhibit 50% inhibition of an enzyme such as HDAC at a concentration of 100 uM or below (for example at a concentration of below 50 µM, or even below 10 µM, such as below 1 µM), when tested in an assay (or other test), for example as described hereinafter, or otherwise another suitable assay or test known to the skilled person.

Compounds of the invention are thus expected to be useful in the treatment of a disorder in which a protein or lipid kinase (e.g. PI3K, such as class I PI3K, and/or mTOR) and additionally an enzyme such as HDAC is known to play a role and which are characterised by or associated with an overall elevated activity of that kinase (due to, for example, increased amount of the kinase or increased catalytic activity of the kinase). Hence, compounds of the invention are expected to be useful in the treatment of a disease/disorder arising from abnormal cell growth, function or behaviour associated with the protein or lipid kinase (e.g. PI3K, such as class I PI3K, and/or mTOR) and/or the enzyme such as HDAC. Such conditions/disorders include cancer, immune disorders, cardiovascular diseases, viral infections, inflammation, metabolism/endocrine function disorders and neurological disorders.

The disorders/conditions that the compounds of the invention may be useful in treating hence includes cancer (such as lymphomas, solid tumours or a cancer as described hereinafter), obstructive airways diseases, allergic diseases, inflammatory diseases (such as asthma, allergy and Chrohn's disease), immunosuppression (such as transplantation rejection and autoimmune diseases), disorders commonly connected with organ transplantation, AIDS-related diseases and other associated diseases. Other associated diseases that may be mentioned (particularly due to the key role of kinases in the regulation of cellular proliferation) include other cell proliferative disorders and/or non-malignant diseases, such as benign prostate hyperplasia, familial adenomatosis, polyposis, neuro-fibromatosis, psoriasis, bone disorders, atherosclerosis, vascular smooth cell proliferation associated with atherosclerosis, pulmonary fibrosis, arthritis glomerulonephritis and post-surgical stenosis and restenosis. Other disease states that may be mentioned include cardiovascular disease, stroke, diabetes, hepatomegaly, Alzheimer's disease, cystic fibrosis, hormone-related diseases, immunodeficiency disorders, destructive bone disorders, infectious diseases, conditions associated with cell death, thrombin-induced platelet aggregation, chronic myelogenous leukaemia, liver disease, pathologic immune conditions involving T cell activation and CNS disorders.

As stated above, the compounds of the invention may be useful in the treatment of cancer. More, specifically, the compounds of the invention may therefore be useful in the treatment of a variety of cancer including, but not limited to: carcinoma such as cancer of the bladder, breast, colon, kidney, liver, lung (including non-small cell cancer and small cell lung cancer), esophagus, gall-bladder, ovary, pancreas, stomach, cervix, thyroid, prostate, skin, squamous cell carcinoma, testis, genitourinary tract, larynx, glioblastoma, neuroblastoma, keratoacanthoma, epidermoid carcinoma, large cell carcinoma, non-small cell lung carcinoma, small cell lung carcinoma, lung adenocarcinoma, bone, adenoma, adenocarcinoma, follicular carcinoma, undifferentiated carcinoma, papilliary carcinoma, seminona, melanoma, sarcoma, bladder carcinoma, liver carcinoma and biliary passages, kidney carcinoma, myeloid disorders, lymphoid disorders, hairy cells, buccal cavity and pharynx (oral), lip, tongue, mouth, pharynx, small intestine, colon-rectum, large intestine, rectum, brain and central nervous system, Hodgkin's and leukaemia; hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; and other tumors, including melanoma, seminoma, teratocarcinoma, osteosarcoma, xeroderma pigmentosum, keratoxanthoma, thyroid follicular cancer and Kaposi's sarcoma. Further, the protein or lipid kinases (e.g. PI3K, such as class I PI3K, and/or mTOR) (and additionally HDAC) may also be implicated in the multiplication of viruses and parasites. They may also play a major role in the pathogenesis and development of neurodegenerative disorders. Hence, compounds of the invention may also be useful in the treatment of viral conditions, parasitic conditions, as well as neurodegenerative disorders.

Compounds of the invention are indicated both in the therapeutic and/or prophylactic treatment of the above-mentioned conditions.

According to a further aspect of the present invention, there is provided a method of treatment of a disease (e.g. cancer or another disease as mentioned herein) which is associated with the inhibition of protein or lipid kinase (e.g. PI3K, such as class I PI3K, and/or mTOR) and, additionally, an enzyme such as HDAC, i.e. where such inhibition is desired and/or required (for example, a method of treatment of a disease/disorder arising from abnormal cell growth, function or behaviour associated with protein or lipid kinases, e.g. PI3K, such as class I PI3K, and/or mTOR, or with an enzyme such as HDAC), which method comprises administration of a therapeutically effective amount of a compound of the invention, as hereinbefore defined, to a patient suffering from, or susceptible to, such a condition.

"Patients" include mammalian (including human) patients. Hence, the method of treatment discussed above may include the treatment of a human or animal body.

The term "effective amount" refers to an amount of a compound, which confers a therapeutic effect on the treated patient. The effect may be objective (e.g. measurable by some test or marker) or subjective (e.g. the subject gives an indication of or feels an effect).

Compounds of the invention may be administered orally, intravenously, subcutaneously, buccally, rectally, dermally, nasally, tracheally, bronchially, sublingually, by any other parenteral route or via inhalation, in a pharmaceutically acceptable dosage form.

Compounds of the invention may be administered alone, but are preferably administered by way of known pharmaceutical formulations, including tablets, capsules or elixirs for oral administration, suppositories for rectal administration, sterile solutions or suspensions for parenteral or intramuscular administration, and the like. The type of pharmaceutical formulation may be selected with due regard to the intended route of administration and standard pharmaceutical practice. Such pharmaceutically acceptable carriers may be chemically inert to the active compounds and may have no detrimental side effects or toxicity under the conditions of use.

Such formulations may be prepared in accordance with standard and/or accepted pharmaceutical practice. Otherwise, the preparation of suitable formulations may be achieved non-inventively by the skilled person using routine techniques and/or in accordance with standard and/or accepted pharmaceutical practice.

According to a further aspect of the invention there is thus provided a pharmaceutical formulation including a compound of the invention, as hereinbefore defined, in admixture with a pharmaceutically acceptable adjuvant, diluent and/or carrier.

Depending on e.g. potency and physical characteristics of the compound of the invention (i.e. active ingredient), pharmaceutical formulations that may be mentioned include those in which the active ingredient is present in at least 1% (or at least 10%, at least 30% or at least 50%) by weight. That is, the ratio of active ingredient to the other components (i.e. the addition of adjuvant, diluent and carrier) of the pharmaceutical composition is at least 1:99 (or at least 10:90, at least 30:70 or at least 50:50) by weight.

The amount of compound of the invention in the formulation will depend on the severity of the condition, and on the patient, to be treated, as well as the compound(s) which is/are employed, but may be determined non-inventively by the skilled person.

The invention further provides a process for the preparation of a pharmaceutical formulation, as hereinbefore defined, which process comprises bringing into association a compound of the invention, as hereinbefore defined, or a pharmaceutically acceptable ester, amide, solvate or salt thereof with a pharmaceutically-acceptable adjuvant, diluent or carrier.

Compounds of the invention may also be combined with other therapeutic agents that are inhibitors of protein or lipid kinases (e.g. PI3K, such as class I PI3K, a PIM family kinase (e.g. PIM-1, PIM-2- and/or PIM-3) and/or mTOR), inhibitors of other enzymes such as HDAC and/or useful in the treatment of a cancer and/or a proliferative disease. Compounds of the invention may also be combined with other therapies (e.g. radiation).

For instance, compounds of the invention may be combined with one or more treatments independently selected from surgery, one or more anti-cancer/antineoplastic/anti-tumoral agent, one or more hormone therapies, one or more antibodies, one or more immunotherapies, radioactive iodine therapy, and radiation.

More specifically, compounds of the invention may be combined with an agent that modulates the Ras/Raf/Mek pathway (e.g. an inhibitor of MEK), the Jak/Stat pathway (e.g. an inhibitor of Jak), the PI3K/Akt pathway (e.g. an inhibitor of Akt), the DNA damage response mechanism (e.g. an inhibitor of ATM or ATR) or the stress signaling pathway (an inhibitor of p38 or NF-KB).

For instance, compounds of the invention may be combined with:
(i) a targeted kinase inhibitor;
(ii) a receptor tyrosine kinase (RTK) inhibitor;
(iii) a PIM family kinase inhibitor, such as SGI-1776;
(iv) an Flt-3 inhibitor;
(v) an EGFR or HER2 inhibitor, such as lapatanib;
(vi) a therapeutic monoclonal antibody, such as the HER2 inhibitor trastuzumab;
(vii) a MEK inhibitor, such as PD-0325901;
(vii) a BRaf inhibitor, such as GDC-0879;
(viii) an anthracyclin, such as doxorubicin;
(ix) a taxane, such as paclitaxel or, particularly, docetaxel;
(x) a platin, such as carboplatin or, particularly, cisplatin;
(xi) a nucleotide analog, such as 5-fluorouracil (5-FU) or gemcitabine);
(xii) an alkylating agent, such as temozolomide;
(xiii) a hormone therapeutic agent, such as an estrogen receptor antagonist e.g. tamoxifen;
(xiv) an anti-tumour compound that has potential radiosensitising and/or chemosensitising effects, such as chloroquine;
(xv) an mTOR inhibitor, such as rapamycin;
(xvi) an Akt or PI3-K inhibitor, such as GDC-0941;
(xvii) a JAK inhibitor; and/or
(xviii) an agent that modulates the DNA damage response mechanism and/or the stress signaling pathway, e.g. an inhibitor of ATM or ATR, an inhibitor of p38 and/or NF-KB.

According to a further aspect of the invention, there is provided a combination product comprising:
(A) a compound of the invention, as hereinbefore defined; and
(B) another therapeutic agent that is useful in the treatment of cancer and/or a proliferative disease,
wherein each of components (A) and (B) is formulated in admixture with a pharmaceutically-acceptable adjuvant, diluent or carrier.

Such combination products provide for the administration of a compound of the invention in conjunction with the other therapeutic agent, and may thus be presented either as separate formulations, wherein at least one of those formulations comprises a compound of the invention, and at least one comprises the other therapeutic agent, or may be presented (i.e. formulated) as a combined preparation (i.e. presented as a single formulation including a compound of the invention and the other therapeutic agent).

Thus, there is further provided:
(1) a pharmaceutical formulation including a compound of the invention, as hereinbefore defined, another therapeutic agent that is useful in the treatment of cancer and/or a proliferative disease, and a pharmaceutically-acceptable adjuvant, diluent or carrier; and
(2) a kit of parts comprising components:
   (a) a pharmaceutical formulation including a compound of the invention, as hereinbefore defined, in admixture with a pharmaceutically-acceptable adjuvant, diluent or carrier; and
   (b) a pharmaceutical formulation including another therapeutic agent that is useful in the treatment of cancer and/or a proliferative disease in admixture with a pharmaceutically-acceptable adjuvant, diluent or carrier,
   which components (a) and (b) are each provided in a form that is suitable for administration in conjunction with the other.

In a particularly preferred aspect of the invention, compounds of the invention may be combined with other therapeutic agents (e.g. chemotherapeutic agents) for use as medicaments (e.g. for use in the treatment of a disease or condition as mentioned herein, such as one in which the inhibition of growth of cancer cells are required and/or desired e.g. for treating hyperproliferative disorders such as cancer (e.g. specific cancers that may be mentioned herein, e.g. in the examples) in mammals, especially humans). Such active ingredients in combinations may act in synergy.

In particular, compounds of the invention may be combined with known chemotherapeutic agents (as may be demonstrated by the examples, for instance where a compound of the examples is employed in combination and inhibits cellular proliferation *in vitro*; in particular such combinations may be useful in treating lung and/or ovarian cancer), for instance:
(i) a MEK inhibitor, such as PD-0325901;
(ii) an EGFR inhibitor, such as Lapatinib; and/or
(iii) docetaxel (Taxotere®, Sanofi-Aventis).

The MEK inhibitor PD-0325901 (CAS RN 391210-10-9, Pfizer) is a second-generation, non-ATP competitive, allosteric MEK inhibitor for the potential oral tablet treatment of cancer (US6960614; US 6972298; US 2004/1147478; US 2005/085550). Phase II clinical trials have been conducted for the potential treatment of breast tumors, colon tumors, and melanoma. PD-0325901 is named (R)-N-(2,3-dihydroxypropoxy)-3,4-difluoro-2-(2-fluoro-4-iodophenylamino)benzamide, and has the structure: 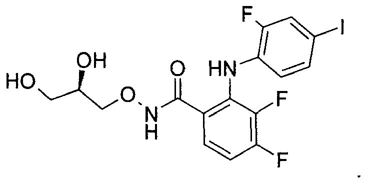 Docetaxel (TAXOTERE®, Sanofi-Aventis) is used to treat breast, ovarian, and NSCLC cancers (US 4814470; US 5438072; US 5698582; US 5714512; US 5750561; Mangatal et al (1989) Tetrahedron 45:4177; Ringel et al (1991) J. Natl. Cancer Inst. 83:288; Bissery et al(1991) Cancer Res. 51:4845; Herbst et al (2003) Cancer Treat. Rev. 29:407-415; Davies et al (2003) Expert. Opin. Pharmacother. 4:553-565). Docetaxel is named as (2R,3S)-N-carboxy-3-phenylisoserine, N-tert-butyl ester, 13-ester with 5, 20-epoxy-1, 2, 4, 7, 10, 13-hexahydroxytax-11-en-9-one 4-acetate 2-benzoate, trihydrate (US 4814470; EP 253738; CAS Reg. No. 114977-28-5) (or named as 1,7β,10β-trihydroxy-9-oxo-5β,20-epoxytax-11-ene-2α,4,13α-triyl 4-acetate 2-benzoate 13-{(2*R*,3*S*)-3-[(*tert-*butoxycarbonyl)amino]-2-hydroxy-3-phenylpropanoate}) and has the structure: 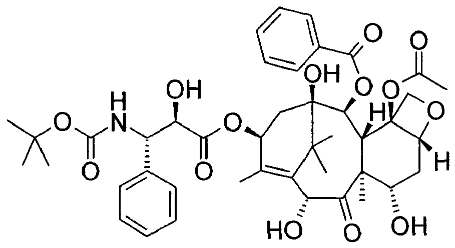 Lapatinib (TYKERB®, GW572016, Glaxo SmithKline) has been approved for use in combination with capecitabine (XELODA®, Roche) for the treatment of patients with advanced or metastatic breast cancer whose tumors over-express HER2 (ErbB2) and who have received prior therapy including an anthracycline, a taxane and trastuzumab. Lapatinib is an ATP-competitive epidermal growth factor (EGFR) and HER2/neu (ErbB-2) dual tyrosine kinase inhibitor (US 6727256; US 6713485; US 7109333; US 6933299; US 7084147; US 7157466; US 7141576) which inhibits receptor autophosphorylation and activation by binding to the ATPbinding pocket of the EGFRIHER2 protein kinase domain. Lapatinib is named as N-(3-chloro-4-(3-fluorobenzyloxy)phenyl)-6-(5-((2-(methylsulfonyl)ethylamino)-methyl)furan-2-yl)quinazolin-4-amine (or alternatively named as *N*-[3-chloro-4-[(3-fluorophenyl)methoxy]phenyl]-6-[5-[(2-methylsulfonylethylamino)methyl]-2-furyl] quinazolin-4-amine), and has the structure:

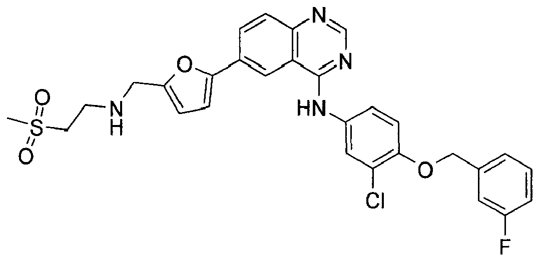

The invention further provides a process for the preparation of a combination product as hereinbefore defined, which process comprises bringing into association a compound of the invention, as hereinbefore defined, or a pharmaceutically acceptable ester, amide, solvate or salt thereof with the other therapeutic agent that is useful in the treatment of cancer and/or a proliferative disease, and at least one pharmaceutically-acceptable adjuvant, diluent or carrier.

For instance, compounds of the invention may be combined with a chemotherapeutic agent. A "chemotherapeutic agent" is a biological (large molecule) or chemical (small molecule) compound useful in the treatment of cancer, regardless of mechanism of action. Classes of chemotherapeutic agents include, but are not limited to: alkylating agents, antimetabolites, spindle poison plant alkaloids, cytotoxic/antitumor antibiotics, topoisomerase inhibitors, proteins, antibodies, photosensitizers, and kinase inhibitors. Chemotherapeutic agents include compounds used in "targeted therapy" and non-targeted, conventional chemotherapy.

Examples of chemotherapeutic agents include those mentioned in e.g. WO 2010/105008, for instance: dexamethasone, thioTEPA, doxorubicin, vincristine, rituximab, cyclophosphamide, prednisone, melphalan, lenalidomide, bortezomib, rapamycin, and cytarabine.

Examples of chemotherapeutic agents also include: erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine, dichloroplatinum(II), CAS No. 15663-27-1), carboplatin (CAS No. 41575-94-4), paclitaxel (TAXOL®, Bristol-Myers Squibb Oncology), temozolomide (4-methyl-5-oxo-2,3,4,6,8-pentazabicyclo [4.3.0]nona-2,7,9-triene-9-carboxamide, CAS No. 85622-93-1, TEMODAR®, TEMODAL®, Schering Plough), tamoxifen ((Z)-2-[4-(1,2-diphenylbut-1-enyl)phenoxy]-N,N-dimethyl-ethanamine, NOLVADEX®, ISTUBAL®, VALODEX®), doxorubicin (ADRIAMYCIN®), Akti-1/2, HPPD, rapamycin, and lapatinib (TYKERB®, Glaxo SmithKline).

More examples of chemotherapeutic agents include: oxaliplatin (ELOXATIN®, Sanofi), bortezomib (VELCADE®, Millennium Pharm.), sutent (SUNITINIB®, SU11248, Pfizer), letrozole (FEMARA®, Novartis), imatinib mesylate (GLEEVEC®, Novartis), XL-518 (MEK inhibitor, Exelixis, WO 2007/044515), ARRY - 8 8 6 (MEK inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), ABT-869 (multi-targeted inhibitor of VEGF and PDGF family receptor tyrosine kinases, Abbott Laboratories and Genentech), ABT-263 (Bc1-2/Bcl-xL inhibitor, Abbott Laboratories and Genentech), PTK787/ZK 222584 (Novartis), fulvestrant (FASLODEX®, AstraZeneca), leucovorin (folinic acid), lonafamib (SARASAR^{™}, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA^{™}, Johnson & Johnson), capecitabine (XELODA®, Roche), ABRAXANE^{™} (Cremophor-free), albumin-engineered nanoparticle formulations of paclitaxel (American Pharmaceutical Partners, Schaumberg, I1), vandetanib (rINN, ZD6474, ZACTIMA®, AstraZeneca), chloranmbucil, AG1478, AG1571 (SU 5271; Sugen), temsirolimus (TORISEL®, Wyeth), pazopanib (GlaxoSmithKline), canfosfamide (TELCYTA®, Telik), thioTepa and cyclosphosphamide (CYTOXAN®, NEOSAR®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); a camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, calicheamicin gamma II, calicheamicin omega II, dynemicin, dynemicin A; bisphosphonates, such as clodronate; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid,
nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; antiadrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfomithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; tiaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thioTepa; 6-thioguanine; mercaptopurine; methotrexate; platinum analogs such as cisplatin and carboplatin; vinblastine; etoposide (VP-16); ifosfamide; mitoxantrone; vincristine; vinorelbine (NAVELBINE®); novantrone; teniposide; edatrexate; daunomycin; aminopterin; ibandronate; CPT-11; topoisomerase inhibitor RFS 2000; difluoromethylomithine (DMFO); retinoids such as retinoic acid; and pharmaceutically acceptable salts, acids and derivatives of any of the above.

Also included in the definition of "chemotherapeutic agent" are: (i) antihormonal agents that act to regulate or inhibit hormone action on tumors such as antiestrogens and selective estrogen receptor modulators (SERMs), including, for example, tamoxifen (including NOLVADEX®; tamoxifen citrate), raloxifene, droloxifene, 4-hydroxytamoxifen, trioxifene, keoxifene, LY117018, onapristone, and FARESTON® (toremifine citrate); (ii) aromatase inhibitors that inhibit the enzyme aromatase, which regulates estrogen production in the adrenal glands, such as, for example, 4(5)-imidazoles, aminoglutethimide, MEGASE® (megestrol acetate), AROMASN® (exemestane; Pfizer), formestanie, fadrozole, RIVISOR® (vorozole), FEMARA® (letrozole; Novartis), and ARIMIDEX® (anastrozole; AstraZeneca); (iii) anti-androgens such as flutamide, nilutamide, bicalutamide, leuprolide, and goserelin; as well as troxacitabine (a 1,3-dioxolane nucleoside cytosine analog); (iv) protein kinase inhibitors such as MEK inhibitors (WO 2007/044515); (v) lipid kinase inhibitors; (vi) antisense oligonucleotides, particularly those which inhibit expression of genes in signaling pathways implicated in aberrant cell proliferation, for example, PKC-alpha, Raf and H-Ras, such as oblimersen (GENASENSE®, Genta Inc.); (vii) ribozymes such as VEGF expression inhibitors (e.g., ANGIOZYME®) and HER2 expression inhibitors; (viii) vaccines such as gene therapy vaccines, for example, ALLOVECTIN®, LEUVECTIN®, and VAXID®; PROLEUKN® rIL-2; topoisomerase 1 inhibitors such as LURTOTECAN®; ABARELIX® rmRH; (ix) anti-angiogenic agents such as bevacizumab (AVASTIN®, Genentech); and pharmaceutically acceptable salts, acids and derivatives of any of the above.

Also included in the definition of "chemotherapeutic agent" are therapeutic antibodies such as alemtuzumab (Campath), bevacizumab (AVASTN®, Genentech); cetuximab (ERBITUX®, Imclone); panitumumab (VECTIBIX®, Amgen), rituximab (RITUXAN®, Genentech/Biogen Idec), pertuzumab (OMNITARG^{™}, rhuMab 2C4, Genentech), trastuzumab (HERCEPTIN®, Genentech), tositumomab (Bexxar, Corixia), and the antibody drug conjugate, gemtuzumab ozogamicin (MYLOTARG®, Wyeth).

Humanised monoclonal antibodies with therapeutic potential as chemotherapeutic agents in combination with the PI3K inhibitors of the invention include: alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, rolizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

By "bringing into association", we mean that the two components are rendered suitable for administration in conjunction with each other.

Thus, in relation to the process for the preparation of a kit of parts as hereinbefore defined, by bringing the two components "into association with" each other, we include that the two components of the kit of parts may be:
(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Depending on the disorder, and the patient, to be treated, as well as the route of administration, compounds of the invention may be administered at varying therapeutically effective doses to a patient in need thereof. However, the dose administered to a mammal, particularly a human, in the context of the present invention should be sufficient to effect a therapeutic response in the mammal over a reasonable timeframe. One skilled in the art will recognize that the selection of the exact dose and composition and the most appropriate delivery regimen will also be influenced by *inter alia* the pharmacological properties of the formulation, the nature and severity of the condition being treated, and the physical condition and mental acuity of the recipient, as well as the potency of the specific compound, the age, condition, body weight, sex and response of the patient to be treated, and the stage/severity of the disease.

Administration may be continuous or intermittent (e.g. by bolus injection). The dosage may also be determined by the timing and frequency of administration. In the case of oral or parenteral administration the dosage can vary from about 0.01 mg to about 1000 mg per day of a compound of the invention.

In any event, the medical practitioner, or other skilled person, will be able to determine routinely the actual dosage, which will be most suitable for an individual patient. The above-mentioned dosages are exemplary of the average case; there can, of course, be individual instances where higher or lower dosage ranges are merited, and such are within the scope of this invention.

Compounds of the invention may have the advantage that they are effective inhibitors of protein or lipid kinases (e.g. PI3K, such as class I PI3K and/or mTOR) and, additionally, inhibitors of HDAC. The compounds therefore have the advantage that they are (at least) dual inhibitors. In an embodiment, compounds of the invention may have the advantage that they are both PI3K (e.g. class I PI3K, such as PI3Kα) inhibitors and mTOR inhibitors and, additionally, HDAC inhibitors, i.e. they may exhibit dual or triple inhibition (dual kinase inhibition and, additionally, inhibition of HDAC).

Compounds of the invention may also have the advantage that they may be more efficacious than, be less toxic than, be longer acting than, be more potent than, produce fewer side effects than, be more easily absorbed than, and/or have a better pharmacokinetic profile (e.g. higher oral bioavailability and/or lower clearance) than, and/or have other useful pharmacological, physical, or chemical properties over, compounds known in the prior art, whether for use in the above-stated indications or otherwise.

As stated hereinbefore, compounds of the invention may have the advantage that they may exhibit dual or triple inhibitory activity (e.g. may act as inhibitors of PI3K (such PI3Kα), mTOR and HDAC). In this respect, advantageously, compounds of the invention may be considered as multi-targeted inhibitors (multi-targeted, or dual targeted, kinase inhibitors and, additionally, HDAC inhibitors). Compounds of the invention that exhibit single selectivity for a target may have the additional benefit that they exhibit less side effects, whereas compounds of the invention that exhibit multiple target selectivity may have the additional benefit that they exhibit better potency and/or efficacy. Compounds of the invention may not target other kinases (i.e. they may be selective for PI3K and/or mTOR; in addition to being HDAC inhibitors).

To date, clinical development of PI3K and dual PI3K/mTOR inhibitors have shown moderate activities, suggesting that either more potent/efficacious inhibitors are required or that inhibition of multiple targets or even pathways might be required for effective treatments (see e.g. Bunney, Tom D., Katan, Matilda, Phosphoinositide signalling in cancer: beyond PI3K and PTEN, Nature Reviews Cancer (2010), 10(5), 342-352; Cleary, James M. and Shapiro, Geoffrey I., Development of phosphoinositide-3 kinase pathway inhibitors for advanced cancer, Current Oncology Report (2010), 12, 87-94; and van der Heijden, Michiel S. and Bernards, René; Inhibition of the PI3K Pathway: Hope We Can Believe in? Clinical Cancer Research (2010),16, 3094-3099).

Advantageously, the compounds of the invention may have the benefit that they inhibit multiple targets (or even multiple pathways). For instance, in addition to being inhibitors of PI3K (e.g PI3Kα) and mTOR (and HDAC inhibitors), they may also be effective inhibitors of other protein or lipid kinases or other enzymes. In this respect, compounds of the invention may be considered to have an improved kinase inhibition cross-reactivity profile, e.g. by being selective against multiple kinases of therapeutic interest, for instance compared to compounds known in the prior art. For instance, they may have a favorable activity profile and therefore have advantages in the clinic.

Synergistic effects have been reported for inhibitors of the PI3K/Akt/mTOR signaling pathway in combination with inhibitors of the RAF/MEK pathway (see "The future of targeted therapy approaches in melanoma", L.P.Cantini et a/., Expert Opin Drug Discov 2009, 4, 445). Targeted inhibition of receptor tyrosine kinases (RTK) often leads to drug resistance (see "FLT3 inhibition and mechanisms of drug resistance in mutant FLT3-positive AML", E. Weisberg et al., Drug Resistance Updates 2009, 12, 81-89). Spontaneous mutations of Flt3 (and Kit) and other RTK (like VEGFR and PDGFR) have been shown to activate the PI3K/Akt/mTOR pathway, and combination studies of PI3K and/or mTOR inhibitors with RTK inhibitors like sorafenib, sunitinib, gefitini, erlotinib and others have led to synergistic effects (see "Efficacy and mechanisms of apoptosis induction by simultaneous inhibition of PI3K with GDC-0941 and blockade of Bcl-2 (ABT-737) or Flt3 (sorafenib) in AML cells in the hypoxic bone marrow microenvironment", L. Jin et al., 52nd Ash annual meeting, 2010 and "Activation of PI3K/Akt signaling pathway mediates acquired resistance to sorafenib in hepatocellular carcinoma cells", K.-F. Chen et al., Journal of Pharmacology and Experimental Therapeutics 2010 (online); see also Fan, Qi-Wen and Weiss, William A., Targeting the RTK-PI3K-mTOR Axis in Malignant Glioma: Overcoming Resistance; Curr Top Microbiol Immunol. (2010), 347, 279-296; Agarval, Roshan et al, PI3K pathway-directed therapeutic strategies in cancer, Current Opinion in Investigational Drugs (2010), 11 (6), 615-628, as well as the Weisberg *et al* reference above). Wu and Hu, PI3K/Akt/mTOR Pathway Inhibitors in Cancer: A Perspective on Clinical Progress, Current Medicinal Chemistry, 2010, 17, 4326-4341 also provides a review on this topic.

Compounds of the invention may therefore combine dual PI3K/mTOR activity with activity on other key enzymes such as HDAC (indeed, combination products covering this spectrum of kinases are currently being evaluated as mentioned above), thereby allowing single-agent administration (or, potentially, combination products with reduced dosages) and providing the associated benefits, e.g. reducing the risk of drug-drug interactions, etc.

Compounds of the invention may be beneficial as they are medicaments with targeted therapy, i.e. which target a particular molecular entity by inferring or inhibiting it (e.g. in this case by inhibiting one or more protein or lipid kinases as hereinbefore described). Compounds of the invention may therefore also have the benefit that they have a new effect (for instance as compared to known compounds in the prior art), for instance, the new effect may be a particular mode of action or another effect resultant of the targeted therapy. Targeted therapies may be beneficial as they may have the desired effect (e.g. reduce cancer, by reducing tumor growth or carcinogenisis) but may also have the advantage of reducing side effects (e.g. by preventing the killing of normal cells, as may occur using e.g. chemotherapy).

Furthermore, compounds of the invention may selectively target particular protein or lipid kinases (e.g. the ones described herein) and, additionally, HDAC, for instance compared to other known protein or lipid kinases and HDAC inhibitors (as may be shown experimentally). Accordingly, compounds of the invention may have the advantage that certain, specific, cancers may be treated selectively, which selective treatment may also have the effect of reducing side effects.

### Examples/Biological Tests

Determination of the activity of PI3 kinase activity of compounds of the invention is possible by a number of direct and indirect detection methods. Certain exemplary compounds described herein were prepared, characterized, and tested for their PI3K binding activity and *in vitro* activity against tumor cells. The range of PI3K binding activities was less than 1 nM to about 10 µM (i.e. certain compounds of the examples/invention had PI3K binding activity IC₅₀ values of less than 10 nM). Compounds of the examples/invention had tumor cell-based activity IC₅₀ values less than 100 nM (see Tables below).

### PI3K activity assay

The kinase activity was measured by using the commercial ADP Hunter™ Plus assay available from DiscoveRₓ (#33-016), which is a homogeneous assay to measure the accumulation of ADP, a universal product of kinase activity. The enzyme, PI3K (p110α/p85α was purchased from Carna Biosciences (#07CBS-0402A). The assay was done following the manufacturer recommendations with slight modifications: Mainly the kinase buffer was replace by 50 mM HEPES, pH 7.5, 3 mM MgCl₂, 100 mM NaCl, 1 mM EGTA, 0.04% CHAPS, 2 mM TCEP and 0.01 mg/ml BGG. The PI3K was assayed in a titration experiment to determine the optimal protein concentration for the inhibition assay. To calculate the IC₅₀ of the ETP-compounds, serial 1:5 dilutions of the compounds were added to the enzyme at a fixed concentration (2.5 µg/ml. The enzyme was preincubated with the inhibitor and 30 µM PIP₂ substrate (P9763, Sigma) for 5 min and then ATP was added to a final 50 µM concentration. Reaction was carried out for 1 hour at 25°C. Reagent A and B were sequentially added to the wells and plates were incubated for 30 min at 37 °C. Fluorescence counts were read in a Victor instrument (Perkin Elmer) with the recommended settings (544 and 580 nm as excitation and emission wavelengths, respectively). Values were normalized against the control activity included for each enzyme (i.e., 100 % PI3 kinase activity, without compound). These values were plot against the inhibitor concentration and were fit to a sigmoid dose-response curve by using the Graphad software.

### Cellular Mode of Action

**Cell culture:** The cell lines were obtained from the American Type Culture Collection (ATCC). U2OS (human osteosarcoma) was cultured in Dulbecco's modified Eagle's medium (DMEM). PC3 (human prostate carcinoma), MCF7 (human breast cardinoma), HCT116 (human colon carcinoma), 768-0 (human neuroblastoma), U251 (human glyoblastoma) were grown in RPMI. All media were supplemented with 10% fetal bovine serum (FBS) (Sigma) and antibiotics-antimycotics. Cell were maintained in a humidified incubator at 37°C with 5% CO₂ and passaged when confluent using trypsin/EDTA.

**U2foxRELOC and U2nesRELOC assay:** The U2nesRELOC assay and the U2foxRELOC assay have been described previously (1, 2). Briefly, cells were seeded at a density of 1.0×10⁵ cells/ml into black-wall clear-bottom 96-well microplates (BD Biosciences) After incubation at 37°C with 5% CO₂ for 12 hours, 2µl of each test compound were transferred from the mother plates to the assay plates. Cells were incubated in the presence of the compounds for one hour. Then cells were fixed and the nucleus stained with DAPI (Invitrogen). Finally the plates were washed with 1X PBS twice and stored at 4°C before analysis. Compounds of the invention have a range of *in vitro* cell potency activities from about 1 nM to about 10 µM.

**Image acquirement and processing:** Assay plates were read on the BD Pathway™ 855 Bioimager equipped with a 488/10 nm EGFP excitation filter, a 380/10 nm DAPI excitation filter, a 515LP nm EGFP emission filter and a 435LP nm DAPI emission filter. Images were acquired in the DAPI and GFP channels of each well using 10x dry objective. The plates were exposed 0.066 ms (Gain 31) to acquire DAPI images and 0.55 ms (Gain 30) for GFP images.

**Data analysis:** The BD Pathway Bioimager outputs its data in standard text files. Data were imported into the data analysis software BD Image Data Explorer. The nuclear/cytoplasmic (Nuc/Cyt) ratios of fluorescence intensity were determined by dividing the fluorescence intensity of the nucleus by the cytoplasmic. A threshold ratio of greater than 1.8 was employed to define nuclear accumulation of fluorescent signal for each cell. Based on this procedure we calculated the percentage of cells per well displaying nuclear translocation or inhibition of nuclear export. Compounds that induced a nuclear accumulation of the fluorescent signal greater than 60% of that obtained from wells treated with 4nM LMB were considered as hits. In order to estimate the quality of the HCS assay, the Z' factor was calculated by the equation: Z' = 1 - [(3 × std. dev. of positive controls) + (3 × std. dev. of negative controls) / (mean of positive controls) - (mean of negative controls)].

### PI3K signalling

**AKT phosphorylation Inhibition.Western Blot Analysis:** Subconfluent cells were incubated under different conditions and washed twice with TBS prior to lysis. Lysis buffer was added containing 50 mM Tris HCl, 150 mM NaCl, 1% NP-40, 2mM Na₃VO₄, 100 mM NaF, 20 mM Na₄P₂O₇ and protease inhibitor cocktail (Roche Molecular Biochemicals). The proteins were resolved on 10% SDS-PAGE and transferred to nitrocellulose membrane (Schleicher & Schuell, Dassel, Germany). The membranes were incubated overnight at 4°C with antibodies specific for Akt, phospho-Ser-473-Akt (Cell Signaling Technology) and α-tubulin (Sigma), they were washed and then incubated with IRDye800 conjugated antimouse and Alexa Fluor 680 goat anti-rabbit IgG secondary antibodies. The bands were visualized using an Odyssey infrared imaging system (Li-Cor Biosciences). Compounds of the invention have a range of *in vitro* cell potency activities from about 1 nM to about 10 µM.

### In Vitro Cell Proliferation Assays

The *in vitro* potency of the compounds was measured by the cell proliferation assay of the Example; the CellTiter-Glo® Luminescent Cell Viability Assay, commercially available from Promega Corp., Madison, WI. This homogeneous assay method is based on the recombinant expression of *Coleoptera* luciferase (US 5583024; US 5674713; US 5700670) and determines the number of viable cells in culture based on quantitation of the ATP present, an indicator of metabolically active cells (Crouch et al (1993) J. Immunol. Meth. 160:81-88; US 6602677). The CellTiterGlo® Assay was conducted in 96 making it amenable to automated highthroughput screening (HTS).

### mTOR assay

Mammalian target of rapamycin (mTOR) was assayed by monitoring phosphorylation of GFP-4EBP using a homogeneous time-resolved fluorescence resonante energy transfer format and assay reagents from Invitrogen. In the presence of 10 µM ATP, 50 mM Hepes (pH 7.5), 0.01 % (v/v) Polysorbate 20, 10 mM MnCl₂, 1mM EGTA, and 2.5 mM DTT, the mTOR-mediated phosphorylation of 200 nM GFP-4E-BP1 was measured under initial rate conditions. After incubation at room temperature for 60 min, the reaction was terminated by addition of 10 mM EDTA, and phosphorylated GFP-4E-BP1 was detected with 2 nM Tb-anti-p4E-BP1 antibody before reading on a Perkin-Elmer Wallac 1420 Fluorescence Reader (exc 340; em 490/520).

### PI3K cellular activity (Elisa assay)

Activity is measured as endogenous levels of phospho-Akt1 (Ser473) protein. Osteosarcoma U2OS cells are plated in 96 Poly-D-Lysine coating tissue culture plates (18.000 cells/well). After the treatment with serial dilutions of the compound during 3h, the cells are fixed directly in the wells with 4% paraformaldehyde.

After fixing, individual wells go through the same series of steps used for a conventional immunoblot: including blocking with 5% BSA, incubation with 1/1000 of primary antibody-AKT (Ser 74) in PBS containing 5% BSA at 4°C overnight (Cell Signalling), washing and incubation with second antibody HRP-anti-mouse IgG for 1 h at RT (Amersham). After the addition of SuperSignal ELISA Femto maximum sensitivity chemiluminescent substrate (Pierce) the results are read using a luminescence plate reader (Victor).

### Cell viability assays and combination assays

Cells were seeded at 10000-50000 cells/well in 96 plates for 16 h. On day two, nine serial 1:3 compound dilutions were made in DMSO in a 96 well plate. The compounds were added to duplicate wells in 96-well cell plates a using a FX BECKMAN robot (Beckman Coulter) and incubated at 37°C with CO₂ atmosphere. After 3 days, relative numbers of viable cells were measured by MTT (Sigma) according to manufacturer's instruction and read on EndVision (Perkin Elmer). EC₅₀ values were calculated using ActivityBase from IDBS. Drugs in combination assays were dosed starting at 4 x EC₅₀ concentrations and continuing with serial dilutions 1:2. PI3K inhibitors and chemotherapeutic agents were added simultaneously.

An additional exemplary *in vitro* cell proliferation assay includes the following steps:
1. An aliquot of 200 µl of cell culture containing optimal density (between 10⁴ - 5x10⁴ cells) (see Examples for cell lines and tumour type) in medium was deposited in each well of a 96-well flat bottom plates.
2. Control wells were prepared containing medium without cells
3. The compound was added to the experimental wells and incubated for 3 days.
4. One quarter volume of MTT reagent with respect to the volume of cell culture medium present in each well was added and incubated 24h at 37°C with 5% CO₂.
5. One quarter volume of solubilisation buffer with respect to the volume of cell culture medium present in each well was added and incubated 24h at 37°C with 5% CO₂.
6. Formazan salt formed was recorded and reported in graphs as relative growth vs. cells treated only with dmso.

The individual measured EC₅₀ values against the particular cell of the exemplary compounds and of the chemotherapeutic agent are compared to the combination EC₅₀ value. The combination Index (CI) score is calculated by the Chou and Talalay method (CalcuSyn software, Biosoft). A CI less 0.8 indicates synergy. A CI between 0.8 and 1.2 indicates additivity. A CI greater than 1.2 indicates antagonism.

### HDAC enzymatic activity

HDAC inhibitory activity was evaluated using the HDAC-Glo^{™} I/II Screening System from Promega (G6430) using manufacturing recommendations. The system emploies HeLa cell nuclear extracts as a source of HDAC activities. The assay uses an acetylated, live-cell-permeant, luminogenic peptide substrate that can be deacetylated by HDAC activities. Substrate deacetylation is measured by a coupled protease reaction that cleaves the peptide from aminoluciferin, which is quantified in a reaction using recombinant Ultra-Glo^{™} firefly luciferase. Test compounds were serially diluted in dimethylsulphoside (DMSO) and added to HeLa cell nuclear extract in the presence of the acetylated luminogenic peptide substrate and developer reagent. Reactions were incubated at 25°C for 30 minutes and luminescence was measured in the EnVision plate reader (Perkin Elmer). Data were analyzed using GraphPad Prism (v5.03) with a sigmoidal dose-response curve fitting for IC₅₀ calculation.

Where compound names are given herein, they are typically generated with ChemDraw.

### Examples and Experimental

### Experimental part:

The compound names given herein were generated with MDL ISIS/DRAW 2.5 SP 2, Autonom 2000.

Hereinafter, the term "DCM" means dichloromethane, "DCE" means dichloroethane "MeOH" means methanol, "THF" means tetrahydrofuran, "DMF" means dimethylformamide, "DME" means 1,2-dimethoxyethane, "EtOAc" means ethyl acetate, "Pd(PPh₃)₄" means tetrakis(triphenylphosphine)palladium, "DIPEA" means diisopropylethylamine, "TEA" means triethylamine, "BINAP" means (R)/(+_)-2,2'-bis(diphenylphosphino)-1,1'-binaphtyl, "Pd₂(dba)₃" means tris(dibenzylideneacetone)dipalladium(0), "eq" means equivalents, "EtOH" means Ethanol, "nBuOH " means n-butanol, "tBuOH" means tert-butanol, "DIAD" means diethylazodicarboxilate, "DavePhos" means 2-dicyclohexylphosphino-2'-(n,n-dimethylamino)biphenyl, "HATU" means O-(7-azabenzotriazole-1-yl)-1,1,3,3-tetramethyluronium hexafluorophosphate, "Pd(dppf)Cl₂.DCM" means 1,1'-bis(diphenylphosphino)ferrocenepalladium(II) dichloride, dichloromethane, "NCS" means N-chlorosuccinimide, "NBS" means N-bromosuccinimide "mw" means microwave, "RT" means room temperature, "CCTLC" means centrifugal circular thin-layer chromatography, "min" means minutes, "h" means hours.

### General Procedure

NMR spectra were recorded in a Bruker Avance II 300 spectrometer and Bruker Avance II 700 spectrometer fitted with 5mm QXI 700 S4 inverse phase, Z-gradient unit and variable temperature controller.

The HPLC measurements were performed using a HP 1100 from Agilent Technologies comprising a pump (binary) with degasser, an autosampler, a column oven, a diode-array detector (DAD) and a column as specified in the respective methods below. Flow from the column was split to a MS spectrometer. The MS detector was configured with an electrospray ionization source or API/APCI. Nitrogen was used as the nebulizer gas. Data acquisition was performed with ChemStation LC/MSD quad, software.

### Method 1

Reversed phase HPLC was carried out on a Gemini-NX C18 (100 x 2.0 mm; 5um).

Solvent A: water with 0.1% formic acid; Solvent B: acetonitrile with 0.1% formic acid. Gradient: 5% to 100% of B within 8 min at 50 °C, DAD.

### Method 2

Reversed phase HPLC was carried out on a Gemini-NX C18 (100 x 2.0 mm; 5um).

Solvent A: water with 0.1% formic acid; Solvent B: acetonitrile with 0.1% formic acid. Gradient: 5% to 40% of B within 8 min at 50 °C, DAD.

### Method 3

Reversed phase HPLC was carried out on a Gemini-NX C18 (100 x 2.0 mm; 5um).

Solvent A: water with 0.1 % formic acid; Solvent B: acetonitrile with 0.1 % formic acid. Gradient: 0% to 30% of B within 8 min at 50 °C, DAD.

### Method 4

Reversed phase HPLC was carried out on a Gemini C18 column (50 x 2 mm, 3 um).

Solvent A: water with 0.1 % formic acid; Solvent B: acetonitrile with 0.1 % formic acid. Gradient: 10% to 95% of B within 4 min at 50°C, DAD.

### Method 5

Reversed phase HPLC was carried out on a Gemini C18 column (50 x 2 mm, 3 um). Solvent A: water with 0.1% formic acid; Solvent B: acetonitrile with 0.1% formic acid. Gradient: 0% to 30% of B within 4 min at 50°C, DAD.

"Found mass" refers to the most abundant isotope detected in the HPLC-MS.

### Final Compounds:

| | | **NMR data** | **LC/MS** | **Synthetic Method** | **PI3Kα IC50 (nM)** | **HDAC IC50 (nM)** |
|---|---|---|---|---|---|---|
| **1** | 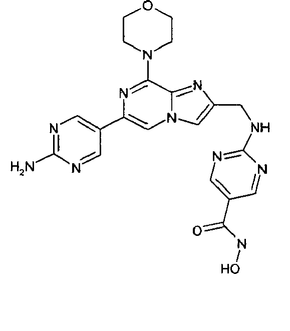 | ¹H-NMR (300 MHz, DMSO) δ 8.74 (s, 2H), 8.56 (s, 2H), 8.40 (s, 1H), 7.85 (t, J = 5.86 Hz, 1H), 7.64 (s, 1H), 6.83 (s, 2H), 4.60 (d, J = 5.8 Hz, 2H), 4.22 (m, 4H), 3.76 (m, 4H). | Method 1: Rt = 2.98 min, [M+H]⁺= m/z 464.1. | **3** | 5.7 | 21.1 |
| **2** | 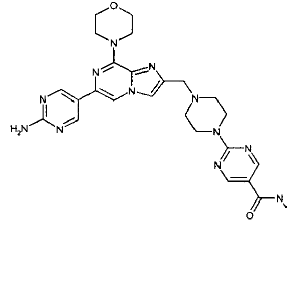 | ¹H NMR (300 MHz, DMSO) δ 8.77 (s, 2H), 8.55 (s, 2H), 8.43 (s, 1H), 7.79 (s, 1H), 6.84 (s, 2H), 4.23 (m, 4H), 3.76 (m, 8H), 3.64 (s, 2H), 2.50 (m, 4H). | Method 3: Rt = 4.32 min, [M+H]⁺ = m/z 533.1. | **3** | 3.2 | 9.0 |
| **3** | 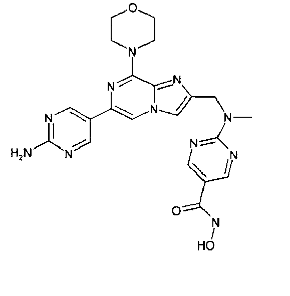 | ¹H NMR (300 MHz, DMSO) δ 8.72 (s, 2H), 8.57 (s, 2H), 8.36 (s, 1H), 7.57 (s, 1H), 6.82 (s, 2H), 4.91 (s, 2H), 4.22 (m, 4H), 3.75 (m, 4H), 3.17 (s, 3H). | Method 1: Rt = 3.37 min, [M+H]⁺ = m/z 478.3. | **3** | 7.5 | 10.6 |

### Method A: Alkylation reaction

### Example 1

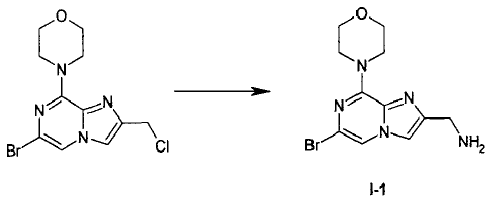

To a solution of Imidazo[1,2-a]pyrazine, 6-bromo-2-(chloromethyl)-8-(4-morpholinyl)-(CAS: 1252597-75-3), (1 mmol) in MeOH (1.5 mL) was added 32% aqueous ammonia (3.5 mL). The suspension obtained was heated at 145 °C for 1 h in the microwave. The reaction was cooled to r.t. and a precipitate appeared. The solid was filtered, washed with H₂O and dried under vacuum to afford 246 mg as a yellow-orange solid, as the expected compound.

### Example 2

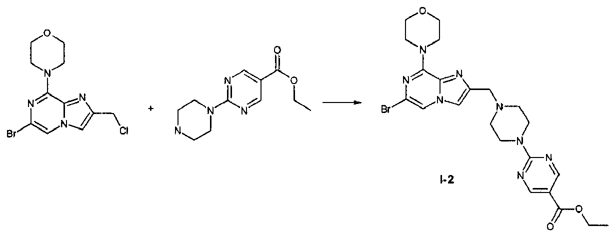

A mixture of Imidazo[1,2-a]pyrazine, 6-bromo-2-(chloromethyl)-8-(4-morpholinyl)-(CAS: 1252597-75-3), (0.45mmol), Ethyl 2-(piperazin-1-yl)pyrimidine-5-carboxylate (CAS:603965-77-1) (0.47 mmol) , K₂CO₃ (0.68 mmol) in AcCN was heated at 100°C in a seal tube overnight. The mixture was evaporated and the residue was suspended in DCM/H2O. The organic phase was dried (Na2SO4), filtered and evaporated. The residue was purified by automated chromatography in DCM/MeOH 100 to 95:5 to obtain 169.4 mg, beige solid, of the desired compound.

### Example 3

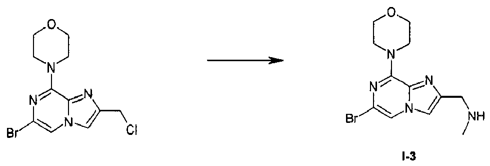

A mixture of Imidazo[1,2-a]pyrazine, 6-bromo-2-(chloromethyl)-8-(4-morpholinyl)-(CAS: 1252597-75-3), (0.75 mmol) and methylamine (25 mL; solution 2M in MeOH) was stirred at reflux for 2h15min. The solvent was removed at reduced pressure and the residue was purified by chromatography in DCM/MeOH 100 to 95:5 to obtain 256 mg of a brown solid, as the desired compound.

### Method B: Amine substitution Reaction

### Example 4

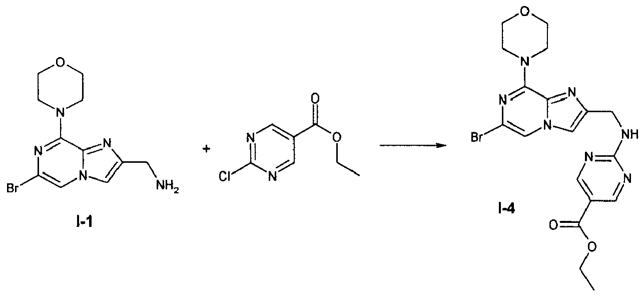

A mixture of compound I-1 (0.90 mmol), ethyl 2-chloropyrimidine-5-carboxylate (2.24 mmol) and DIPEA (0.78 mL) in AcN (9 mL) and 4.5 mL of DMF was stirred at r.t. overnight. Water was added. The resulting precipite was filtered off, washed with water and Et₂O and dried in vacuo to obtain 272.4 mg as a beige solid of desired product I-4 that was used in next reaction step without further purification.

### Example 5

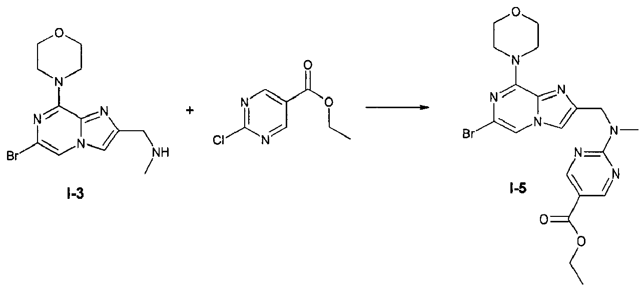

A mixture of compound I-3 (0.79 mmol), ethyl 2-chloropyrimidine-5-carboxylate (1.98 mmol) and DIPEA (3.96 mmol) in AcN (6 mL) and 3 mL of DMF was stirred at r.t. overnight. The solvent was removed at reduced pressure and was purified in DCM/EtOAc 100 to 50:50 to obtain 288 mg of a white solid, pure expected compound.

### Method C: Coupling Reaction

### Example 6

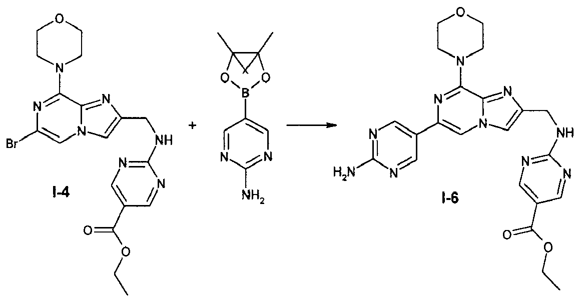

A mixture of I-4 (0.42 mmol), 2-aminopyrimidine-5-boronic acid (pinacol ester; 0.64 mmol), NaHCO₃ (1.27 mmol) and PdCl₂(PPh₃)₂ (0.02 mmol) in toluene (2.7 mL), EtOH (1.5 mL) and water (0.7 mL) was flushed with Argon and heated under MW irradiation: 120 °C, 1h. Reaction mixture was concentrated. The crude product was purified by flash chromatography in DCM/MeOH 100 to 50:50 to obtain 62 mg of a light yellow solid as the expected pure compound and a second fraction, 107 mg, brown solid, as impure expected compound.

### Example 7

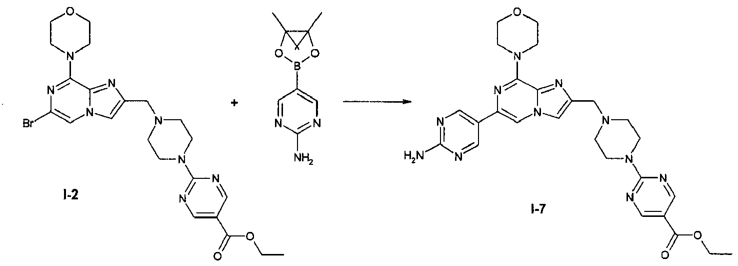

To a mixture of I-2 (0.22 mmol) in DME (1 mL), 2-aminopyrimidine-5-boronic acid (0.25 mmol), pinacol ester, PdCl₂(dppf) (0.02 mmol) and K₂CO₃ solution (sat aq sol; 0.13 mL) were added and the mixture was heated at 120°C for 45min. in MW; and then at 120°C for 15 min; Excess of reagents were added till completion of reaction after 15 min more at 120°C. The mixture was evaporated and the residue was purified by automated chromatography in DCM/MeOH 100 to 70:30 to obtain 41 mg pure of the desired compounds and 70 mg still impure, to repurify again.

### Example 8

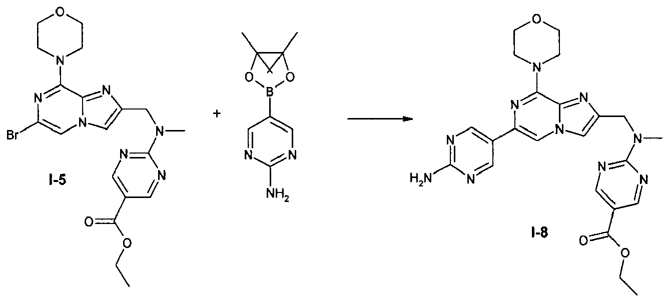

Intermediate I-5 (0.25 mmol), 2-aminopyrimidine-5-boronic acid (pinacol ester; 0.38 mmol), NaHCO₃ (0.76 mmol) and PdCl₂(PPh₃)₂ (0.01 mmol) in toluene (1.6 mL), EtOH (0.9 mL) and water (0.4 mL) was flushed with Argon and heated under MW irradiation: 120°C, 1 h. To the reaction mixture was added water and extracted with AcOEt (x3).The combined organic layers were washed with NaCl (x1), dried over Na₂SO₄ and evaporated to obtain a brown solid, that was purified by automated chromatography in DCM/MeOH 100 to 50:50 to obtain 232 mg of a light yellow solid which was triturated with DCM/MeOH to obtain expected compound pure, 91 mg, as a white solid.

### Method C: Hydroxamic acid formation

### Final Product 1

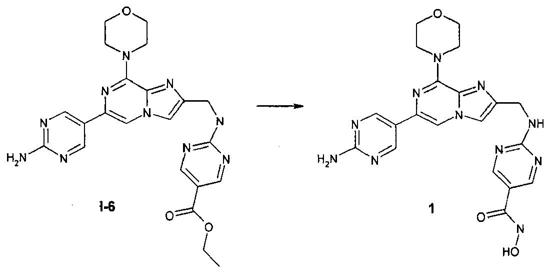

### Preparation of freshly hydroxylamine solution:

To a stirred solution of NH₂OH.HCl (18 mmol) in MeOH (6 mL) at 0 °C was added a solution of KOH (27 mmol, 1.5 eq) in MeOH (4 mL).After addition, the mixture was stirred for 30 min at 0 °C. The resulting precipitate was filtered off and the filtrate was prepared as free hydroxylamine solution: 1.80 M in MeOH.

The freshly prepared NH₂OH solution (10 mL) was placed in a flask. Compound I-6 (0.23 mmol) was added to this solution and stirred at 0-10 °C for 30 min. The mixture was heated at reflux for 4h. The reaction was cooled and filtered; the collected solid was washed with H₂O, MeOH and Et₂O, dried under vacuum to give 32.7 mg as a white solid.

### Final Product 2

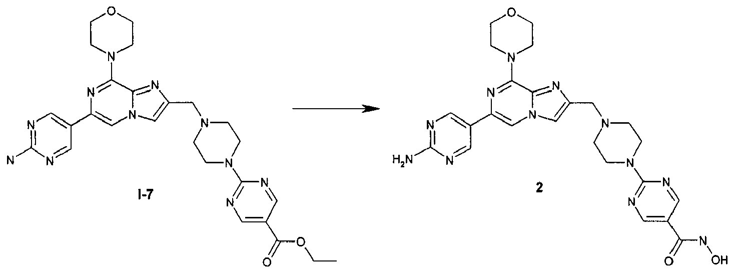

The freshly prepared NH₂OH solution (10 mL) was placed in a flask. Compound I-7 (0.24 mmol) was added to this solution and stirred at 0-10 °C for 30 min. The reaction mixture was stirred at r.t. for 2h and then the mixture was heated at reflux for 3h,till completion of the reaction. The reaction was cooled and filtered; the collected solid was washed with H₂O, MeOH and Et₂O, dried under vacuum to give 125.8 mg as a light pink solid, which was triturated with a mixture MeOH:CH₂Cl₂ and filtered; the collected solid was washed with Et₂O, dried under vacuum to give 118.2 mg as a beige solid, desired pure compound.

### Final Product 3

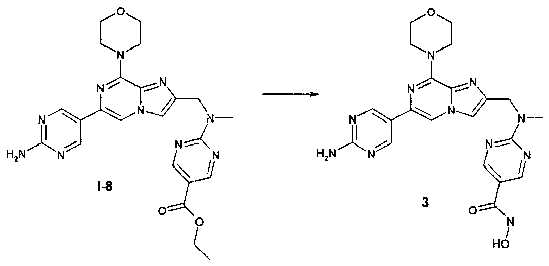

The freshly prepared NH₂OH solution (12 mL) was placed in a flask. Compound I-8 (0.28 mmol) was added to this solution and stirred at 0-10 °C for 30 min and then heated at reflux for 16h, The reaction was cooled and filtered; the collected solid was washed with H₂O, MeOH and Et₂O, dried under vacuum to give 77.1 mg as a beige solid.

### Further Biological Example

Final compounds of the examples were tested using the biological tests described herein and the following results were obtained (Elisa Assay and In Vitro Cell Proliferaiton Assay; cancer cell lines are available from known sources).

**Table 1 - Inhibition of phosphorylation of AKT in different cell lines by Elisa assay**

| **Final Compound** | **AKT_P_U20S_ELISA_EC50** | **AKT_P_SKMEL_ELISA_EC50** |
|---|---|---|
| **3** | 2.60E-08 | 1.94E-06 |
| **1** | | 3.30E-07 |
| **2** | 1.36E-06 | 7.00E-08 |

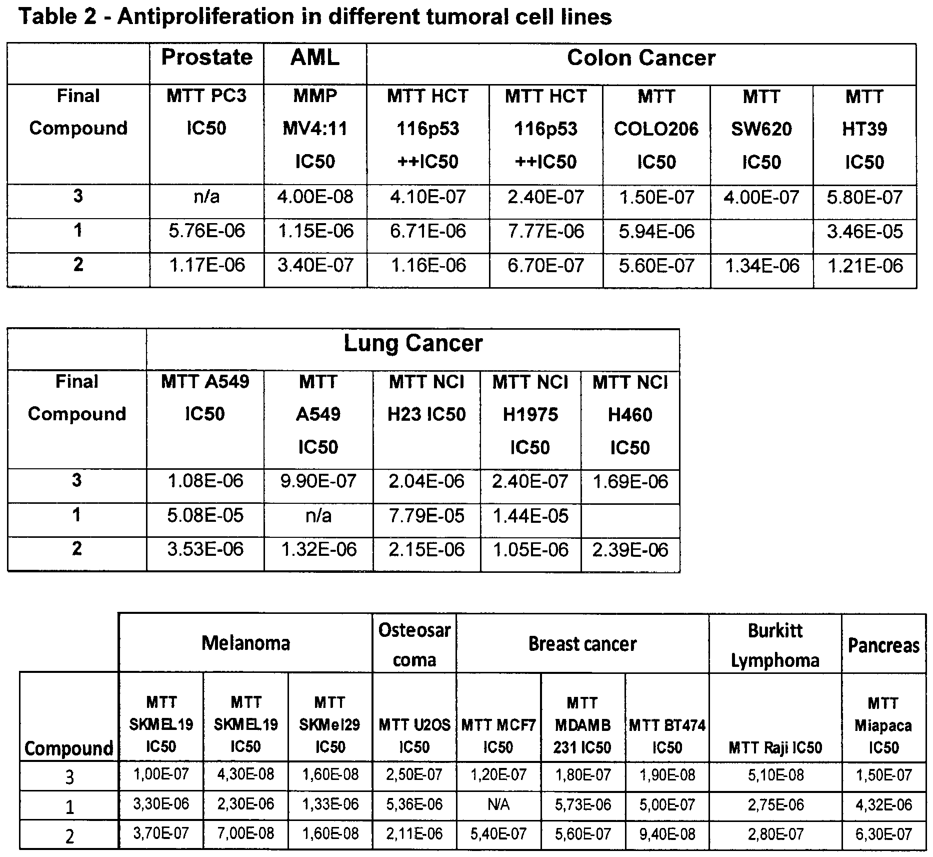

## Claims

1. A compound of formula I, 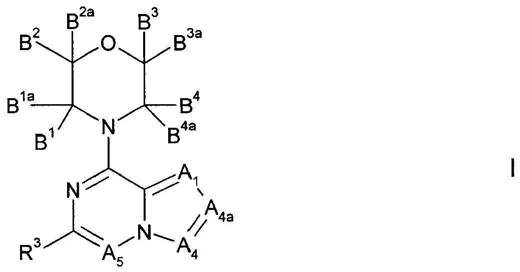 wherein:
A₁ represents N or C(H);
A₄ represents N or C(R^{1a});
A₄ₐ represents N or C(R^{1b});
wherein at least one of A₄ and A₄ₐ does not represent N;
A₅ represents N or C(R²);
each B¹, B^{1a}, B², B^{2a}, B³, B^{3a}, B⁴ and B^{4a} independently represent hydrogen or a substituent selected from halo, -C(=Y)-R^{10a}, -C(=Y)-OR^{10a}, -C(=Y)N(R^{10a})R^{11a}, -S(O)₂N(R^{10a})R^{11a}, C₁₋₁₂ alkyl, heterocycloalkyl (which latter two groups are optionally substituted by one or more substituents selected from =O and E¹), aryl and/or heteroaryl (which latter two groups are optionally substituted by one or more substituents selected from E²); or
any two B¹, B^{1a}, B², B^{2a}, B³, B^{3a}, B⁴ and B^{4a} substituents that are attached to the same carbon atom (i.e. B¹ and B^{1a}; B² and B^{2a}; B³ and B^{3a}; and/or B⁴ and B^{4a}) may together form a =O group;
or, any two B¹, B^{1a}, B², B^{2a}, B³, B^{3a}, B⁴ and B^{4a} substituents may be linked together to form a further 3- to 12- membered ring, optionally containing (in addition to the atom(s) of the morpholine ring) one or more heteroatom(s), which ring optionally contains one or more double bonds, and which ring is itself optionally substituted by one or more substituents selected from halo, =O and C₁₋₃ alkyl optionally substituted by one or more fluoro atoms;
R² (when present) independently represents hydrogen or a substituent selected from halo, -CN, -OR^{10b}, -N(R^{10b})R^{11b}, -C(O)N(R^{10b})R^{11b}, C₁₋₁₂ alkyl and heterocycloalkyl (e.g. a 3- to 7-membered heterocycloalkyl), which latter two groups are optionally substituted by one or more substituents selected from E³ and =O;
**characterised in that**:
one of R^{1a} and R^{1b} is present and represents -T¹-Z¹ and the other (if present) represents R^{1c};
T¹ represents a linker group selected from -T^{1a}-X^{1a}-X^{1b}-T^{1b}-;
Z¹ represents a group selected from:
(i) -C(=Y)N(R^{10a})-OR^{11c};
(ii) 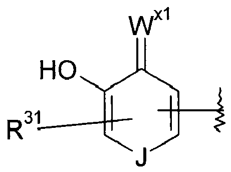 W^{x1} is O or S; J is O, NH or NCH₃; and R³¹ is hydrogen or C₁₋₄alkyl;
(iii) 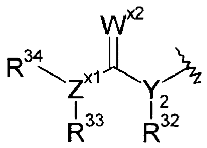 W^{x2} is O or S; Y₂ is absent, N or CH; Z^{x1} is N or CH; R³² and R³⁴ are independently hydrogen, hydroxy, an aliphatic group (i.e. a non-aromatic moiety that may be saturated or contain one or more units of unsaturation), provided that
if both R³² and R³⁴ are present, one of R³² and R³⁴ must be hydroxy and if Y₂ is absent, R³⁴ must be hydroxy; and R³³ is hydrogen or aliphatic group;
(iv) 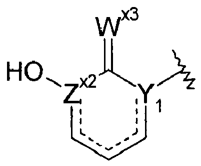 W^{x3} is O or S; Y₁ and Z^{x2} are independently N, C or CH; and
(v) 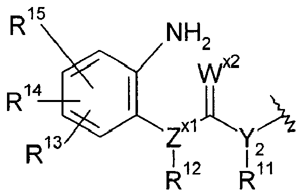
Z^{x1} Y₂ and W^{x2} are as defined above, R¹¹ and R¹² are independently selected from hydrogen and an aliphatic group; R¹³, R¹⁴ and R¹⁵ are independently selected from hydrogen, hydroxy, amino, halo, alkoxy, alkylamino, dialkylamino, CF₃, CN, NO₂, sulfonyl, acyl, an optionally substituted aliphatic group, optionally substituted aryl, optionally substituted heteroaryl and optionally substituted heterocycloalkyl;
T^{1a} and T^{1b} independently represent a direct bond or C₁₋₁₂ alkylene optionally substituted by one or more substituents selected from Q^{1a};
X^{1a} represents a direct bond, -N(R^{t1})-, -C(O)N(R^{t2})-, -N(R^{t3})-C(O)-, -O-, -S-, -S(O)-, -S(O)₂-, arylene (optionally substituted by one or more substituents selected from Q^{1b}), heteroarylene (optionally substituted by one or more substituents selected from Q^{1c}) or cycloalkylene or heterocycloalkylene (which latter two optionally comprise a further ring as defined by Z^{a}, and which ring(s) (i.e. cycloalkyl/heterocycloalkyl and optional further ring) is/are optionally substituted by one or more substituents selected from =O and Q^{1d});
t1, t2 and t3 independently represent hydrogen or C₁₋₆ alkyl optionally substituted by one or more fluoro atoms;
when X^{1a} represents -N(R^{t1})-, -C(O)N(R^{t2})-, -N(R^{t3})-C(O)-, -O-, -S-, -S(O)- or -S(O)₂-, then:
X^{1b} represents a direct bond, arylene (optionally substituted by one or more substituents selected from Q^{1e}), heteroarylene (optionally substituted by one or more substituents selected from Q^{1f}) or cycloalkylene or heterocycloalkylene (which latter two optionally comprise a further ring as defined by Z^{b}, and which ring(s) (i.e. cycloalkyl/heterocycloalkyl and optional further ring) is/are optionally substituted by one or more substituents selected from =O and Q^{1g});
when X^{1a} represents a direct bond, arylene (optionally substituted by one or more substituents selected from Q^{1b}), heteroarylene (optionally substituted by one or more substituents selected from Q^{1c}) or cycloalkylene or heterocycloalkylene (which latter two optionally comprise a further Z^{a} ring; and which are optionally substituted by one or more substituents selected from =O and Q^{1d}), then:
X^{1b} may represent a direct bond, -N(R^{t1})-, -C(O)N(R^{t2})-, -N(R^{t3})-C(O)-, -O-, -S-, -S(O)-, -S(O)₂-, arylene (optionally substituted by one or more substituents selected from Q^{1e}), heteroarylene (optionally substituted by one or more substituents selected from Q^{1f}) or cycloalkylene or heterocycloalkylene (which latter two optionally comprise a further ring as defined by Z^{b}, and which ring(s) (i.e. cycloalkyl/heterocycloalkyl and optional further ring) is/are optionally substituted by one or more substituents selected from =O and Q^{1g});
R^{1c} (when present) represents hydrogen or a substituent selected from halo and C₁₋₃ alkyl (optionally substituted by one or more substituents selected from =O, fluoro and -OCH₃);
Z^{a} and Z^{b} each independently represent a moiety that results in a further ring sytem (that is present in addition to the "first ring" i.e. in addition to the monocyclic cycloalkyl/heterocycloalkyl groups, to which that Z^{a} to Z^{b} group is attached) that is formed by that Z^{a} or Z^{b} group representing:
(a) a 3- to 7-membered saturated heterocycloalkyl group containing one to four heteroatoms selected from oxygen, sulfur and nitrogen, a 3- to 12-membered saturated carbocyclic ring, or an unsaturated 5- to 12-membered carbocyclic or heterocyclic ring that is fused to the first ring;
(b) a linker group -(C(R^{x})₂)ₚ- and/or -(C(R^{x})₂)ᵣ-O-(C(R^{x})₂)₁- (wherein p is 1 or 2; r is 0 or 1; s is 0 or 1; and each R^{x} independently represents hydrogen or C₁₋₆ alkyl), linking together any two non-adjacent atoms of the first ring (i.e. forming a bridged structure); or
(c) a second ring that is either a 3- to 12-membered saturated carbocyclic ring or a 3- to 7-membered saturated heterocycloalkyl group containing one to four heteroatoms selected from oxygen and nitrogen, and which second ring is linked together with the first ring *via* a single carbon atom common to both rings (i.e. forming a spiro-cycle);
R³ represents aryl or heteroaryl (both of which are optionally substituted by one or more substituents selected from E⁴);
each Q^{1a}, Q^{1b}, Q^{1c}, Q^{1d}, Q^{1e}, Q^{1f} and Q^{1g} independently represents, on each occasion when used herein:
halo, -CN, -NO₂, -N(R^{10a})R^{11a}, -OR^{10a}, -C(=Y)-R^{10a}, -C(=Y)-OR^{10a}, -C(=Y)N(R^{10a})R^{11a}, -C(=Y)N(R^{10a})-OR^{11c}, -OC(=Y)-R^{10a}, -OC(=Y)-OR^{10a}, -OC(=Y)N(R^{10a})R^{11a}, -OS(O)₂OR^{10a}, -OP(=Y)(OR^{10a})(OR^{11a}), -OP(OR^{10a})(OR^{11a}), -N(R^{12a})C(=Y)R^{11a}, -N(R^{12a})C(=Y)OR^{11a}, -N(R^{12a})C(=Y)N(R^{10a})R^{11a}, -NR^{12a}S(O)₂R^{10a}, -NR^{12a}S(O)₂N(R^{10a})R^{11a}, -S(O)₂N(R^{10a})R^{11a}, -SC(=Y)R^{10a}, -S(O)₂R^{10a}, -SR^{10a}, -S(O)R^{10a}, C₁₋₁₂ alkyl, heterocycloalkyl (which latter two groups are optionally substituted by one or more substituents selected from =O, =S, =N(R^{10a}) and E⁵), aryl or heteroaryl (which latter two groups are optionally substituted by one or more substituents selected from E⁶);
each R^{11c} independently represents C₁₋₁₂ alkyl, heterocycloalkyl (which latter two groups are optionally substituted by one or more substituents selected from =O, =S, =N(R²⁰) and E⁷), aryl or heteroaryl (which latter two groups are optionally substituted by one or more substituents selected from E⁸);
each R^{10a}, R^{11a} R^{10b}, R^{11b} and R^{12a} independently represent, on each occasion when used herein, hydrogen, C₁₋₁₂ alkyl, heterocycloalkyl (which latter two groups are optionally substituted by one or more substituents selected from =O, =S, =N(R²⁰) and E⁷), aryl or heteroaryl (which latter two groups are optionally substituted by one or more substituents selected from E⁸); or
any relevant pair of R^{10a} and R^{11a} or R^{10b} and R^{11b} may be linked together to form a 4- to 20-membered ring, optionally containing one or more heteroatoms, optionally containing one or more unsaturations, and which ring is optionally substituted by one or more substituents selected from =O, =S, =N(R²⁰) and E⁹;
each E¹, E², E³, E⁴, E⁵, E⁶, E⁷, E⁸ and E⁹ independently represents, on each occasion when used herein:
(i) Q⁴;
(ii) C₁₋₁₂ alkyl optionally substituted by one or more substituents selected from =O and Q⁵; or
any two E¹, E², E³, E⁴, E⁵, E⁶, E⁷, E⁸ or E⁹ groups may be linked together to form a 3- to 12-membered ring, optionally containing one or more unsaturations, and which ring is optionally substituted by one or more substituents selected from =O and J¹;
each Q⁴ and Q⁵ independently represent, on each occasion when used herein: halo, -CN, -NO₂, -N(R²⁰)R²¹, -OR²⁰, -C(=Y)-R²⁰, -C(=Y)-OR²⁰, -C(=Y)N(R²⁰)R²¹, -C(=Y)N(R²⁰)-O-R^{21a}, -OC(=Y)-R²⁰, -OC(=Y)-OR²⁰, -OC(=Y)N(R²⁰)R²¹, -OS(O)₂OR²¹, -OP(=Y)(OR²⁰)(OR²¹), -OP(OR²⁰)(OR²¹), -N(R²²)C(=Y)R²¹, -N(R²²)C(=Y)OR²¹, -N(R²²)C(=Y)N(R²⁰)R²¹, -NR²²S(O)₂R²⁰, -NR²²S(O)₂N(R²⁰)R²¹, -S(O)₂N(R²⁰)R²¹, -SC(=Y)R²⁰, -S(O)₂R²⁰, -SR²⁰, -S(O)R²⁰, C₁₋₆ alkyl, heterocycloalkyl (which latter two groups are optionally substituted by one or more substituents selected from =O and J²), aryl or heteroaryl (which latter two groups are optionally substituted by one or more substituents selected from J³).
each Y independently represents, on each occasion when used herein, =O, =S, =NR²³ or =N-CN;
each R^{21a} represents C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵);
each R²⁰, R²¹, R²² and R²³ independently represent, on each occasion when used herein, hydrogen, C₁₋₆ alkyl, heterocycloalkyl (which latter two groups are optionally substituted by one or more substituents selected from J⁴ and =O), aryl or heteroaryl (which latter two groups are optionally substituted by one or more substituents selected from J⁵); or
any relevant pair of R²⁰, R²¹ and R²² may be linked together to form a 4- to 20-membered ring, optionally containing one or more heteroatoms, optionally containing one or more unsaturations, and which ring is optionally substituted by one or more substituents selected from J⁶ and =O;
each J¹, J², J³, J⁴, J⁵ and J⁶ independently represents, on each occasion when used herein:
(i) Q⁷;
(ii) C₁₋₆ alkyl or heterocycloalkyl, both of which are optionally substituted by one or more substituents selected from =O and Q⁸;
each Q⁷ and Q⁸ independently represents, on each occasion when used herein: halo, -CN, -N(R⁵⁰)R⁵¹, -OR⁵⁰, -C(=Y^{a})-R⁵⁰, -C(=Y^{a})-OR⁵⁰, -C(=Ya)N(R⁵⁰)R⁵¹, -N(R⁵²)C(-Y^{a})R⁵¹, -NR⁵²S(O)₂R⁵⁰, -S(O)₂N(R⁵⁰)R⁵¹, -N(R⁵²)-C(=Y^{a})-N(R⁵⁰)R⁵¹, -S(O)₂R⁵⁰, -SR⁵⁰, -S(O)R⁵⁰, C₁₋₆ alkyl (optionally substituted by one or more fluoro atoms), heterocyclalkyl, aryl or heteroaryl (which latter three groups are optionally substituted by one or more substituents selected from halo, -OR⁶⁰ and -N(R⁶¹)R⁶²);
each Y^{a} independently represents, on each occasion when used herein, =O, =S, =NR⁵³ or =N-CN;
each R⁵⁰, R⁵¹, R⁵² and R⁵³ independently represents, on each occasion when used herein, hydrogen or C₁₋₆ alkyl optionally substituted by one or more substituents selected from fluoro, -OR⁶⁰ and -N(R⁶¹)R⁶²; or
any relevant pair of R⁵⁰, R⁵¹ and R⁵² may be linked together to form, a 3- to 8-membered ring, optionally containing one or more heteroatoms, optionally containing one or more unsaturations, and which ring is optionally substituted by one or more substituents selected from =O and C₁₋₃ alkyl;
R⁶⁰, R⁶¹ and R⁶² independently represent hydrogen or C₁₋₆ alkyl optionally substituted by one or more fluoro atoms,
or a pharmaceutically acceptable ester, amide, solvate or salt thereof.

2. A compound as claimed in Claim 1, wherein the requisite bicyclic core (containing A₁, A₄, A₄ₐ and A₅) represents any one of the following:

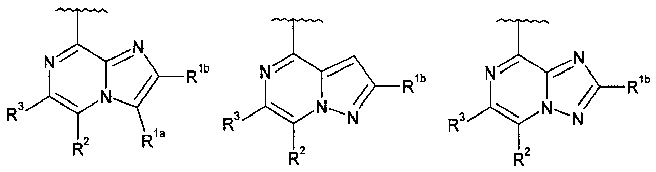

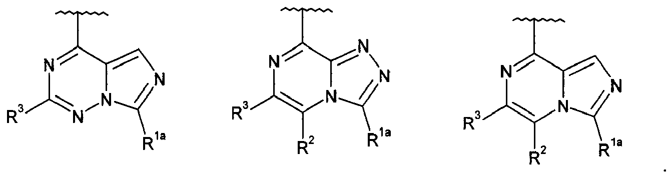

3. A compound as claimed in Claim 1 or Claim 2, wherein the linker groups "*-T^{1a}-X^{1a}-X^{1b}-T^{1b}-*" represent: 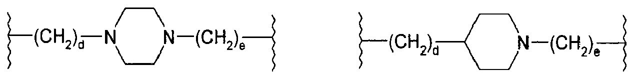 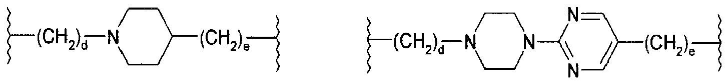 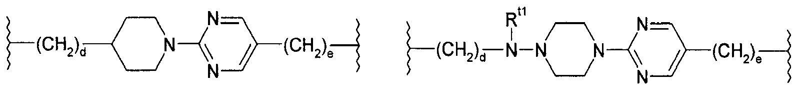 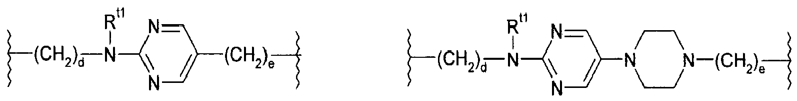 in which preferably:
d represents 1;
e represents 0;
R^{t1} represents hydrogen or C₁₋₂ alkyl (e.g. methyl).

4. A compound as claimed in any one of the preceding claims, wherein:
R³ represents aryl (e.g. phenyl) optionally substituted by one or more (e.g. one) substituents selected from E⁴, or, R³ may represent heteroaryl (e.g. a monocyclic 5- or preferably 6-membered heteroaryl group or a bicyclic 10- or, preferably, 9-membered heteroaryl group, in which there is one or two (e.g. two) heteroatoms present selected from sulfur, oxygen or, preferably, nitrogen, so forming for example a pyridyl, pyrimidinyl or indazolyl group), which is optionally substituted by one or more (e.g. one) substituent selected from E⁴.

5. A compound as claimed in any one of the preceding claims, wherein:
the requisite bicycle of formula I represents: 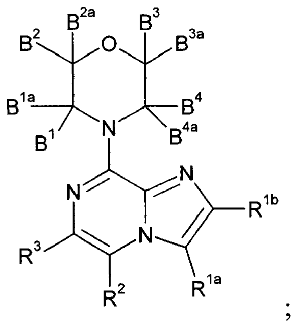 R^{1b} represents -T¹-Z¹;
T¹ represents: 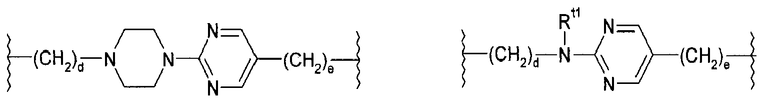
d represents 1; e represents 0; R^{t1} represents hydrogen or C₁₋₂ alkyl (e.g. methyl);
Z¹ represents -C(O)N(H)OH;
R^{1a} represents R^{1c};
R^{1c} represents hydrogen;
R² represents hydrogen;
R³ represents 2-amino-pyrimidin-5-yl.

6. A compound of formula I as defined in any one of Claims 1 to 5, or a pharmaceutically acceptable ester, amide, solvate or salt thereof, for use as a pharmaceutical.

7. A pharmaceutical formulation including a compound of formula I, as defined in any one of Claims 1 to 5, or a pharmaceutically acceptable ester, amide, solvate or salt thereof, in admixture with a pharmaceutically acceptable adjuvant, diluent or carrier.

8. A compound, as defined in any one of Claims 1 to 5, or a pharmaceutically acceptable ester, amide, solvate or salt thereof, for use in the treatment of a disease in which inhibition of a PI3-K and/or mTOR and, additionally, HDAC is desired and/or required.

9. Use of a compound of formula I, as defined in any one of Claims 1 to 5, or a pharmaceutically acceptable ester, amide, solvate or salt thereof, for the manufacture of a medicament for the treatment of a disease in which inhibition of a PI3-K and/or mTOR and, additionally, HDAC is desired and/or required.

10. A compound as claimed in Claim 8 or a use as claimed in Claim 9, wherein the disease is cancer, an immune disorder, a cardiovascular disease, a viral infection, inflammation, a metabolism/endocrine function disorder, a neurological disorder, an obstructive airways disease, an allergic disease, an inflammatory disease, immunosuppression, a disorder commonly connected with organ transplantation, an AIDS-related disease, benign prostate hyperplasia, familial adenomatosis, polyposis, neuro-fibromatosis, psoriasis, a bone disorder, atherosclerosis, vascular smooth cell proliferation associated with atherosclerosis, pulmonary fibrosis, arthritis glomerulonephritis and post-surgical stenosis, restenosis, stroke, diabetes, hepatomegaly, Alzheimer's disease, cystic fibrosis, a hormone-related disease, an immunodeficiency disorder, a destructive bone disorder, an infectious disease, a condition associated with cell death, thrombin-induced platelet aggregation, chronic myelogenous leukaemia, liver disease, a pathologic immune condition involving T cell activation, CNS disorders, and other associated diseases.

11. A method of treatment of a disease in which inhibition of a PI3-K and/or mTOR and, additionally, HDAC is desired and/or required, which method comprises administration of a therapeutically effective amount of a compound of formula I as defined in any one of Claims 1 to 5, or a pharmaceutically-acceptable ester, amide, solvate or salt thereof, to a patient suffering from, or susceptible to, such a condition.

12. A combination product comprising:
(A) a compound of formula I as defined in any one of Claims 1 to 5, or a pharmaceutically-acceptable ester, amide, solvate or salt thereof; and
(B) another therapeutic agent that is useful in the treatment of in the treatment of cancer and/or a proliferative disease,
wherein each of components (A) and (B) is formulated in admixture with a pharmaceutically-acceptable adjuvant, diluent or carrier.

13. A process for the preparation of a compound of formula I as defined in Claim 1, which process comprises:
(i) reaction of a compound of formula II, 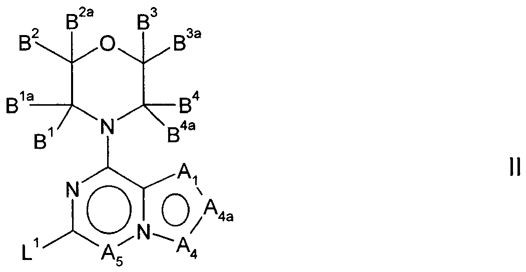 wherein L¹ represents a suitable leaving group, and A₁, A₄, A₄ₐ, A₅, B¹, B^{1a}, B², B^{2a}, B³, B^{3a}, B⁴ and B^{4a} are as defined in Claim 1, with a compound of formula III,
R³-L² III
wherein L² represents a suitable group;
(ii) reaction of a compound of formula IV, 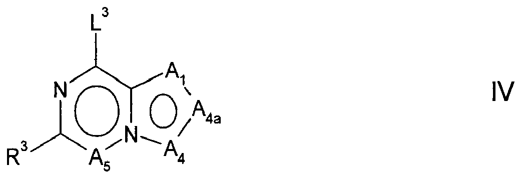 wherein L³ represents a suitable leaving group, and A₁, A₄, A₄ₐ, A₅ and R³ as defined in Claim 1, with a compound of formula V, 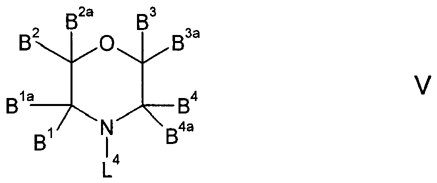 wherein L⁴ may represent hydrogen (so forming an amine group), and B¹, B^{1a}, B², B^{2a}, B³, B^{3a}, B⁴ and B^{4a} are as defined in Claim 1;
(iii) for compounds of formula I in which (A⁵ represents C(R²) and) R² represents halo, reaction of a corresponding compound of formula I, in which R² represents hydrogen, with a reagent that is a source of halide ions (a halogenating reagent);
(iv) for compounds of formula I in which R² (if present; i.e. if A⁵ represents C(R²)) represents a substituent other that hydrogen, or halo, reaction of a corresponding compound of formula I, in which R² represents halo, with a compound of formula VI,
R^{2a}-L⁷ VI
wherein R^{2a} represents R² as described in Claim 1 provided that it does not represent hydrogen or halo, and L⁷ represents a suitable leaving group;
(v) for compounds of formula I in which A^{4a} represents C(R^{1b}) and R^{1b} represents C₁₋₁₂ alkyl or heterocycloalkyl (as a part of the appropriate linker group) or R^{1a} is present, which represents -C(O)OR^{10a}, halo, C₁₋₁₂ alkyl or heterocycloalkyl (which latter two groups are optionally substituted as defined in Claim 1; and hence a -C(O)H group is possible) reaction of corresponding compounds of formula I in which R^{1a} or R^{1b} (as appropriate) represents hydrogen, by reaction in the presence of a suitable base, followed by reaction in the presence of an electrophile that is a source of halide ions, or a compound of formula VII, L⁸-R^{1b1} VII
wherein L⁸ represents a suitable leaving group, or -N(CH₃)₂, and R^{1b1} represents -C(O)OR^{10a}, C₁₋₁₂ alkyl or heterocycloalkyl (which latter two groups are a part of the appropriate linker/spacer group); i.e. R^{1b1} may represent -C(O)H);
(vi) for compounds of formula I which contain a -C(OH)(H)-C₁₋₁₁ alkyl group (which alkyl group may be substituted by one or more substituents selected from those defined in Claim 1, but is preferably unsubstituted), for example when there is a R¹, R^{1a}, R^{1b} and/or R² group present which represents such a -C(OH)(H)-C₁₋₁₁ alkyl group, reaction of a corresponding compound of formula I in which there is a -C(O)H group present (i.e. R¹, R^{1a}, R^{1b} and/or R² represents -C(O)H), with a compound of formula VIII,
R^{xx}MgX¹ VIII
wherein R^{xx} represents C₁₋₁₁ alkyl optionally substituted by one or more substituents selected from those defined in Claim 1 and X¹ represents halo;
(vii) compounds of formula I in which A₁ and A₄ both represent N, A₅ represents C(R²) and A₄ₐ represents C(R^{1b}) may be prepared by reaction of a compound of formula IX, 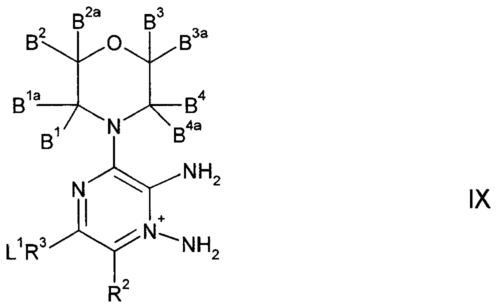 wherein L¹R³ represents either L¹ as defined above or R³ as defined in Claim 1, and R², B¹, B^{1a}, B², B^{2a}, B³, B^{3a}, B⁴ and B^{4a} are as defined in Claim 1, with a compound of formula X,
H-C(O)-R^{1b} X
wherein R^{1b} is as defined in Claim 1, and, when L¹R³ in the compound of formula IX represents L¹, then this process step may be proceeded by process step (i) as defined above;
(viii) compounds of formula I in which A₁ represents N, A₄ represents C(R^{1a}), A₄ₐ represents N and A₅ represents C(R²) may also be prepared by reaction of a compound of formula XI, 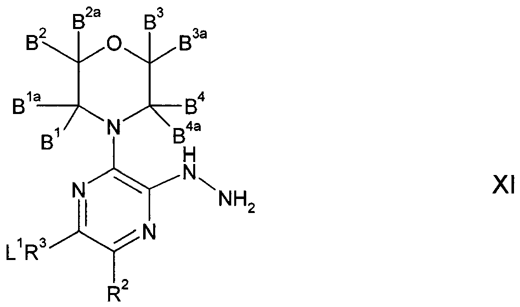 wherein L¹R³ is as defined above and R², B¹, B^{1a}, B², B^{2a}, B³, B^{3a}, B⁴ and B^{4a} are as defined in Claim 1, with a compound of formula XII,
R^{1a}-C(OC₁₋₆ alkyl)₃ XII
or, a compound of formula XIII,
R^{1a}-C(O)OH XIII
or, derivatives of either, wherein R^{1a} is as defined in Claim 1 and when L¹R³ in the compound of formula XI represents L¹, then this process step may be proceeded by process step (i) as defined above;
(ix) compounds of formula I in which Z¹ represents -C(=Y)N(R^{10a})-OH (and Y is preferably =O) may be prepared by reaction of a corresponding compound of formula I in which there is a -C(=Y)OR^{xx} (in which R^{xx} represents H or C₁₋₆ alkyl such as ethyl) with a compound of formula XIIIA,
HN(R^{10a})-OH XIIIA
wherein R^{10a} is as hereinbefore defined;
(x) for compounds of formula I in which T^{1a} represents -CH₂- and X^{1a} represents -N(R^{t1})- or heterocycloalkylene (linked to T^{1a} *via* a nitrogen atom of that cyclic group), reaction of a compound corresponding to a compound of formula I but in which -T¹-Z¹ represents -CH₂-L^{xx} (in which L^{xx} represents a suitable leaving group, such as chloro) with a compound containing the X^{1a} moiety (i.e. HN(R^{t1})-X^{1b}-T^{1b}-Z¹ or heterocycloalkyl-X^{1b}-T^{1b}-Z¹);
(xi) for compounds of formula I in which T^{1a} represents -CH₂- and X^{1a} represents -N(R^{t1})-, reaction of a compound corresponding to a compound of formula I but in which -T¹-Z¹ represents -CH₂-N(R^{t1})H with a compound L^{xx}-X^{1b}-T^{1b}-Z¹ (where the integers are defined above or in Claim 1.

14. A process for the preparation of a pharmaceutical formulation as defined in Claim 7, which process comprises bringing into association a compound of formula I, as defined in any one of one of Claims 1 to 5, or a pharmaceutically acceptable ester, amide, solvate or salt thereof with a pharmaceutically-acceptable adjuvant, diluent or carrier.

15. A process for the preparation of a combination product as defined in Claim 12, which process comprises bringing into association a compound of formula I, as defined in any one of Claims 1 to 5, or a pharmaceutically acceptable ester, amide, solvate or salt thereof with the other therapeutic agent that is useful in the treatment of cancer and/or a proliferative disease, and at least one pharmaceutically-acceptable adjuvant, diluent or carrier.
